# EUROPEAN PATENT SPECIFICATION

(11) **EP 1 414 953 B1**
(45) Date of publication and mention of the grant of the patent: **11.04.2007**
(21) Application number: 02743460.4
(22) Date of filing: 19.06.2002
(51) Int. Cl.: C12N 9/12

(54) **IDENTIFICATION AND CLONING OF THE RECEPTOR GENE FOR SYMBIOTIC NITROGEN FIXATION**
IDENTIFIZIERUNG UND KLONIERUNG DES REZEPTORGENS ZUR SYMBIOTISCHEN STICKSTOFFFIXIERUNG RESISTENZ
IDENTIFICATION ET CLONAGE D'UN GENE RECEPTEUR POUR FIXATION D'AZOTE SYMBIOTIQUE

(30) Priority: 20.06.2001 HU 0102561
(43) Date of publication of application: 06.05.2004
(73) Proprietor: MTA Szegedi Biologiai Központ, 6726 Szeged (HU)
(72) Inventor: KISS, György, Botond, H-6725 Szeged (HU); ENDRE, Gabriella, H-6723 Szeged (HU); KERESZT, Attila, H-6723 Szeged (HU); KEVEI, Zoltán, H-6724 Szeged (HU); KALO, Péter, H-6723 Szeged (HU); KISS, Péter, H-6725 Szeged (HU)
(74) Representative: Szentpéteri, Adam
(86) International application number: PCT/HU2002/000055
(87) International publication number: WO 2002/102841

(56) References cited:
- WO-A-00/42171
- DATABASE SWALL [Online] 1 March 2001 (2001-03-01) KOTANI,H. ET AL.: "Receptor protein kinase-like protein" Database accession no. Q9FKC2 XP002245537
- OLDROYD GILES E D: "Dissecting symbiosis: Developments in Nod factor signal transduction." ANNALS OF BOTANY (LONDON), vol. 87, no. 6, June 2001 (2001-06), pages 709-718, XP002245534 ISSN: 0305-7364
- ENDRE GABRIELLA ET AL: "A receptor kinase gene regulating symbiotic nodule development." NATURE (LONDON), vol. 417, no. 6892, 2002, pages 962-966, XP002245535 27 June, 2002 ISSN: 0028-0836
- STRACKE SILKE ET AL: "A plant receptor-like kinase required for both bacterial and fungal symbiosis." NATURE (LONDON), vol. 417, no. 6892, 2002, pages 959-962, XP002245536 27 June, 2002 ISSN: 0028-0836

## Description

### TECHNICAL FIELD

The invention relates to the *Medicago sativa* DNA sequence encoding the NORK polypeptide as well as to the protein determined by this DNA sequence. In another aspect, the invention relates to conserved DNA regions, primers, DNA probes, polypeptides and corresponding antibodies characteristic to the NORK gene family, as well as to methods and their utilization to isolate additional NORK genes and proteins from different legumes or other plants, and their use for producing transgenic plants.

In another aspect, the invention relates to a method of cloning a DNA sequence encoding a function involved in one of the steps of symbiotic nitrogen fixation, as well as to methods of producing transgenic plant cells and cloned entire plants wherein the biosynthesis of the NORK polypeptide takes place.

The invention also relates to the identification of a gene present in leguminous plants, encoding a function indispensable for symbiotic nitrogen fixation, to the sequencing of the gene, confirmation of the biological function of the gene, and the transformation of the gene into plants unable for symbiotic nitrogen fixation.

### BACKGROUND ART

It is well known that symbiotic nitrogen fixation covers the requirements of the nitrogen demands of a plant and ensures normal growth, development and seed production in soils with limited amount of fixed nitrogen without added artificial nitrogen fertilizers (1). Plants capable of symbiotic fixation differ from other plants in that they carry all of the genetic determinants in their genome which are needed for the biological process of symbiotic nitrogen fixation. For those experts who are familiar with the recombinant DNA techniques it is obvious that these genes can be identified, isolated and transferred to other plants on such a way that they retain their function. In theory, all genes required for symbiotic nitrogen fixation can be transferred to recipient plants which eventually will be able to establish symbiotic nitrogen fixation, consequently, they can be grown on a more economical and environmental friendly manner.

The identification of genes involved in symbiotic nitrogen fixation are being studied very intensively worldwide, still the nucleotide and amino acid sequences which are presented in this patent application are completely novel; we had access only to randomly sequenced cDNA sequences.

The symbiotic nitrogen fixation is a biological process established between particular dicotyledonous plants and soil bacteria (2, 3). The symbiotic nitrogen fixation takes place mostly in the root system where root nodules develop, and are invaded by the bacteria. During the development of root nodules bacteria enter the plant cells and change to bacteroids, and in such form they fix nitrogen that is they convert the inert dinitrogen of the air into ammonia. This ammonia (which is a form of the so-called fixed nitrogen) can be utilized by the plants for their development. Since no artificial nitrogen fertilization is needed, symbiotic nitrogen fixation is an environmental friendly, cost effective and improve the quality of the soil, therefore, these plants are very suitable as green crops. Agricultural legumes, which are capable to fix the nitrogen from the air, are therefore very important part of the agriculture. Their seeds and green mass contribute to large extent to the human and animal amino acid and oil supply, therefore, legumes are extremely important in the worldwide food supply.

Some soil bacteria, like *Rhizobia* and *Actinomycetes,* are able to establish symbiotic nitrogen fixation relationship with given plants, like many legumes, *Trema, Casuarina, Alnus, Parasponia* (2, 3).

As a consequence of the evolution this symbiotic relationship is strictly species specific, a given group of bacteria establishes symbiosis only with a given group of plants (2, 3, 4, 5). Such symbiosis is established between *Sinorhizobium meliloti* and *Medicago, Melilotus, Trigonella* species (6), between *Rhizobium leguminosarum* bv. *viciae* and *Vicia* species, between *Rhizobium leguminosarum* bv. *trifolii* and given members of *Trifolium* species, between *Rhizobium etli* and *Phaseolus* species, between *Bradyrhizobium japonicum* and *Glycine max*, between *Azorhizobium caulinodans* and *Sesbania rostrata,* between *Mesorhizobium loti* and *Lotus japonicus (7).*

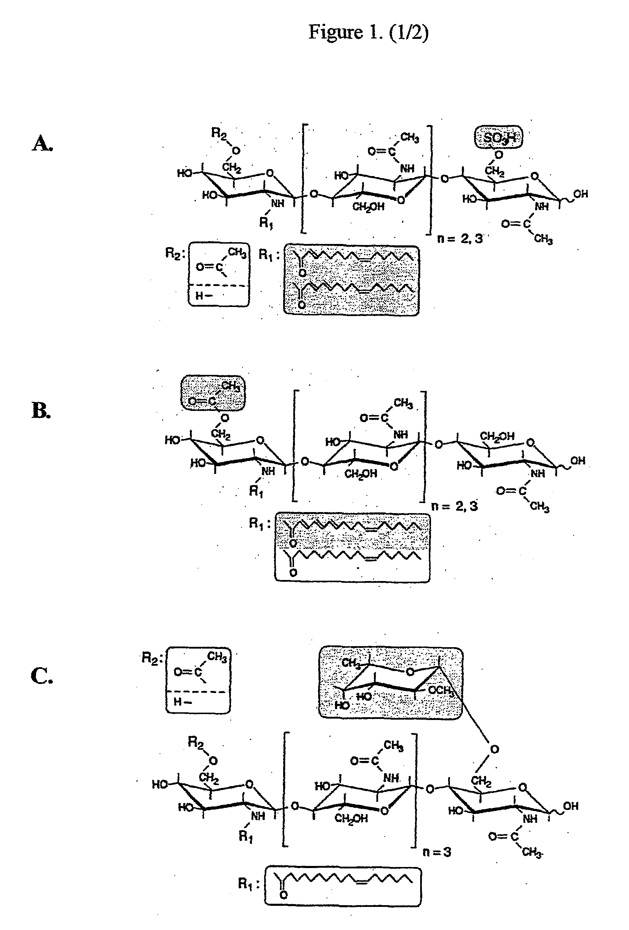

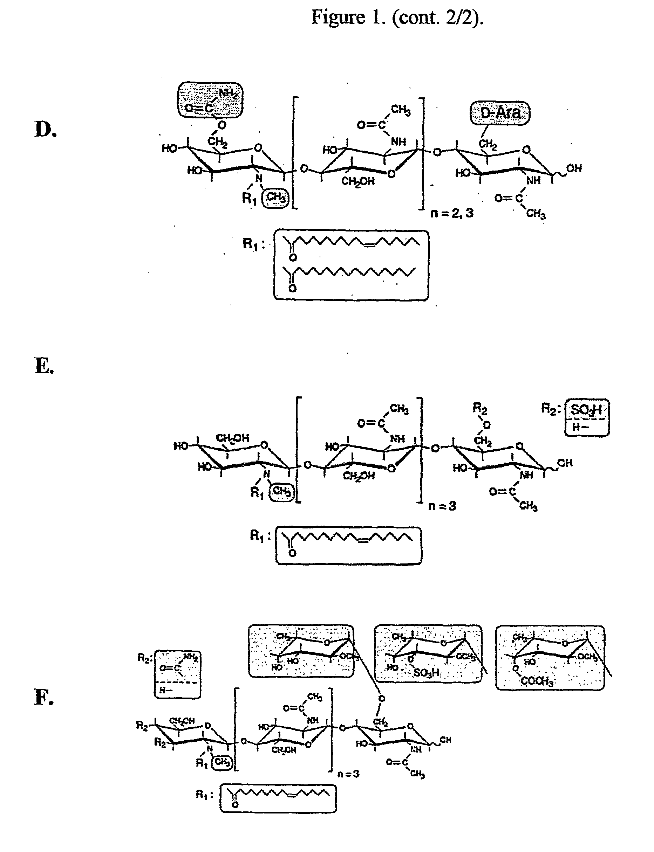

The biogenesis and development of the symbiotic nodule, as well as the invasion of the plants cells by the bacteria is a complex; multi-step process taking place through series of gene-interaction involving both bacterial and plant symbiotic genes. The formation of the nodule, which is inevitable for the symbiotic nitrogen fixation according to our present knowledge, starts to develop after a specific signal exchange between the microsymbiont (the bacteria) and the macrosymbiont (the plants). Through the root system, the macrosymbionts excrete specific flavonoid and other molecules into the environment, which are characteristic to and determined by the genetic material of the plant species (8, 9). As a response to these excreted molecules compatible bacteria approach by chemotaxis the plant root system (10) and start to produce a signal molecule, the so-called lipo-chitooligosaccharides abbreviated as Nod factors, which in turn are specific nodulation signal for the plant (11, 12, 13). During nodule formation compatible bacteria attach to the root-hair surface on a polar manner. In this joining special molecules like bacterial pili, plant lectins and other adhesive molecules are active components (14). Upon the action of the symbiotic specific Nod factors, characteristic plant responses occur like root-hair deformation, curling and branching (15). Inside the root tissue, the cortical cells start to divide. As a response of the specific Nod factor produced by the compatible bacteria a symbiotic nodule start to develop on the root or on the stem depending on the type of symbiosis. Some of the specific Nod factors are shown in Fig. 1. without the sake of completeness (5, 7). As a consequence of the root-hair curling provoked by the specific Nod factor, the bacteria become surrounded and enter the so-called infection threads produced by the plant. The infection threads start to grow towards the core tissue of the plant, where they start to branch. Inside the infection thread, the bacteria divide and move along the infection threads. At the same time the cortical cells continue to divide and the nodule meristeme is formed. The branching infection threads enter the dividing cells and the bacteria are released inside the plants cells, where they continue to divide and in a short time they occupy most of the plant cytoplasm establishing the so-called infected cells. In the infected cells bacteria are surrounded by plant plasma membrane (peribacteroid membrane) in which the bacteria change to bacteroids, in which form they are capable to fix nitrogen (16, 17). The derepression of the nitrogenase enzyme complex takes place only in the bacteriods. The bacteroids, with the help of their nitrogenase enzyme complex, can reduce the inert dinitrogen into ammonia, the so-called combined nitrogen, which can be utilized by the macrosymbiont. Most of the ammonia formed during nitrogen fixation (that part which is not utilized by the bacteroids) is excreted by the bacteroids. The excreted ammonia is taken up by the nodule cells and transferred towards the vascular tissue by a proper conducting mechanism. The fully developed and active nodules are able to supply the plants with enough combined nitrogen which is needed for their normal growth and development.

Root-hair deformation and cortical cell division can be evoked by very low (10⁻⁸-10⁻¹² M) concentration of the compatible Nod factor even if the bacteria are not present, if the Nod factor is applied to the root. Following Nod factor treatment normal nodule structures are formed on the plant roots, in which no bacteria are present. From this phenomenon it is obvious, that the Nod factors are recognized by the root cells, which in turn start the specific signal transduction pathway leading to nodule formation. The recognition of the excreted Nod factor on the surface of the root cells is taking place by an unknown mechanism. Nod factor binding receptors were looked for by biochemical methods, but no such a supposed protein responsible for the binding and transmission of the Nod factor could have been identified so far (18). However, it is generally accepted, that the bacterial Nod factor is recognized and bound by a specific receptor and through this mechanism the signal transduction cascade evokes nodule induction and development.

The steps leading to nodule development, the functions involved, and the genes coding for these functions in the plants are not known at present, however, one can understand that the presence of the microsymbiont for the formation of the nodule structure is not necessary. Some plants like alfalfa (*Medicago sativa* L.) is able to form spontaneous nodules. Under sterile conditions and in the absence of microbes and higher concentration of combined nitrogen (more than 1 mM) alfalfa develops some nodule-like structure, which are very similar to the morphology and tissue characteristics of the normal nodules (19). As a consequence, all those genes and functions, which are necessary for nodule development are encoded by the plant genetic material. However, the Nod factors produced by the compatible microosymbiont are responsible to what extend, and what type of nodules are formed by the plant.

Several genes, both bacterial and plant origin, are involved in the development and effective functioning of the nodules. Most of the genes in S. *Meliloti* and in other *Rhizobia* are knnown (5, 20, and personal information from Michael Göttfert, Technische Universität Dresden, Germany). According to Prof. Alfred Pühler, University of Bielelfeld, Germany the nucleotide sequence of the entire *S. meliloti* geome is known. However, the identification and the function of the plant genes involved in this biological process is still in its infancy. At present, biochemical and combined genetic and biochemical approaches are applied to study plant genes. Genes identified by biochemical methods are questionable as to whether they are involved in symbiotic nitrogen fixation. The nodule specific expression of many genes isolated by biochemical approaches was demonstrated nevertheless their real function was not uncovered (21, 22, 23), or it turned out that they are not essential to effective symbiotic nitrogen fixation (24).

Among others the *Medicago* EST programs will be useful to get acquainted with the nodule specific genes. These programs generated until now more than 100 000 partial EST sequences. These sequences are open for public on the Internet (**http://www.ncbi.nlm.nih.gov**). Among these sequences there are three partial cDNA sequences homologous to the NORK gene of *M*. *truncatula.* Form the mere sequence, however, it is impossible to predict the function of the gene, therefore more analysis is needed to reveal the function. The most powerful tool for the study of gene functions if mutant alleles of the genes are available. One can generate a mutant allele of a gene, or one can isolate a gene from a mutant allele. The first possibility (homologue recombination) is not available for plants. The later, however is feasible through map based cloning or insertion mutagenesis (tranposons, T-DNA) based gene isolation procedures. According to mutant phenotype (T-DNA insertion) only one gene has been isolated form *Lotus japonicus:* this gene *(nin)* encoded a transcription factor (25). In this patent we describe for the first time the isolation of a gene by map based cloning that is the isolation of the NORK gene with the help of the *Medicago sativa* mutant MN1008.

Classical genetic strategy leads to unambiguous results. The isolated gene is an essential component of the biological process in question if the gene affected by the mutation has a phenotype (unable to carry out certain functions or less active in a given function) and the gene isolated based on the phenotype. In the mutant individuals an essential function affected which is involved in the biological process studied. Appropriate methods are available by which the mutant gene ant ist wild type counterpart can be isolated. In order to acquire mutant individuals mutations can be induced by physical, chemical or biological means. Mutant genes, in which no insertions of known sequences are present, or the nature of the mutation is not known (like in the case of large deletions, inversions) can be isolated by the so-called map based cloning. The principal of the map based cloning is the following: first the phenotype is mapped genetically with molecular (DNA) markers, and then with the help of these markers chromosomal walking is performed and the gene is isolated form clones originated form large insert libraries (26).

Mutant plants impaired in symbiotic nitrogen fixation can be grouped into two categories: mutants in the first class are the Nod⁻ mutants that is no nodules appear on their root system; mutants in the second class are the Fix⁻ mutants which develop nodules or nodule like structures but the nodules are ineffective, that is no effective Ůrogen fixation takes place. It is a characteristic phenomenon of both mutant types, that in the presence of the compatible bacteria and in the absence of combined nitrogen the symptoms of nitrogen deficiency develop on the mutants, that is yellow leaves and stunted growth. There are many symbiotic mutants available published in the literature. Among the Nod⁻ mutants the most important ones are the *Medicago sativa* MN1008 mutant (27, 28), the *Medicago truncatula* B129, Tr25, Trv25, B85 mutants (29), the *Pisum sativum sym*8*, sym*10, *sym*19 mutants (30), the *Lotus japonicus sym*2 mutant (31), and the *Glycine max nod*49 and *nod*139 mutants (14).

The MnNC-1008 (NN) alfalfa (*Medicago sativa*) mutant (abbreviated as MN1008) is incapable to form nodules on its root system in the presence of its compatible microsymbiont (*Sinorhizobium meliloti*) (27, 28). Further studies showed that there was no sign of any response detectable by microscopy, which was characteristic to the wild type plant early nodulation response. There was no root hair deformation and cortical cell division (32), in addition to the lack of the specific calcium spiking response (special calcium oscillation inside the cells) (33). According to unpublished results, there was no root hair curling or cortical cell division observed in the mutant MN1008 (unlike in the wild type plant) upon addition the specific *S. meliloti* Nod factor. Mutant MN1008, on the other hand, was able to develop nodules, the so-called spontaneous nodules, in the absence of microbes (see above). This phenomenon reflects that the plant nodulation program was intact. From the above results the most plausible conclusion was that the alfalfa mutant MN-1008 had a defect in the perception of the Nod factor, that is the assumed Nod factor receptor gene suffered mutation, or one of the member in the early signal transduction chain.

### DISCLOSURE OF INVENTION

In agreement with the patent the knowledge and use of the DNA sequence, the described technology, specifically the isolation of the Nod factor receptor gene, or individual genes in the signal transduction has a great importance in the agriculture. The transfer of these genes into plants which are unable to fix nitrogen but have great agricultural importance, plants like rice, corn, wheat, barley, rye and so on, may ensure the possibility, that in the presence of appropriate Nod factors produced by *Rhizobium* bacteria the transgenic plants carrying and expressing Nod factor receptor genes and genes of the member of the signal transduction chain might be able to perform the first biological steps leading to nodule formation. Cloning and transfer other nodule specific genes might lead to the formation of normal symbiotic nodule and nitrogen fixation.

In accordance with the above, the invention relates to the *Medicago sativa* DNA sequence encoding the NORK polypeptide as well as to the protein determined by the mentioned DNA sequence. In addition the invention features conserved DNA regions, primers, DNA probes, polypeptides and corresponding antibodies characteristic to the NORK gene family, as well as to methods and their utilization to isolate additional NORK genes from different legumes or other plants, and to use those genes for producing transgenic plants.

In another aspect, the invention features a method of cloning a DNA sequence encoding a function involved in one of the steps of symbiotic nitrogen fixation, as well as to methods of producing transgenic plant cells and cloned entire plants wherein the biosynthesis of the NORK polypeptide expresses.

The experiments described in this invention relates to the identification and isolation of genetic determinants (genes) involved in the determination and development of symbiotic nodules of alfalfa (*Medicago sativa*) and in the perception and/or signal transduction of the Nod factor originating from the bacteria, with the help of the MN-1008 mutant incapable for Nod factor triggered nodule development, on such a way, that the mutant phenotype is genetically mapped with the use of molecular markers, followed by the isolation of overlapping BAC clones using the tightly linked markers and by recombinant DNA techniques, then sequencing the genes in the overlapping BAC clones, and finally sequencing the mutant alleles responsible for the mutant phenotype. Applying the above procedure the following nucleotide sequence was identified:

The invention relates to the above genomic or synthetic nucleotide sequence molecules, to the amino acid sequence deduced from this DNA having NORK specific biological activity. In another aspect, the invention features those antibodies suitable for the detection of NORK polypeptides. In addition the invention relates to cells and transgenic plants as well transformed with DNA molecules protected by this invention.

The invention presents a procedure by which certain plants with Nod⁻ phenotype can be converted to Nod⁺ phenotype.

To achieve the aim of the invention the MN-1008 mutant plant was grown. As opposed to the wild type plant, mutant MN-1008 respond (does not start nodulation consequently does not form nodules) neither to the compatible *Sinorhizobium meliloti* bacterium, nor to the Nod factor produced by the bacteria. It is supposed that the mutation in MN-1008 plant affected one of the genes involved in the signal perception and/or one of the early steps in the signal transduction pathway, it is likely the mutation affected the Nod factor receptor genes.

Under appropriate conditions, e.g. in the presence of sufficient nitrogen supply or in soil mutant MN1008 can grow and start to flourish. Mutant MN1008 was crossed with a nodulating wild type plant, the F1 generation and then by self-mating the F2 populations were established. In the F2 population (mapping or segregating population) the traits are segregating. From the individuals of the F2 population Nod⁻ and Nod⁺ individuals were colleted (see Example 1.) and linked genetic markers were looked for (see Example 2.). With the help of the diploid mapping population the linked markers were mapped and it was concluded that all these markers mapped in the same region (see Example 3.). According to the map position of the diploid map, linked markers were selected from this region, which were mapped in the Nod⁻ segregating population (see Example 4.). It turned out that these markers were linked

To achieve the aim of the invention the MN-1008 mutant plant was grown. As opposed to the wild type plant, mutant MN-1008 respond (does not start nodulation consequently does not form nodules) neither to the compatible *Sinorhizobium meliloti* bacterium, nor to the Nod factor produced by the bacteria. It is supposed that the mutation in MN-1008 plant affected one of the genes involved in the signal perception and/or one of the early steps in the signal transduction pathway, it is likely the mutation affected the Nod factor receptor genes.

Under appropriate conditions, e.g. in the presence of sufficient nitrogen supply or in soil mutant MN1008 can grow and start to.flourish. Mutant MN1008 was crossed with a nodulating wild type plant, the F1 generation and then by self-mating the F2 populations were established. In the F2 population (mapping or segregating population) the traits are segregating. From the individuals of the F2 population Nod⁻ and Nod⁺ individuals were colleted (see Example 1.) and linked genetic markers were looked for (see Example 2.). With the help of the diploid mapping population the linked markers were mapped and it was concluded that all these markers mapped in the same region (see Example 3.). According to the map position of the diploid map, linked markers were selected from this region, which were mapped in the Nod⁻ segregating population (see Example 4.). It turned out that these markers were linked to the Nod' phenotype, therefore mapped in the region where the Nod⁻ mutation mapped to, and two of these were tightly linked (see Example 4.).

With the help of the tightly linked markers so-called BAC clones were isolated from the BAC library of M. *truncatula,* followed by the ordering of the BAC clones into so-called contigs (see Example 5.). The so-called Nod contig was about a 500 kilobase-pairs (kb) long DNA region consisting of overlapping individual BAC clones. By the analysis of about 5000 F2 plants it turned out that in two individuals the recombination breakpoint situated within the Nod contig flanking both sides. This region was about 400 kb containing the Nod⁻ mutation (see Example 7.).

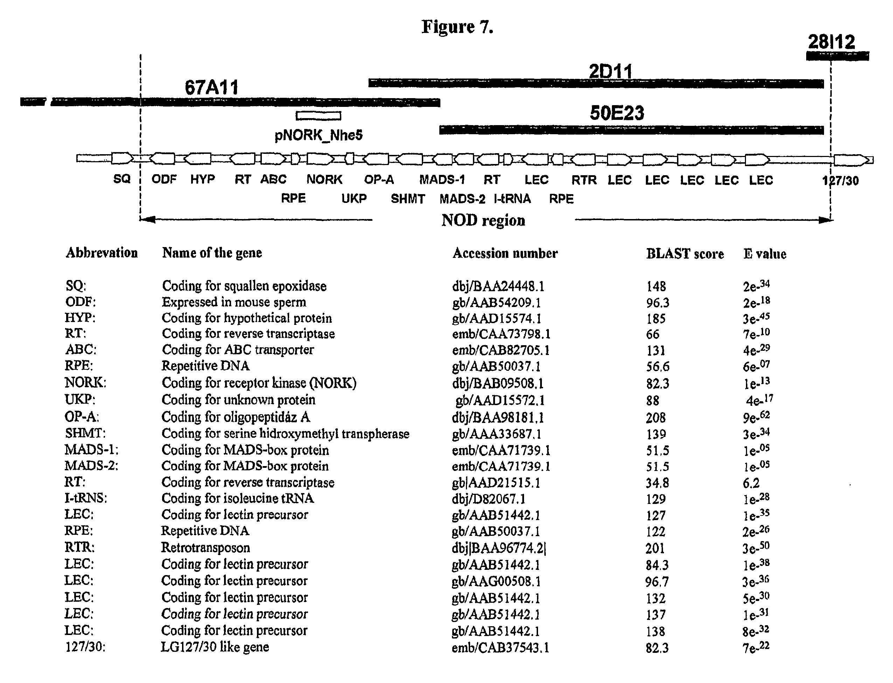

The BAC clones constituting the 400 kb contig were sub-cloned (see Example 6.), and the nucleotide sequence of the Nod region was determined, and the genes in this region were identified (see Example 8.). The Nod⁻ mutant phenotype could be explained by mutation in some of the genes. Without limitation only two genes are mentioned as an example. One of these genes is an ABC. transporter gene. Mutation in ABC transporter genes are responsible for the multidrog resistance phenotype (34) and for the human cystic fibrosis disease (35), therefore it is possible that the Nod factor uptake is mediated by ABC transporter proteins. The other gene is coding for a receptor kinase which was named as the NORK gene. The receptor kinase genes in plants are responsible for the perception and transduction of signal molecules (36, 37, 38). The lack of one of these functions could lead to Nod⁻ phenotype. However, there are some more genes in the Nod region (see Fig. 7.) of which some are listed like the ODF gene, the MADS box gene, and the lectin genes, which could be responsible for the Nod⁻ phenotype if they carry mutation alone or in any combination.

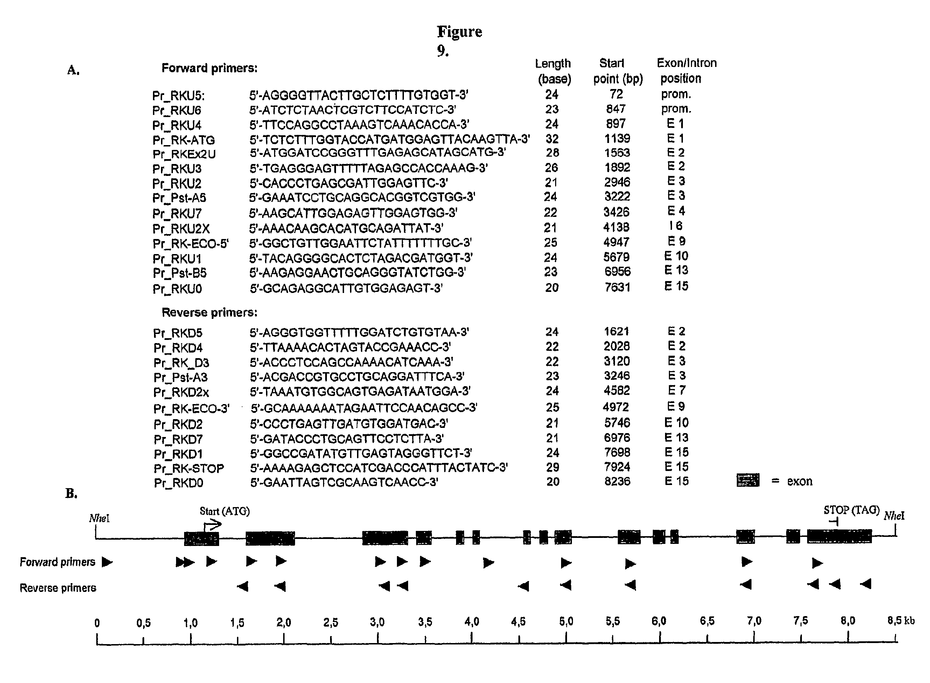

After the sequencing of the wild type allele of these genes, the mutant alleles were also sequenced on such a way, that gene specific primer pairs were designed, synthesized and used for the amplification of the appropriate DNA region of the MN-1008 mutant plant. No nucleotide changes were found in the ABC transporter, ODF, MADS box and lectin genes which could be responsible for the mutant phenotype. Some allelic variations were found, but these mutations could be neutral changes. On the other hand, however, an in frame stop codon mutation was found in the coding region of the NORK gene originated from the MN-1008 mutant (see Fig. 9) which terminated the protein synthesis before the naturally occurring termination. As a consequence the mutant would be shorter as compared to the wild type protein, and therefore the protein is inactive. According to the sequence analysis the mutation (the stop codon) occurred in the kinase domain in a region which is functionally active and highly conserved. With the help of the RT-PCR method and specific primers (see Fig. 9.) NORK specific cDNA fragments could be amplified. According to this result NORK specific receptor kinase mRNA was synthesized consequently the NORK gene was transcribed in both wild type and mutant plants.

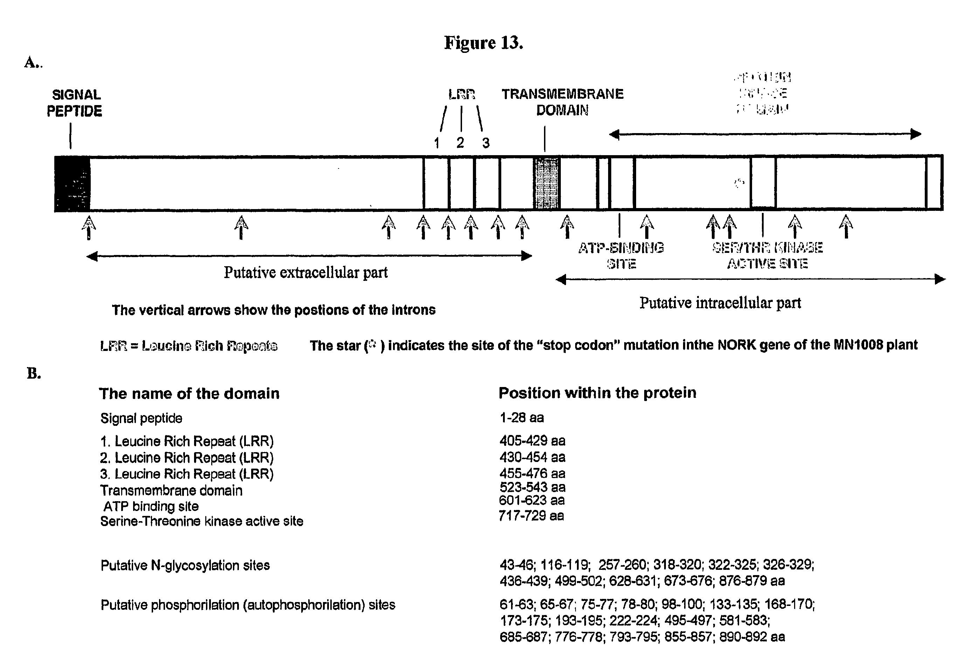

It is obvious for experts in molecular biology that the mutation in the NORK gene (the *nn1* mutation which generated an in frame stop codon) leads to loss of function because the active site needed for the kinase activity was missing (see Fig. 13). It is not excluded, however, that more mutation in the MN-1008 plant occurred (like in the ABC transporte gene, ODF gene, MADS box gene, and lectine gene), and the mutation leads to Nod⁻ phenotype as well, consequently, these genes are protected by this invention too.

Abbreviations used in this inventions:
- NORK:: NOD region specific Receptor Kinase gene.
- nod genes:: Genes found in Rhizobium bacteria and which are responsible for the synthesis and excretion of the Nod factor.
- Nod factor:: The lipo-chitooligosaccharide produced by *Rhizobium* bacteria which induces nodule formation on plant roots (see Fig. 1.).
- Nod':: Phenotype of those plants lacking nodules
- Nod+:: Phenotype of those plants with nodules
- Diploid:: Somatic cells of the plants with double chromosome set
- Teraploid:: Somatic cells of the plants with quadruplex chromosome set
- RFLP marker:: Restriction Fragment Length Polymorphism, which is used as genetic marker. The lenght polymorphis is detected in most cases by DNA-DNA huybridization.
- SHMT:: Serine-hydoxymethyl-transferase
- BAC:: Bacterial Artificial Chromosome
- BAC vector:: Low copy number cloning vector which can accomodate large (more than 100 kb) DNA fragments.
- BAC clone:: Insert cloned into BAC vector, or both the vector and the insert together.
- BAC library:: BAC clones representing the entire DNA content of an organism.
- bp:: base pairs.
- kb:: kilobase pairs.
- allele:: gene variant.
- primer:: short (5-50 bp long) single stranded oligonucleotide.
- contig:: DNA region covered by overlapping clones
- in frame:: according to the open reading frame
- RT-PCR:: Reverse transcription PCR, that is amplification product of transcribed mRNA.
- NAB:: one of the F2 family segregating the Nod⁻ character
- NBW:: the other F2 family segregating the Nod⁻ character
- RAPD:: Random Amplified Polymorphic DNA (46)

### BRIEF DESCRIPTION OF DRAWINGS

Fig.1. Structure of the signal molecules (Nod factors) produced by *Rhizobium* bacteria. Nod factors produced by: A. *Sinorhizobium meliloti;* B. *Rhizobium leguminosarum* bv. *viciae;* C. *Bradyrhizobium japonicum;* D. *Azorhizobium caulinodans;* E. *Rhizobium tropici;* F. *Rhizobium* sp. NGR234.
Fig. 2. F2 alfalfa populations segregating the Nod⁻ phenotype. The *nn1*/NN1 alleles are highlighted.
   A. Crossing of the parents; highlighting linkage group 5 with the *nn1* és *NN₁* alleles
   B. The *nn1* and *NN₁* alleles inherited by the F1 NAB plant.
   C. Segregating *nn1*/*NN₁* alleles in the F2 population originating from self mating of the F1 NAB plant.
   D. Generating F3 homozygous individulas from the self mating of the appropriate F2 individuals.

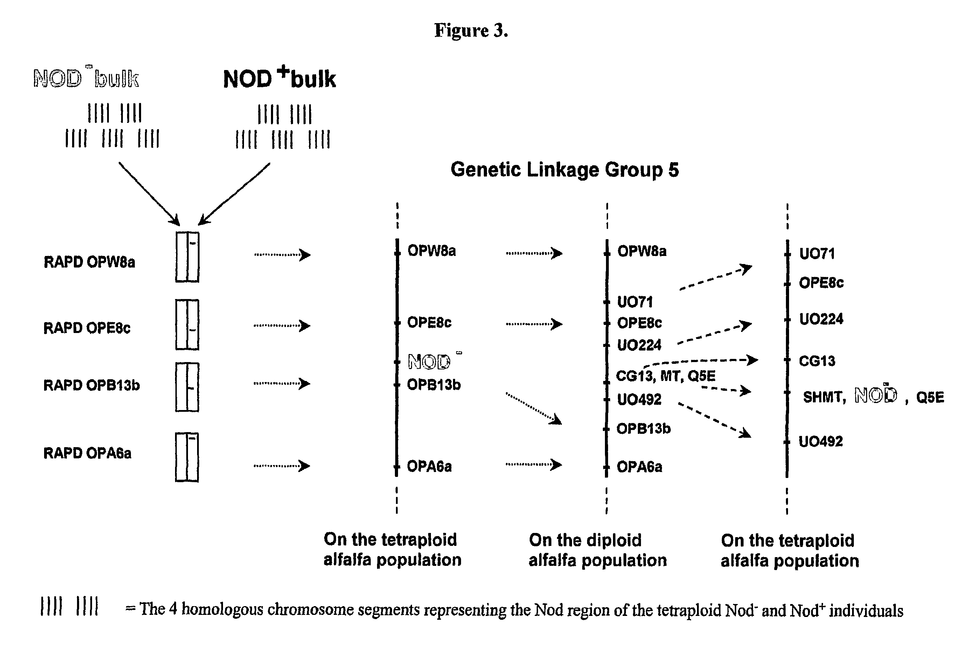

Fig.3. Genetic mapping of the *nn1* gene in the tetraploid and diploid populations of *Medicago sativa.* Markers linked to the Nod⁻ phenotype were screened by the Bulked Segregant Analysis with the help of the Nod⁻ F2 NAB plants and Operon Primers. The position of the selected RAPD markers were then determined on the diploid alfalfa map. Finally RFLP markers linked to the RAPD markers were picked and mapped in the tetraploid population in relation to the *nn1* gene.

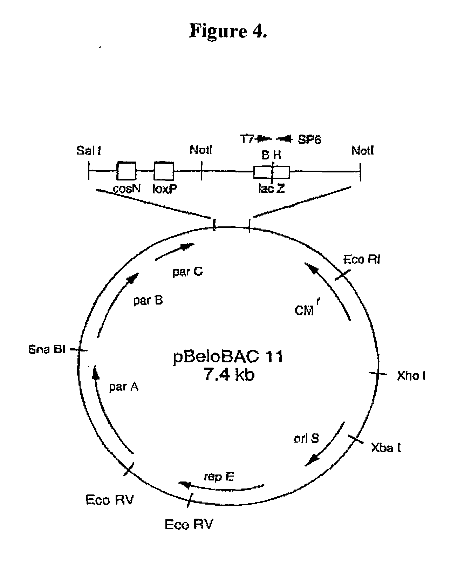

Fig. 4. Functional and restriction map of vector pBeloBac11. The cloning site (H = *Hin*dIII) sits in the *lac*Z gene. B = *Bam*HI; CM = chloramphenicol, see ref (52).

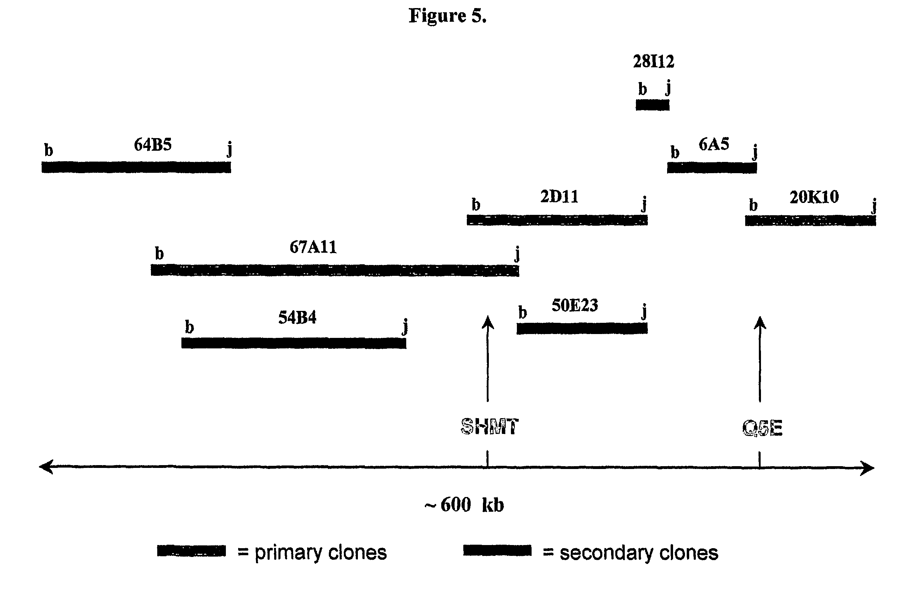

Fig.5. BAC clones in the Nod contig. Primary clones isolated by the help of the Q5E and SHMT markers are highlighted by green. Blue colors represent secondary clones isolated with the help of the end sequences of the primary clones. "b" and "j" represent the left and right side of the inserts, respectively.

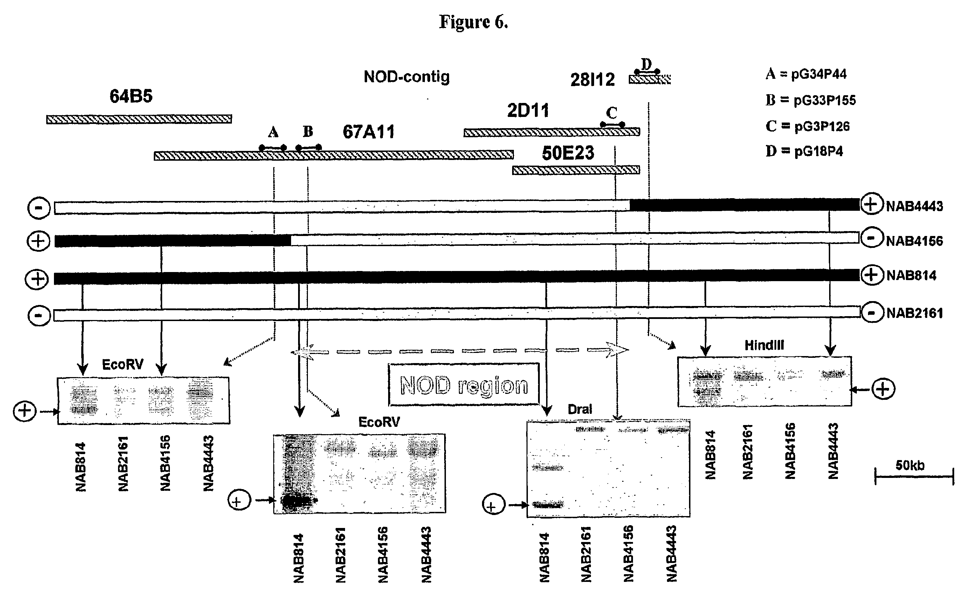

Fig. 6. Determination of the precise position of the *nn1* gene with the help of two close recombination events. The precise localization of the *nn1* gene was established with the help of two DNA originating from individuals carrying recombination events in the Nod region, as well as with the help of two DNAs originating from opposite homozygous individuals. Sub-clone G34P44 originating from BAC 67A11 was used as hybridization probe for filter containing EcoRV digested DNA from NAB4156 (recombinant), NAB814 (homozygous), NAB4443 (recombinant), and NAB2161 (homozygous) individuals. Nod⁺ allele (highlighted by + arrow) could be detected for the two Nod⁺ plants (NAB4156 and NAB 814), while Nod⁻ allele for the two Nod⁻ plants (NAB4443 and NAB2161). Sub-clone G33P135 has been used to identify a Nod⁻ allele for NAB4156. Sub-clone G3P126 identified Nod⁻ allele for plant NAB4443 after Dral digestion. Sub-clone G18P4 on the other hand detected a Nod⁺ allele for this plant after HindIII digestion. Since the two plants displaying recombination were Nod⁻ the *nn1* gene can be limited between these two recombination points.
Fig.7. Genes and there orientation found in the Nod region. The arrows show the orientation of the genes in the Nod region. The lengths of the arrows are out of scale. pNORK_Nhe5 is a pUC19 based recombinant plasmid carrying the entire NORK gene on a 8.5 kb long Nhel fragment. The name of the genes are given after the abbreviations. The accession number of that gene is given which showed the highes BLST score in the NCBl databank. Finally the e value is given, which is a generally accepted value representing the level of homology.

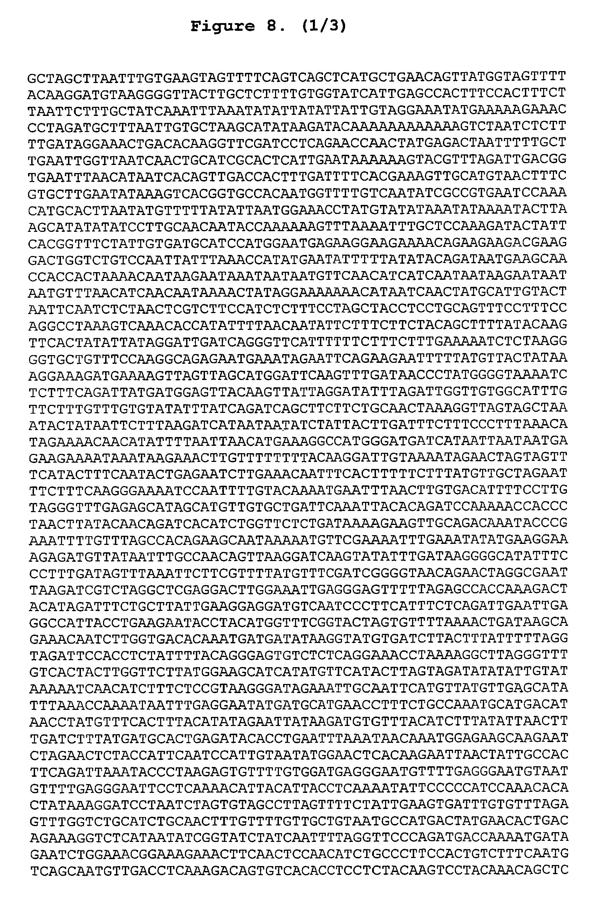

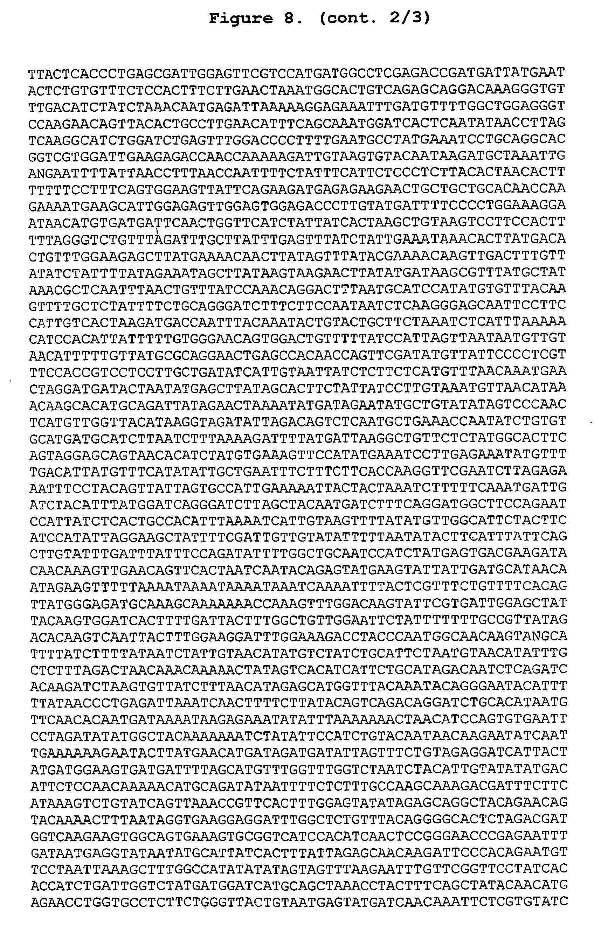

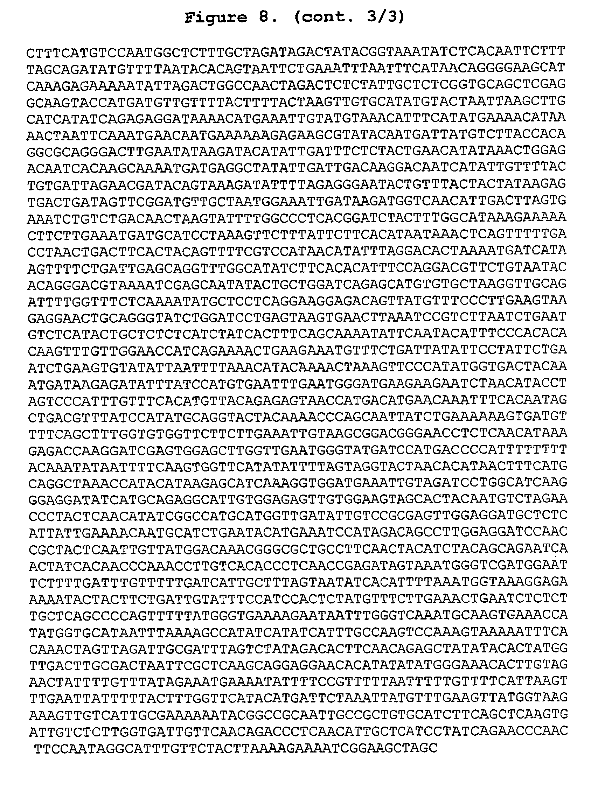

Fig. 8. Nucleotide sequence SEQ ID NO: 1 of the 8563 bp long DNA region containing the entire NORK gene. The transcription start site (ATG) is at position 1153 bp (left to right) form the Nhel site (GCTAGC). The stop codon is at position 663 bp from (right to left) the last nucleotide of the second Nhel site. There are 15 exons and 14 introns in the gene. The position of the exons and introns are highlighted in Table 6. (There are 60 nucleotides in one row).
Fig. 9. List of the oligonucleotide primers used for the amplification of both genomic and cDNA sequences.
   A. Name, sequence, length, and start points of the primers.
   B. Schematic representation of the NORK gene with the position of the primers.

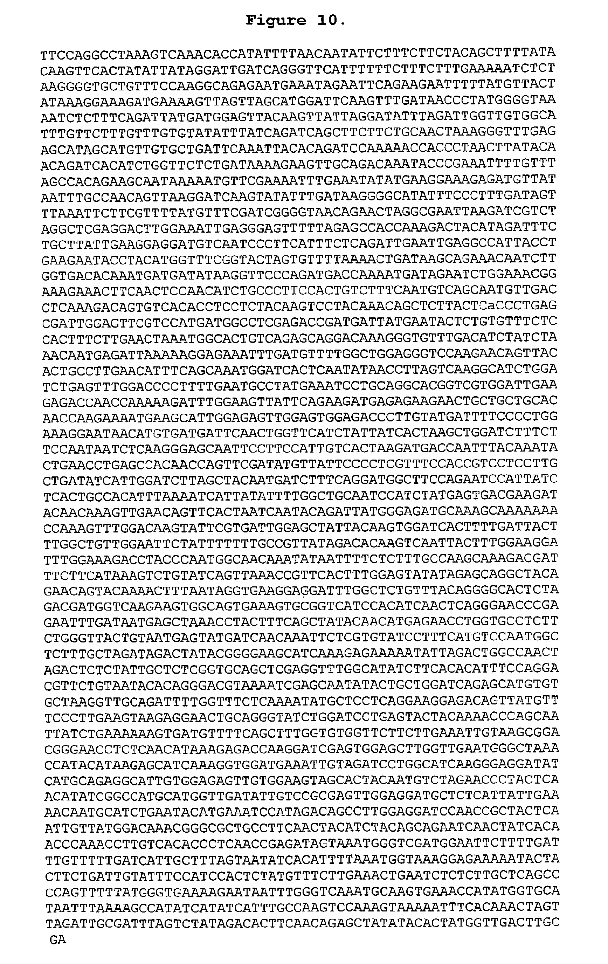

Fig. 10. cDNA sequence SEQ ID NO:1 of the NORK gene from *Medicago truncatula* A17 (There are 60 nucleotides in one row).

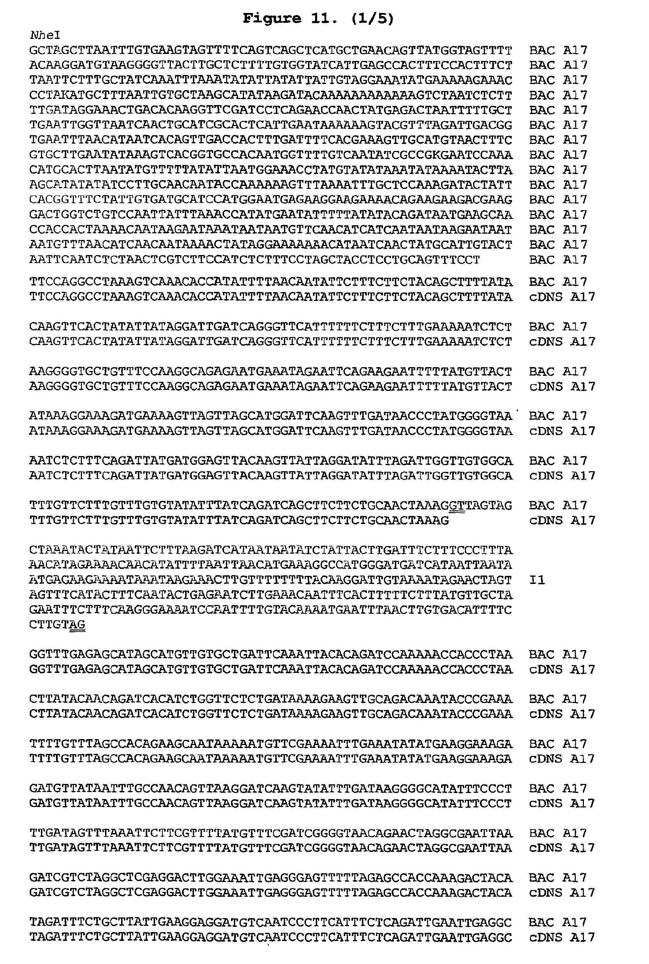

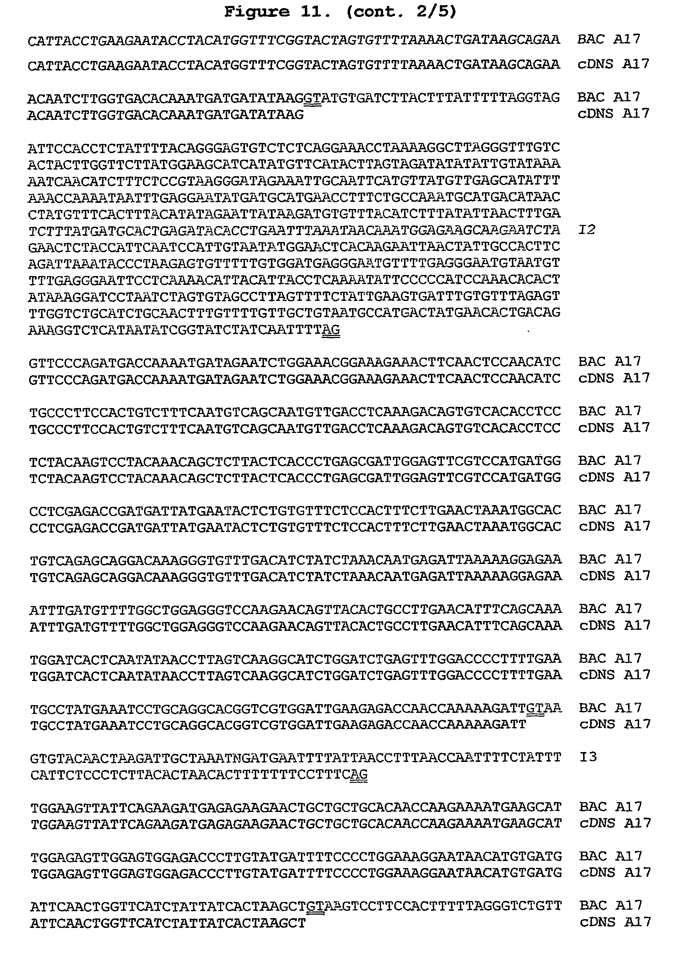

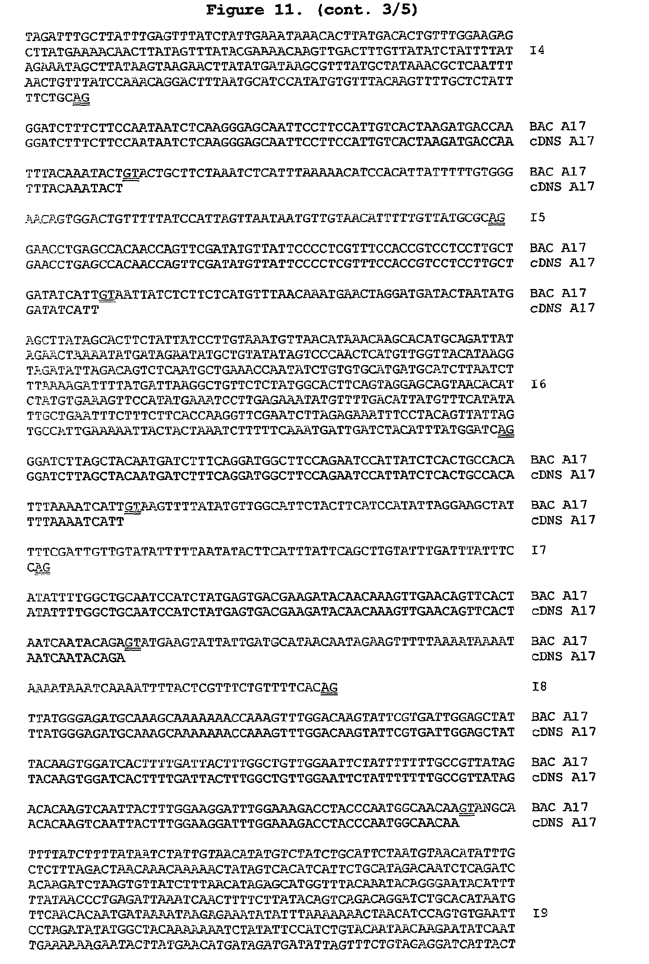

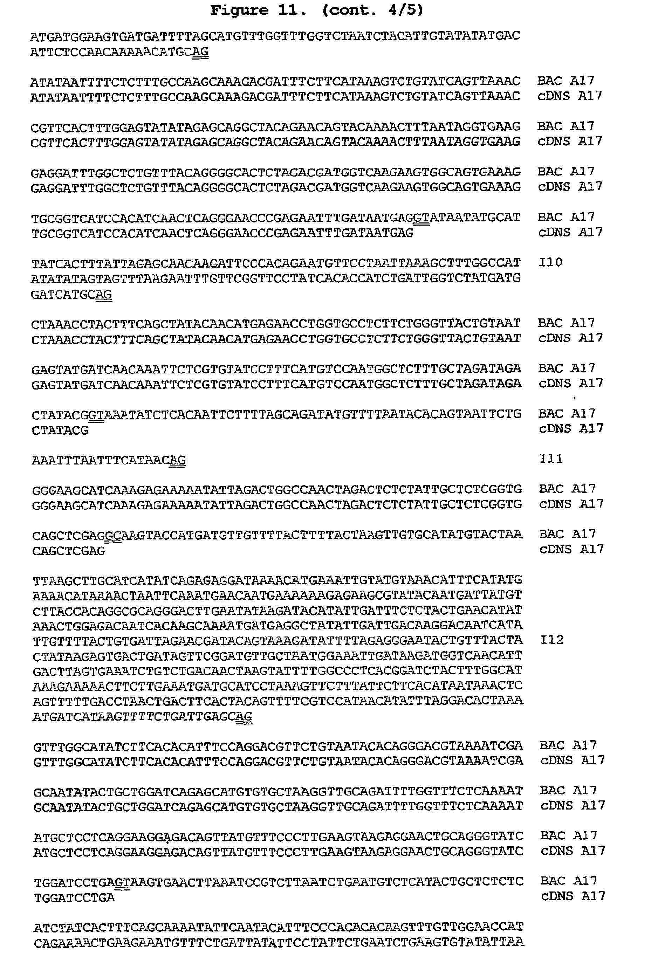

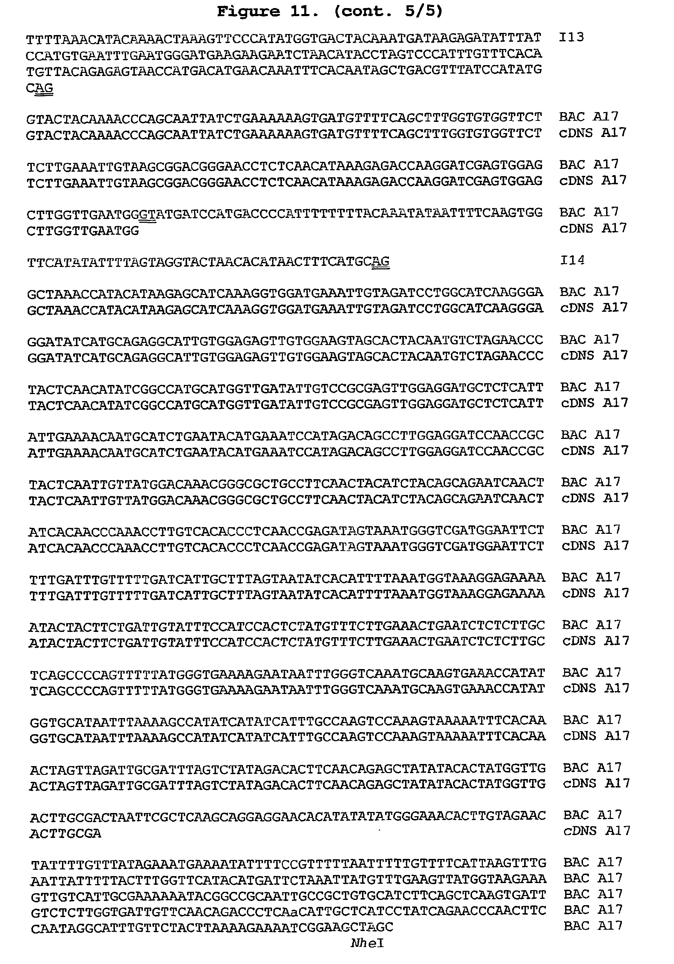

Fig.11. Alignment of the genomic (see Fig. 8.) and cDNA (see Fig. 9.) sequence of the NORK gene from *Medicago truncatula* A17. The genomic sequence (BAC A17) of the NORK gene identified in *Medicago truncatula* A17 was aligned to the cDNA sequence (cDNS A17). For the genomic sequences corresponding to the BAC clones: intron sequences are highlighted by red, the rest are black (Promoter region, exons, 5' and 3' untranslated regions). cDNA sequences are highlighted by blue. Nucleotide sequences corresponding to the first two (GT) and last two (AG) nucleotides in the introns are double underlined. Start codon (ATG) is green, stop codon (TAG) is purple. Nhel sites at the beginning and at the end of the sequences are highlighted by orange.

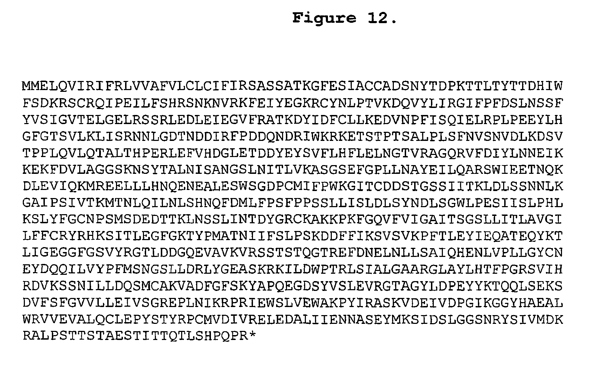

Fig. 12. Deduced amino acid sequence SEQ ID NO:3 from the NORK cDNA of *M. truncatula* A17 plant. (*= stop codon).
Fig.13. Functional representation of the NORK protein by highlighting the characteristic domains.
   A. The NORK protein is 925 aa long. The different structural/functional domains are highlighted by different colors.
   B. More detailed description of the functional domains found in the NORK protein.

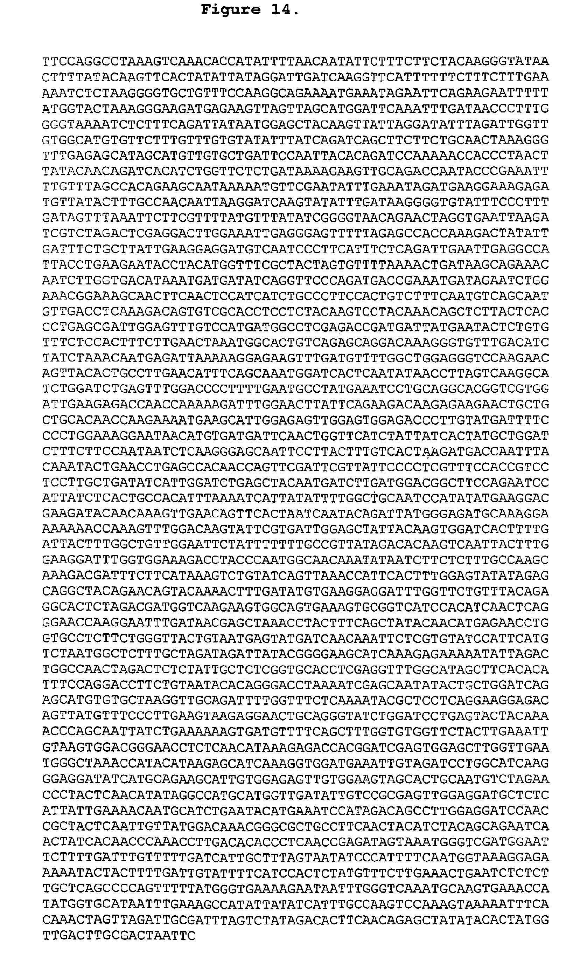

Fig.14. cDNA sequence SEQ ID NO: 4 of the NORK gene for the two Nod⁻ alleles 1 and 2, in plants (NAB1241/6 and NAB701/28) of *Medicago sativa*

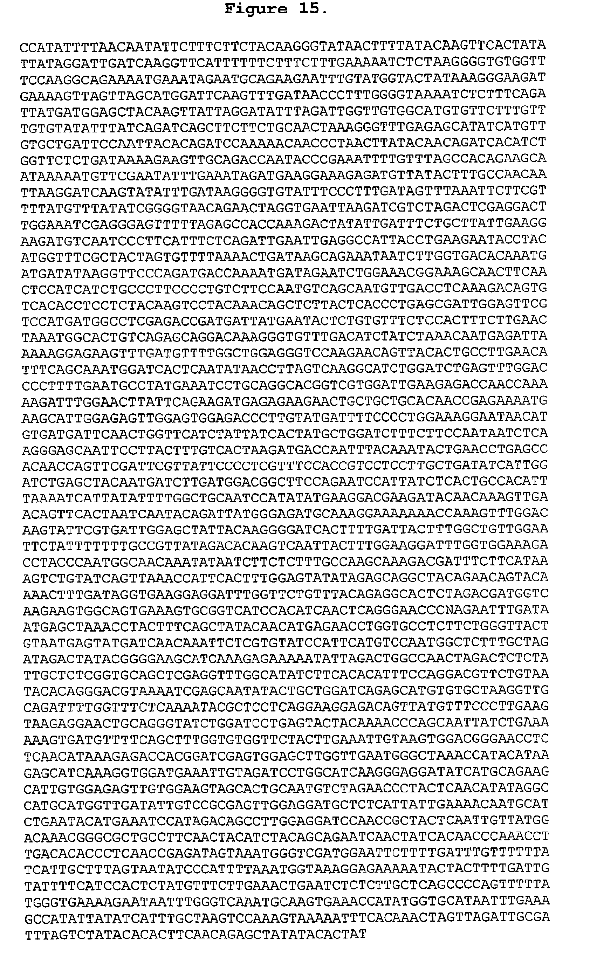

Fig. 15. cDNA sequence SEQ ID NO: 5 of the NORK gene for one of the Nod⁺ alleles (allele 6) in plant (NAB615/28) of *Medicago sativa.*

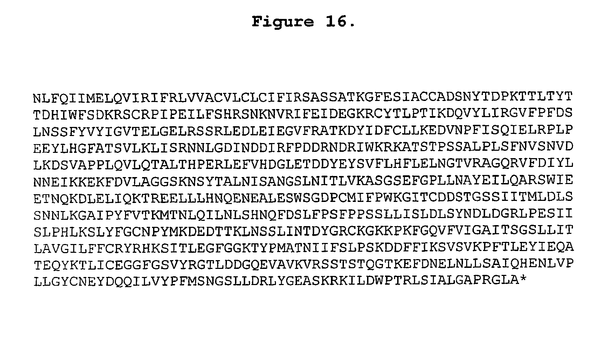

Fig. 16. Deduced amino acid sequence SEQ ID NO: 6 of the cDNA for the NORK gene from Nod⁻ plants (NAB1241/6 és NAB701/28) of M. *sativa.* (* = stop codon).

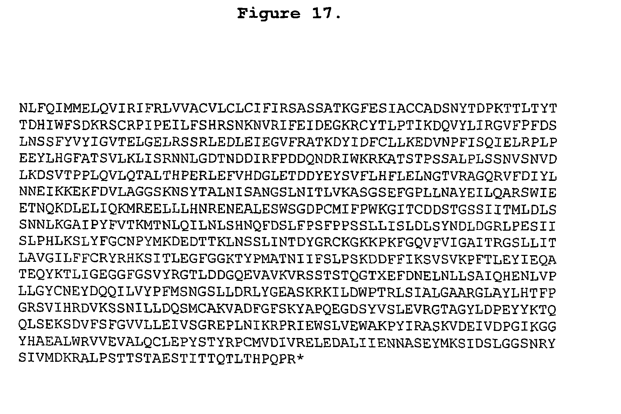

Fig.17. Deduced amino acid sequence SEQ ID NO:7 of the cDNA for the NORK gene from Nod⁺ plants (NAB615/28) of M. *sativa.* (* = stop codon).

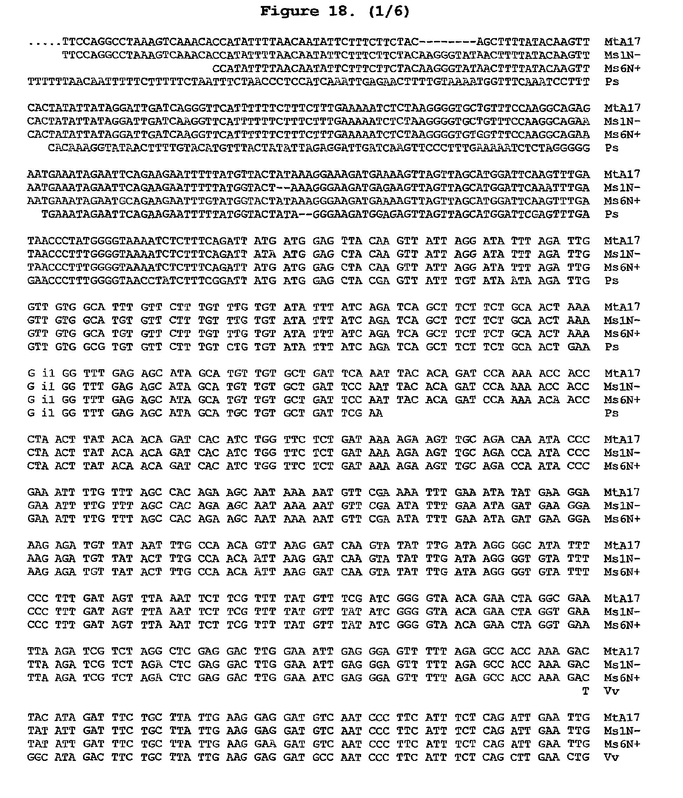

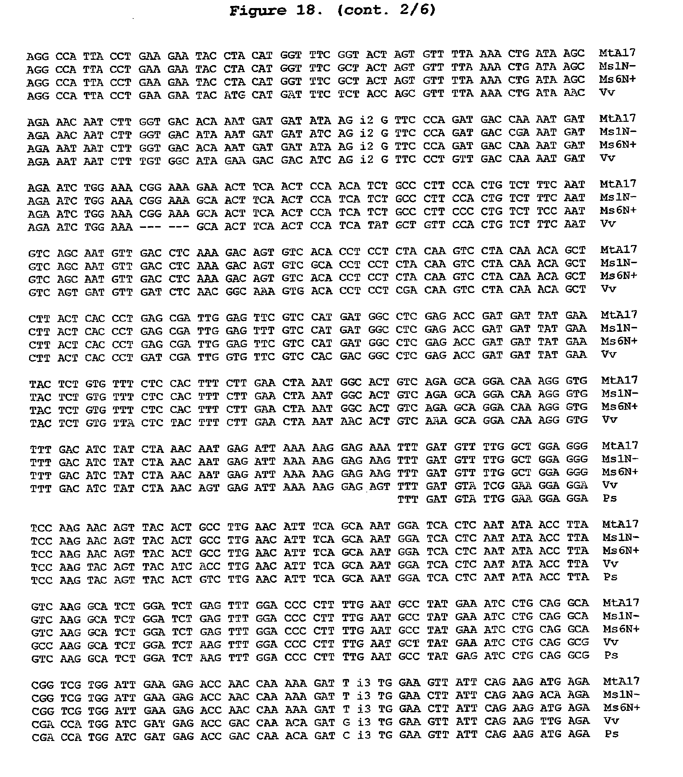

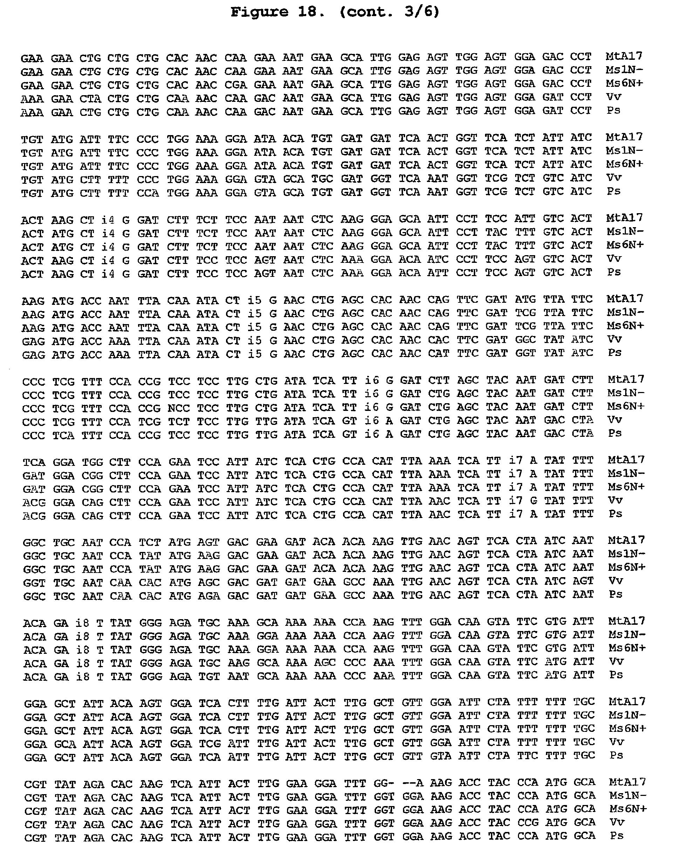

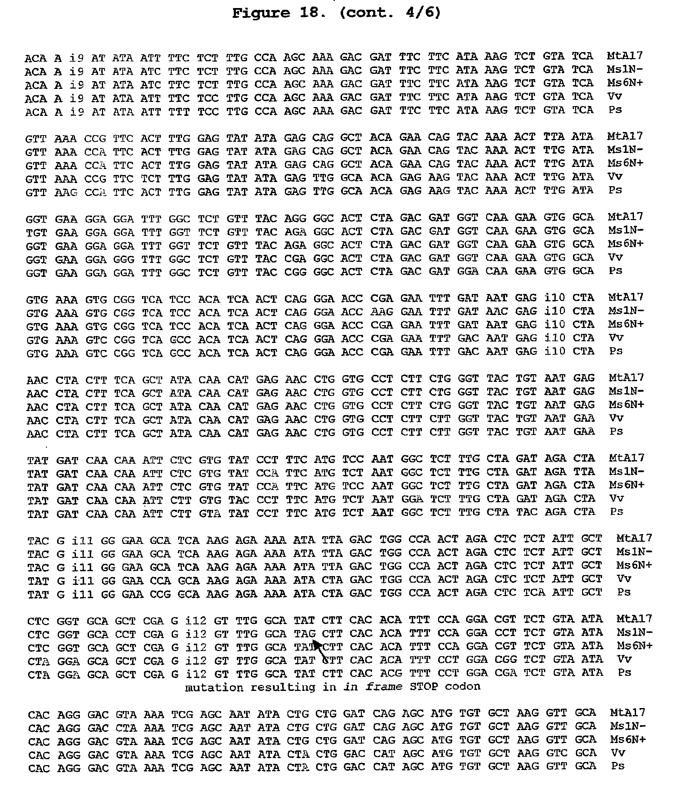

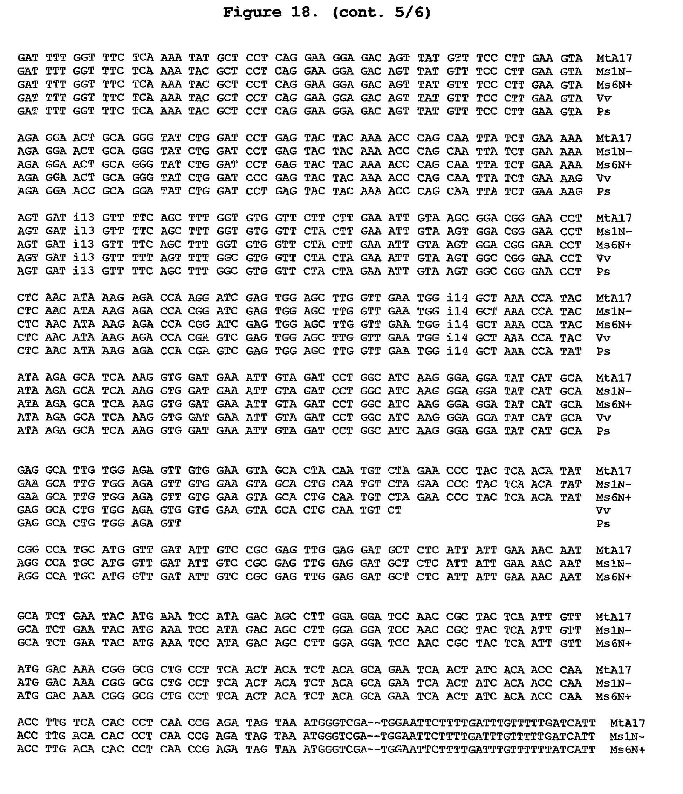

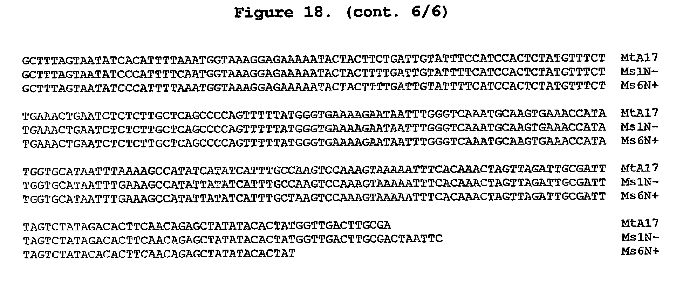

Fig. 18. Alignment of the cDNA sequences of the NORK genes originating from *Medicago truncatula, Medicago sativa, Vicia villosa* and *Pisum sativum. Medicago* alleles are from *Medicago truncatula* A17 (MtA17), *M. sativa* Nod⁻ allele (Ms1N⁻) and Nod⁺ allele (Ms6N+), as well as from *Vicia villosa* (Vv) and *Pisum sativum* (Ps). M. *sativa* Nod⁻ alleles 1 and 2 were identical. Coding regions are grouped in three nucleotide groups according to the genetic code. The position of the introns are highlighted by light purple (i1-i14). Nucleotides are highlighted by red if they are different from the MtA17 sequence. Mutations creating stop codons are shown by arrows. At least two alles are in *V. villosa.* Ambiguous nucleotied are higlighted as: M=A or C; R=A or G; W=A or T; S=C or G; Y=C or T and K=G or T.

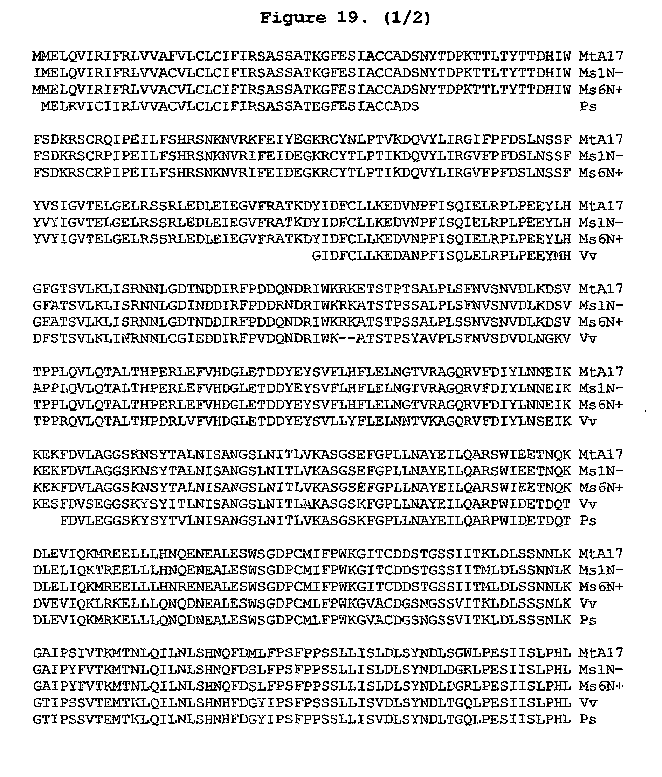

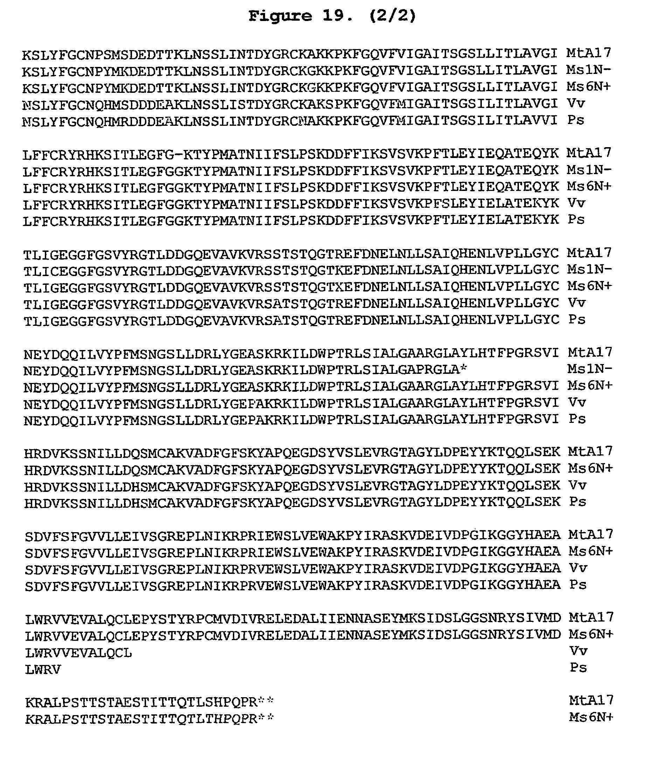

Fig. 19. Alignment of the deduced amino acid sequence of the NORK gene from M. *truncatula* A17, *M. sativa* allele 6 and allele 1 as well as from *Vicia villosa* and *Pisum sativum. Medicago* alleles are from *Medicago truncatula* A17 (MtA17), *M. sativa* Nod⁻ allele (Ms1N⁻) and Nod⁺ allele (Ms6N+), as well as from *Vicia villosa* (Vv) and *Pisum sativum* (Ps). *M. sativa* Nod⁻ alleles 1 and 2 were identical. Amino acid differed form the MtA17 sequence are highlighted by red. At least two alles are in *V. villosa.* The corresponding amino acids are separated by /. Stop codons are highlighted by red. (There are 60 aa in one row).

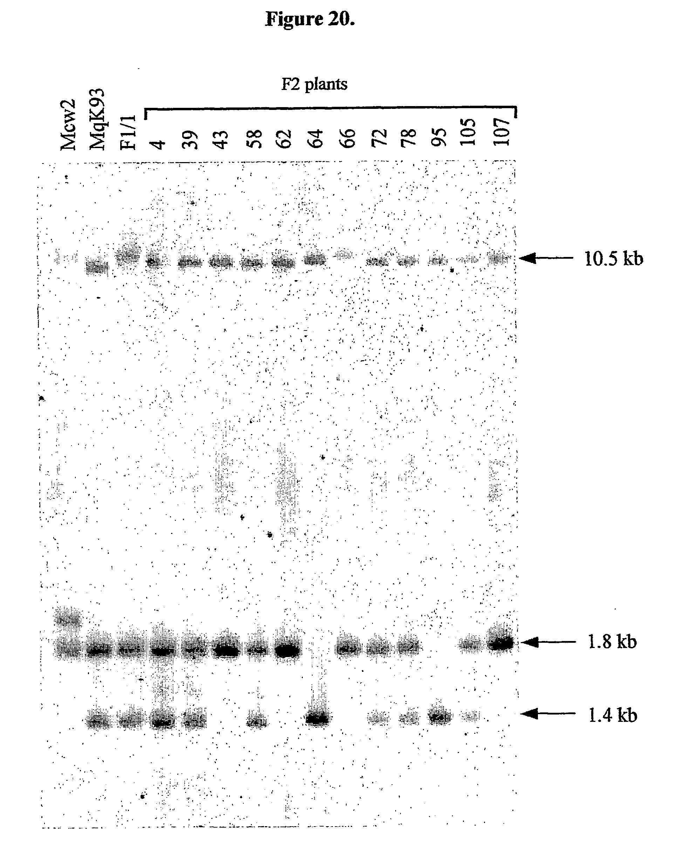

Fig. 20. RFLP pattern of the NORK gene for the individuals from the diploid mapping population to demonstrate the single copy nature of the NORK gene. Hybridization pattern of the of the parents (*M. sativa* ssp. *coerulea* w2 és *M. sativa* ssp. *quasifalcata* k93), the F1/1 and individuals of the F2 generation on a filter containing Dral digested total DNA and hybridized by NORK specific probe (see Example 10.). The DNA used for probe containes two Dral sites, which are probably present in the NORK gene of the diploid species. As a consequence there are at least three hybridizing bands. (Other Dral sites are not excluded in the intron sequences of the genomic DNA). The 212 bp long Dral fragment probably did not transfer to the filter. The 10.5 kb fragments were not polymorphic, as contrast to the small one which is polymorphic. According to the segregation of this fragment there is only one copy of the NORK gene in the diploid alfalfa. Mcw2=*Medicago* sativa ssp.*coerulea* w2; MqK93=Medicago *sativa* ssp. *quasifalcata* k93

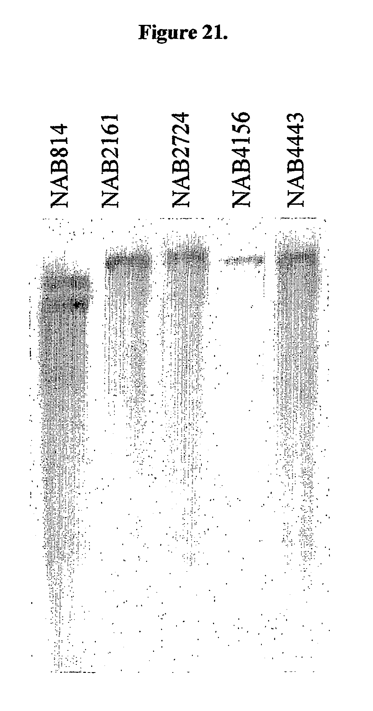

Fig. 21. RFLP hybridization of the NORK gene from tetraploid alfalfa. Hybridization pattern of the Nod⁻/Nod⁺ M. sativa individulas from the segregating population. Total DNA was digested with HindIII, the probe contained only the extracellular part of the cDNA. On the autoradiogram, there is only one strong hybridization band for four individuals, and there are two bands for one individual. Plant NAB814 carries only alleles 5, and 6, respectively. The rest of the plants carry only allele 1 and 2 between the recombination sites (see Example 7.). Allele 5 and 6 can be distinguished according to the size of the two hybridization fragment in NAB814. On the other hand, allele 1 and 2 are indistinguishable (only one hybridizing band). This hybridization pattern indicates that there is only one copy of the NORK gene in the tetraploid alfalfa as well.

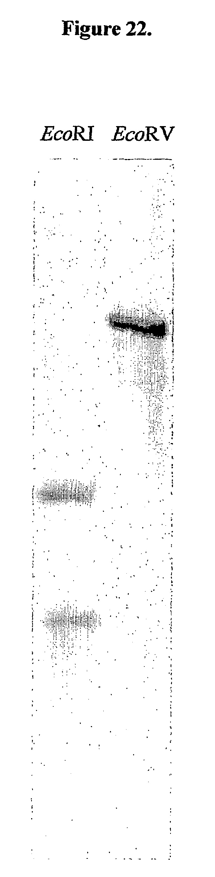

Fig.22. Hybridization pattern of the M. *truncatula* total DNA to the NORK gene. Hybridization pattern of M. *truncatula* total DNA digested by EcoRI and EcoRV enzymes. The probe ("NORK specific") contained only the extracellular part of the cDNA. Hybridization bands represent sequences homologous to the NORK gene.

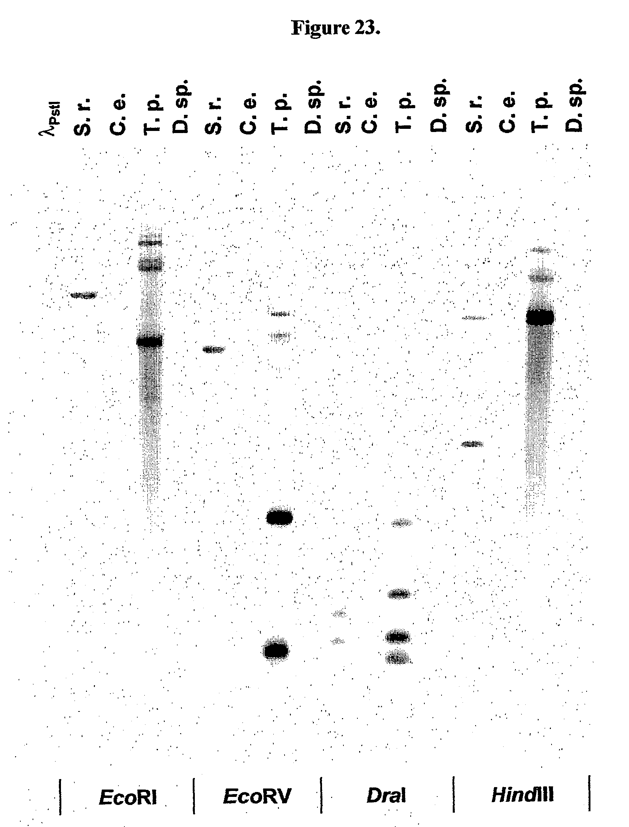

Fig.23. Hybridization pattern of total DNA from different legumes (*Sesbania*, *Cassia, Trifolium* és *Desmodium*) to the NORK gene. The hybridization pattern is shown on filters containing total DNA digested by *Ec*oRI, *Eco*RV, *Dra*I and *Hind*III enzymes. The probe ("NORK specific") contained only the extracellular part of the cDNA (see Example 10.). Hybridization bands represent sequences homologous to the NORK gene. S. r. = *Sesbania rostrata*; C. e. = *Cassia emerginata;* T. p. = *Trifolium pratense;* D. sp. = *Desmodium* species.

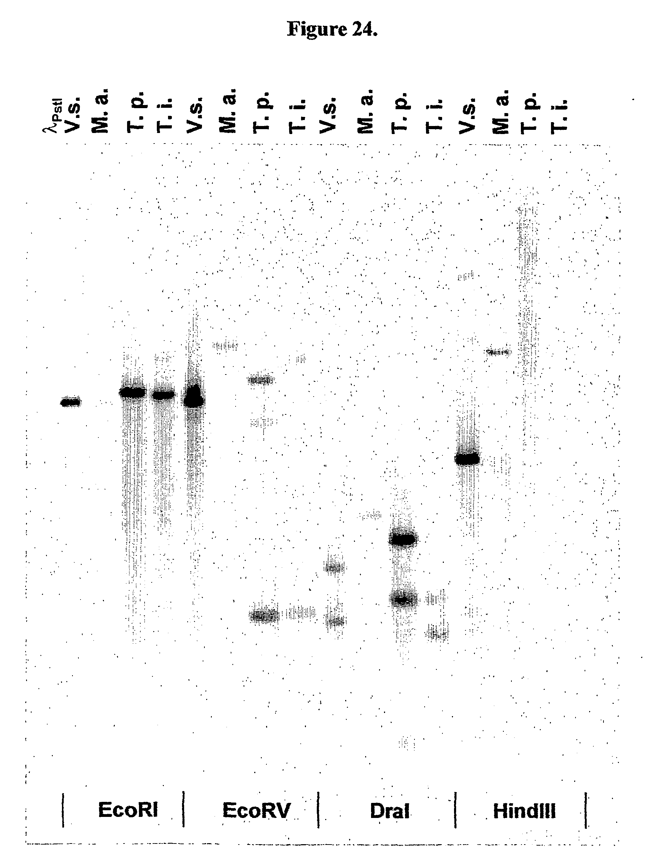

Fig 24. Hybridization pattern of total DNA from different legumes (*Vicia, Trifolium* és *Melilotus*) to the NORK gene. The hybridization pattern is shown on filters containing total DNA digested by *Eco*RI, *Eco*RV, *Dra*l and Hindlll enzymes. The probe ("NORK specific") contained only the extracellular part of the cDNA (see Example 10.). Hybridization bands represent sequences homologous to the NORK gene. V. s. = *Vicia sativa;* M. a. *= Melilotus alba;* T. p. = *Trifolium pratense;* T. i. = *Trifolium incarnatum.*

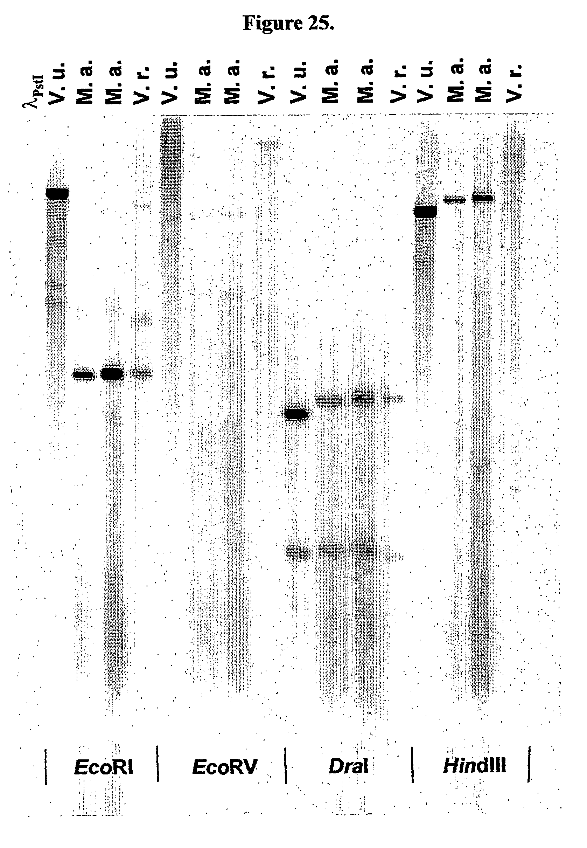

Fig 25. Hybridization pattern of total DNA from different legumes (*Vigna* és *Macroptilium*) to the NORK gene. The hybridization pattern is shown on filters containing total DNA digested by *EcoR*I*, Eco*RV, *Dra*I and *Hind*III enzymes. The probe ("NORK specific") contained only the extracellular part of the cDNA (see Example 10.). Hybridization bands represent sequences homologous to the NORK gene. V. u. = *Vigna unguiculata;* M. a. *= Macroptilium atropurpureum;* V. r. = *Vigna radiata*

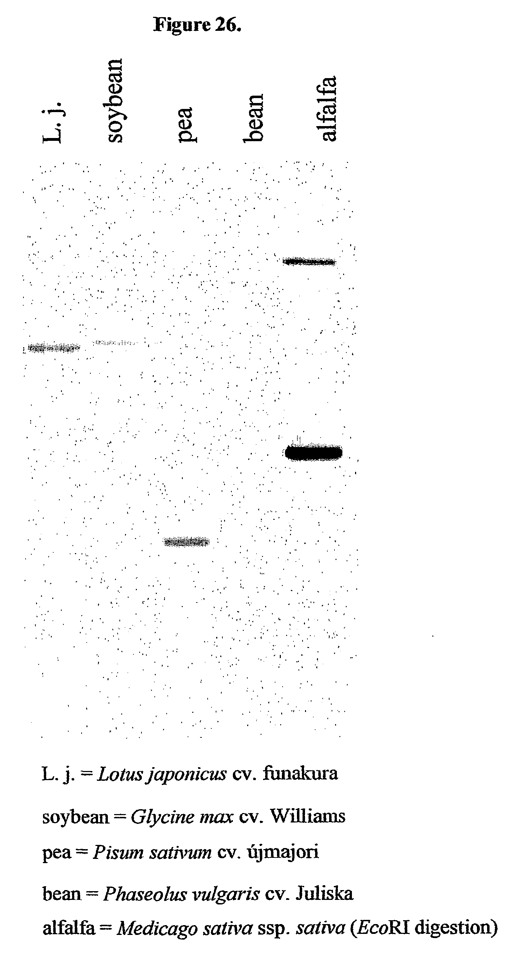

Fig 26. Hybridization pattern of total DNA from different legumes (*Pisum, Glycine, Lotus*) to the NORK gene. The hybridization pattern is shown on filters containing total DNA digested by *Eco*RI enzyme. The probe ("NORK specific") contained only the extracellular part of the cDNA (see Example 10.). Hybridization bands represent sequences homologous to the NORK gene.

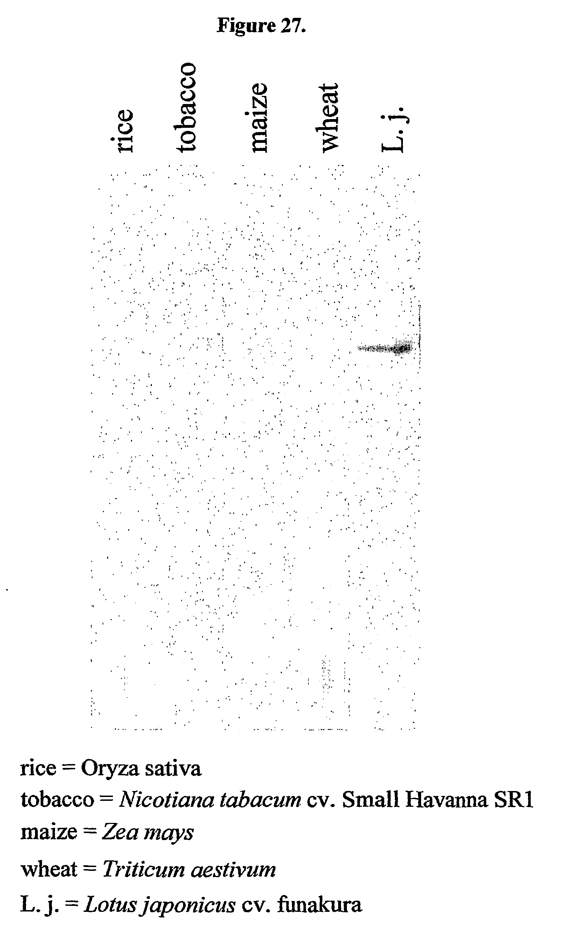

Fig 27. Hybridization pattern of total DNA from different non-legumes (rice, tobacco, wheat and corn) to the NORK gene. The hybridization pattern is shown on filters containing total DNA digested by *Eco*RI enzyme. The probe ("NORK specific") contained only the extracellular part of the cDNA (see Example 10.). Hybridization bands represent sequences homologous to the NORK gene.

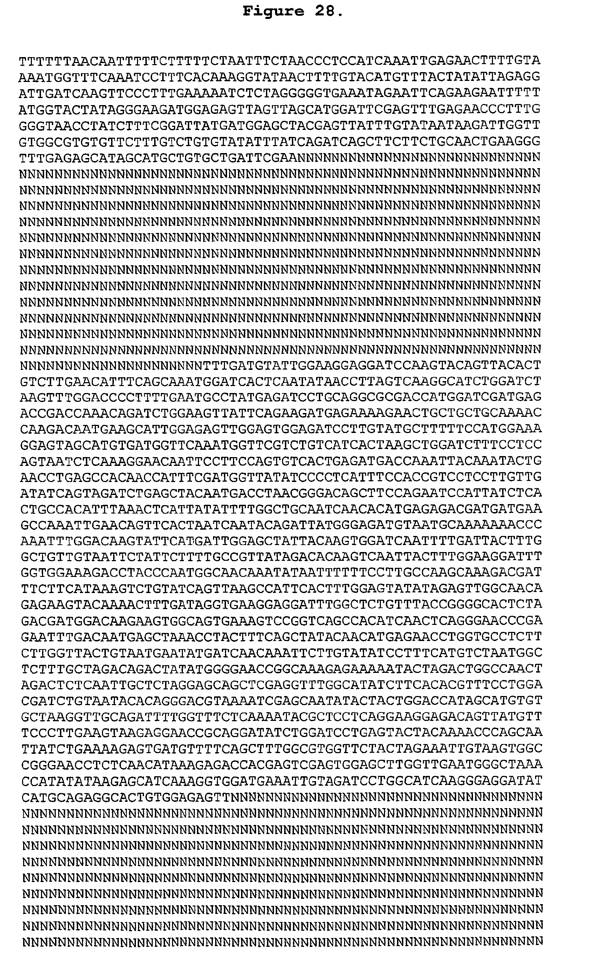

Fig.28. cDNA sequence SEQ ID NO:8 of the NORK gene from *Pisum sativum* cv. Frisson plant. (There are 60 nucleotides in one row).

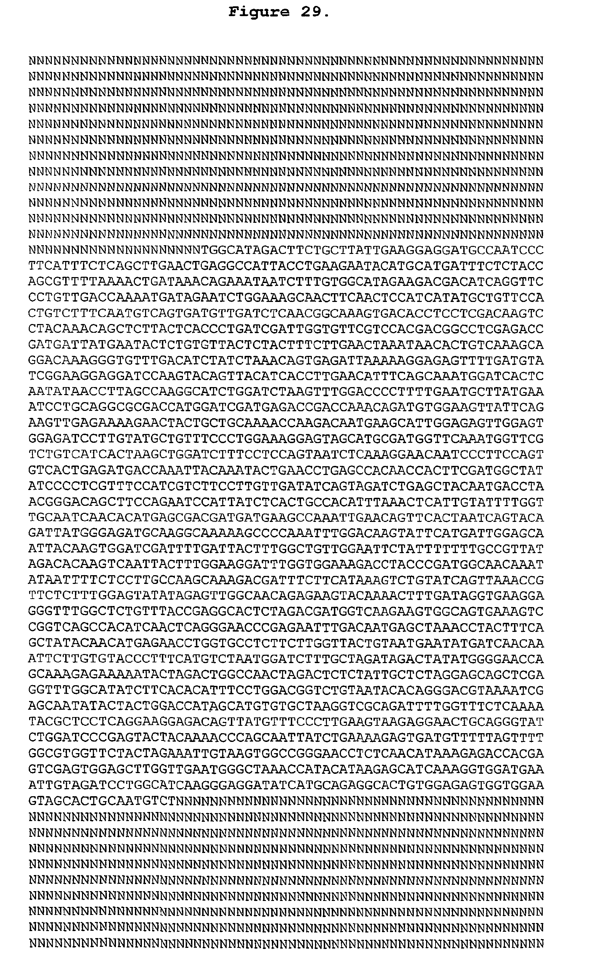

Fig. 29. cDNA sequence SEQ ID NO:9 of the NORK gene from *Vicia villosa* S-1 plant. (There are 60 nucleotides in one row). The nucleotides represented by the two allele of *V. villosa* are highlighted as follows: M=A or C, R =A or G, W=A ro T, S = C vagy G. Y = C or T, K = G or T .

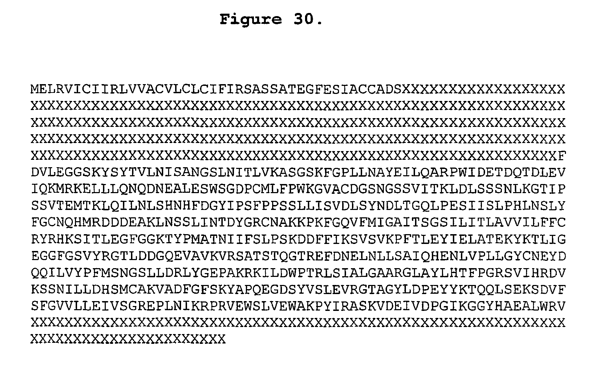

Fig.30. Deduced amino acid sequence SEQ ID NO:10 of the cDNA for the NORK gene from *P. sativum* cv. Frisson plant. Stop codon (*) is highlighted by red. (There are 60 aa in one row).

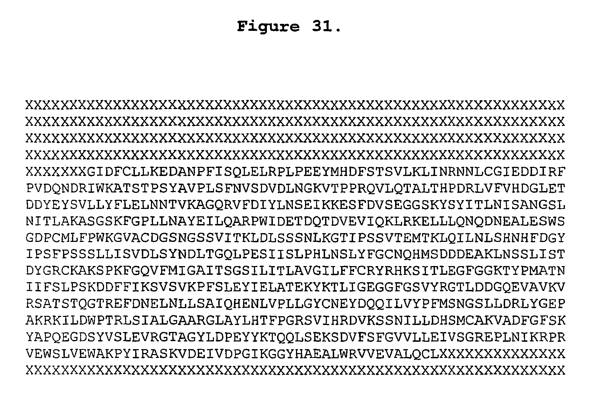

Fig. 31. Deduced amino acid sequence SEQ ID NO: 10 of the cDNA for the NORK gene from *V. villosa* S-1 plant. At least two alles are in *V. villosa.* The corresponding amino acids are separated by /. Stop codon (*) is highlighted by red. (There are 60 aa in one row).

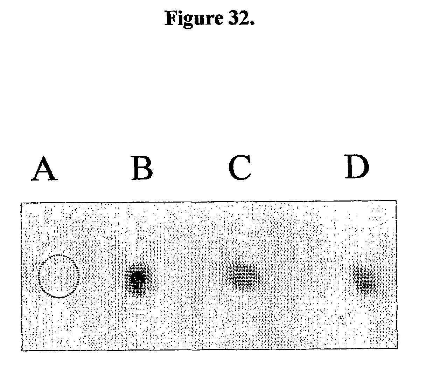

Fig. 32. Antigen-antibody reaction using diluted (1:1000) rabbit serum with cell protein extracts from 10 mg plant tissue. A. control tobacco plant (non-transformed). B. Transgenic plant carrying the MtA17 NORK gene. C. Nod⁺ control alfalfa NAB814. D. Nod⁻ alfafa (MN-1008).

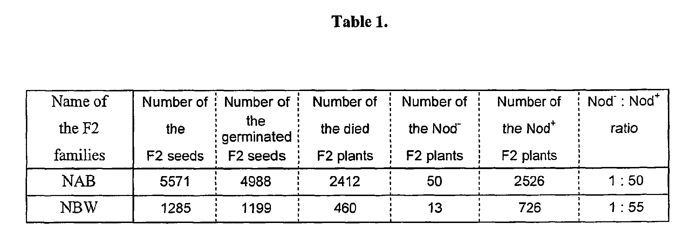

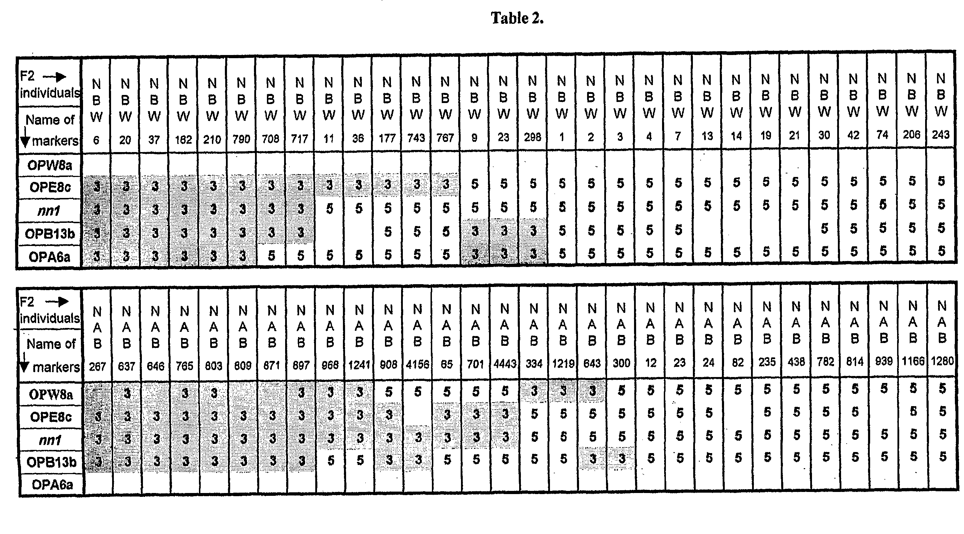

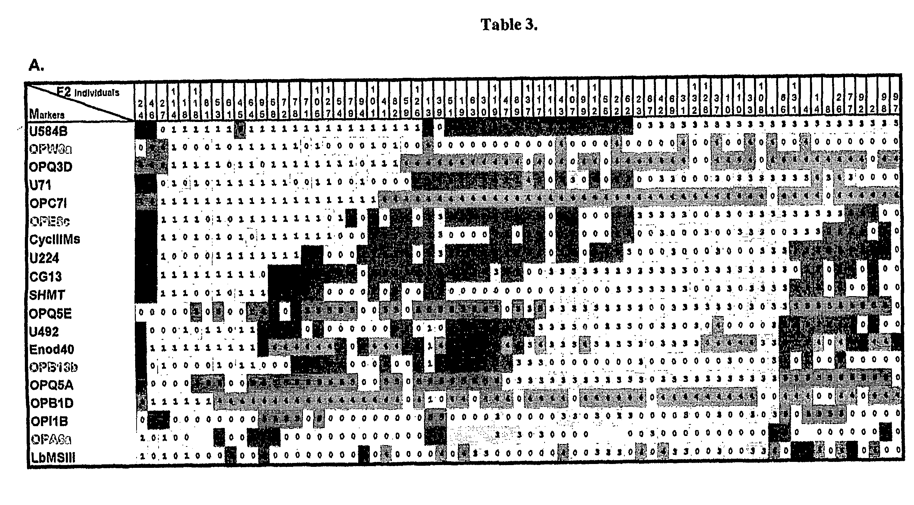

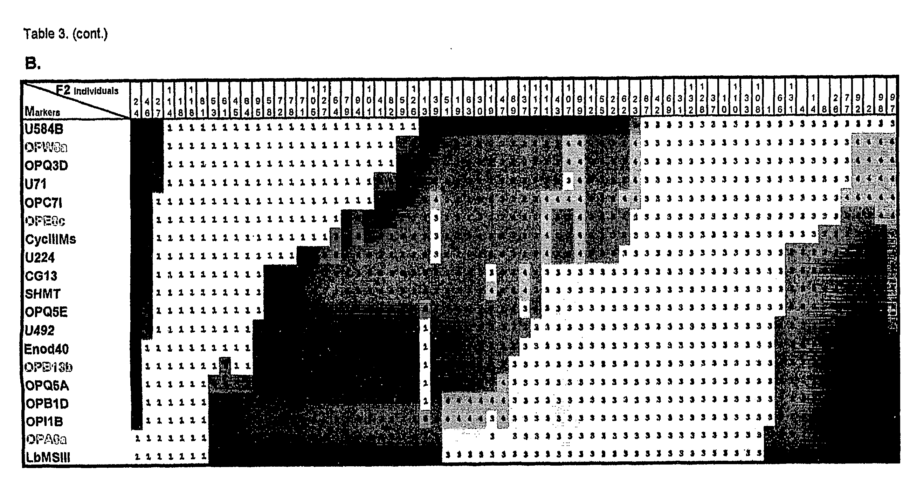

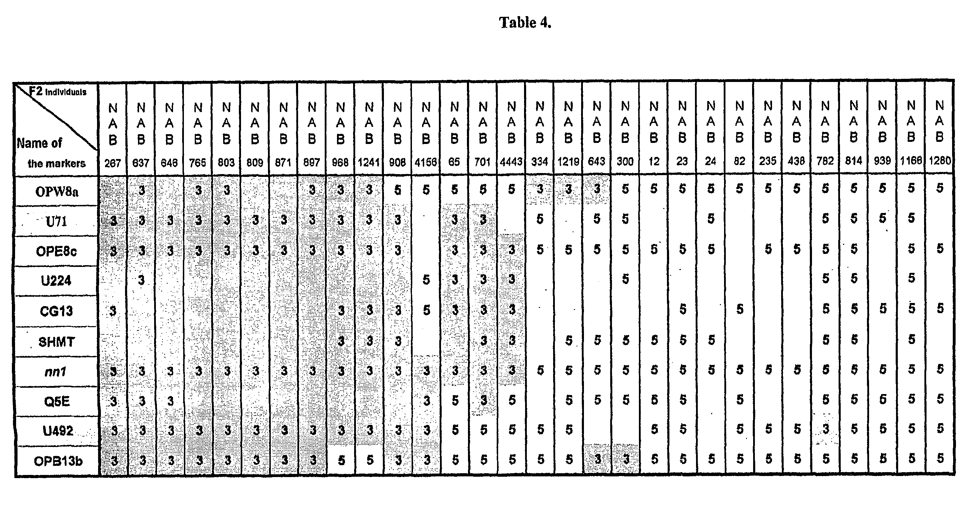

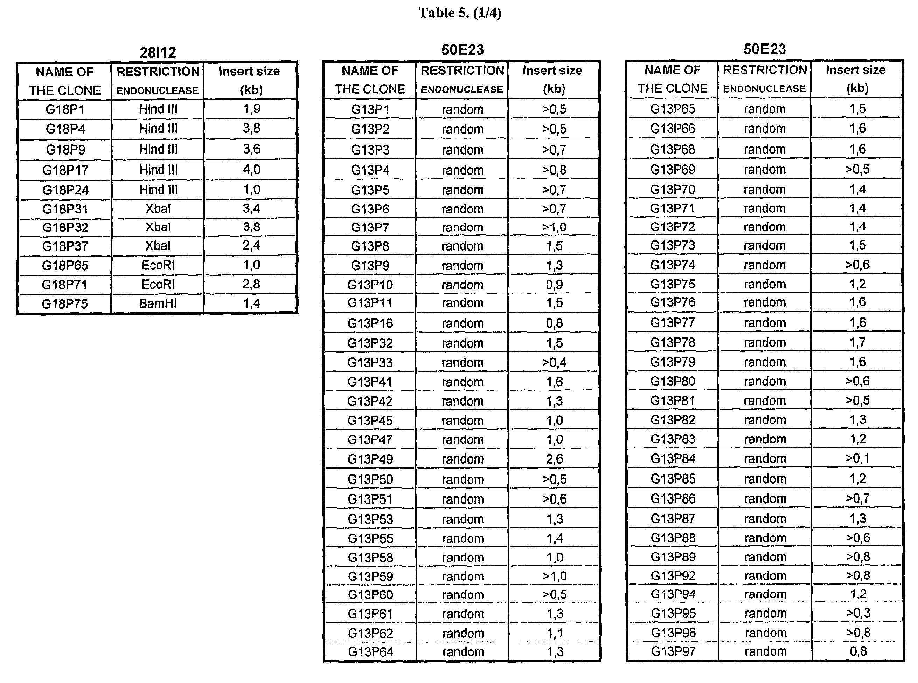

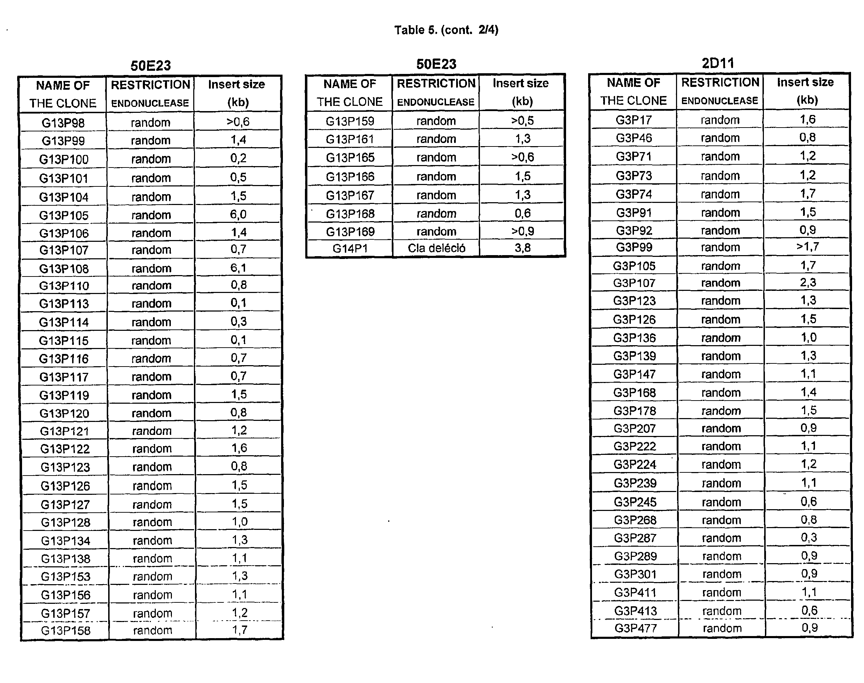

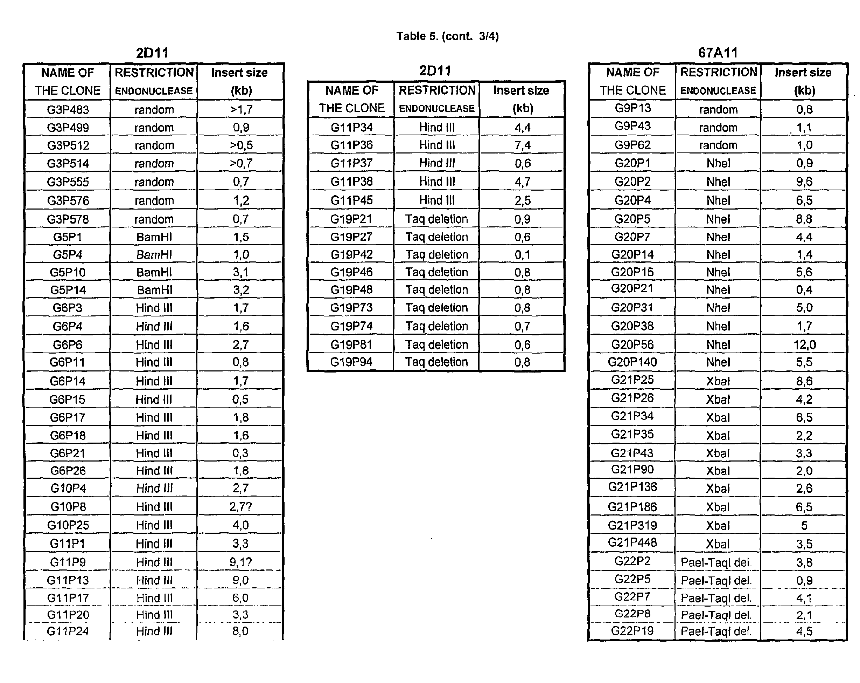

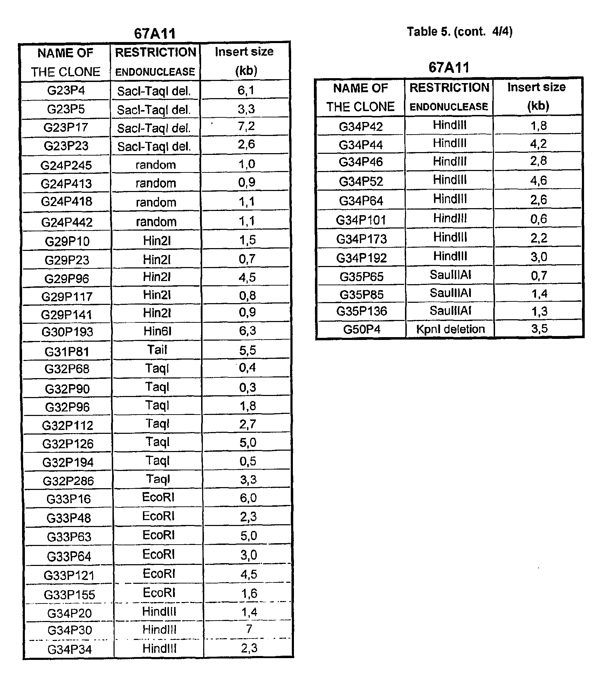

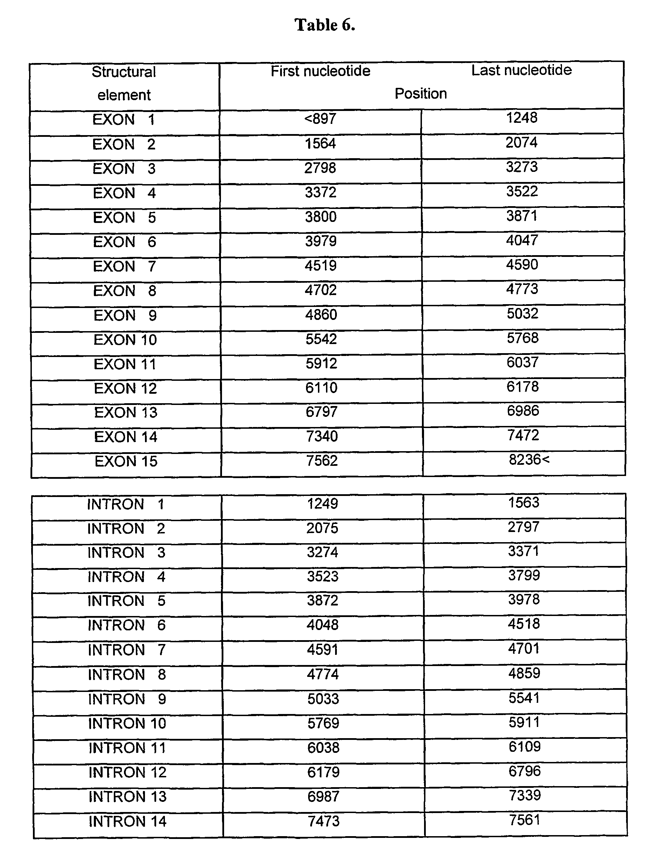

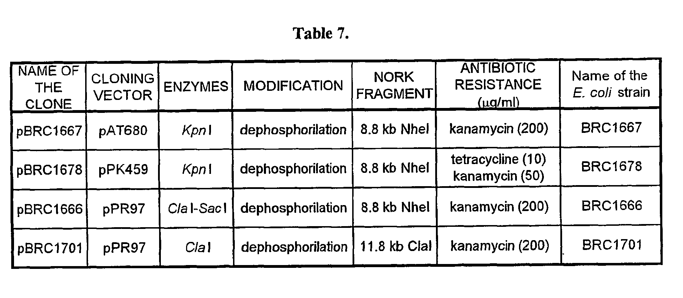

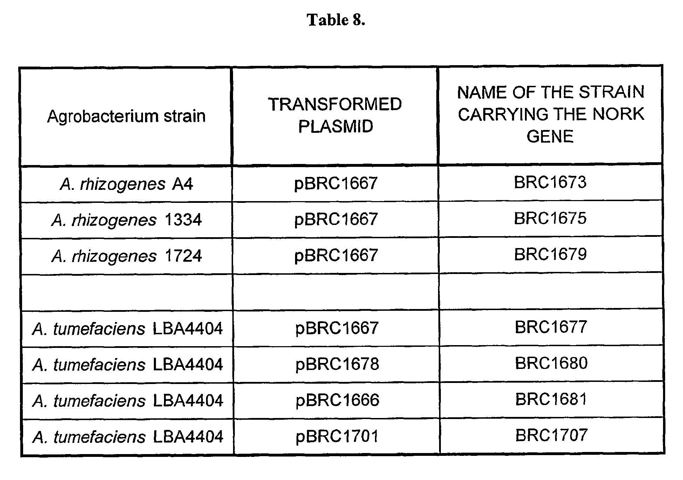

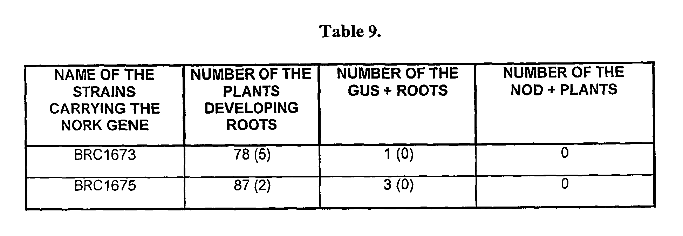

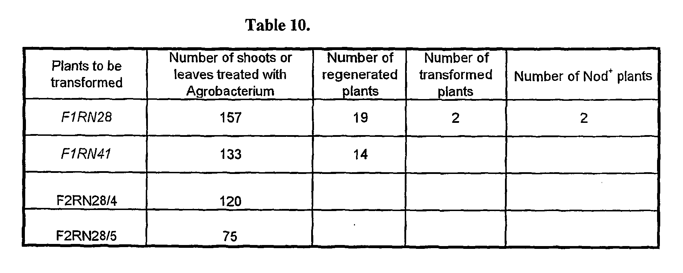

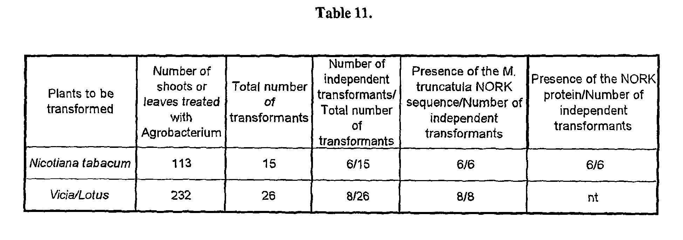

Fig. 33. Multiple alignment of the NSL proteins. Amino acid residues were coloured according to an 80 % consensus. Letters mark the following amino acid classes: h = hydrophobic (ACFGHIKLMRTVWZ) and I = their aliphatic subset (ILV) with turquoise shading; a = aromatic (FHWY) with green shading; + = positive (HKR) with red shading; p = polar (CNEHKNQRST) in red; t = "turn-like" (ACDEGHKNQRST) in green; s = small (ACDGNPSTV) in blue. White letters with dark violet background mark amino acids identical (conserved 100%) in all sequences. Amino acids present in 80% of the sequences are shown by white letter with pink background. Stars indicate the positions where the amino acids of the legume NORKE domain differ significantly from the consensus of other NSL sequences. Mt = *Medicago truncatula,* At = *Arabidopsis thaliana,* Pv = *Phaseolus vulgaris,* Os = *Oryza sativa,* Gm = *Glycine max,* Gos = *Gossypum,* Zm = *Zea mays,* TC = tentative consensus sequences constructed from EST sequences by TIGR (www.tigr.org).

Table 1. Germination ability, viability and nodulation properties of the F2 seeds for the NAB and NBW families. F2 seeds were germinated on 1 % water-agar, after germination seedlings were transferred to tubes containing slant agar (Gibson). After one week plants were inoculated with *S. rhizobium* 41. After 6 weeks and 2 months nodulation was evaluated visually. For detailed description see Example 1.
Table 2. Genotyping the F2 NAB and NBW individuals for markers OPW8a, OPE8c, OPB13b, OPA6a and for nodulation. Total DNA from the highlighted NAB (15 Nod⁻ ; 15 Nod+) and NBW (8 Nod⁻; 22 Nod+) individuals were amplified by OPW8, OPE8, OPB13, OPA6 Operon Primers as described in Example 2. During the amplification reaction fragments (RAPD fragments) corresponding to markers OPW8a, OPE8c, OPB13b and OPA6a either appear (coded by number 5) or not (coded by number 3). Genotypes of the Nod⁺ and Nod⁻ individuals are coded by number 5 or 3, respectively. Genotypes in the table are highlighted according to the concept of colormapping, colors without number are predicted genotypes (see ref. 39).
Table 3. Genotyping the OPW8a, OPE8c, OPB13b, OPA6a markers on the diploid mapping population. Genotyping of the maternal and parternal alleles corresponding to the hybridizing bands were done as follows: maternal homozygous = 1 (yellow); paternal homozygous = 3 (purple); hetrozygous = 2 (green); maternal dominant = 5 (light green); paternal dominant = 4 (blue); missing genotypes = 0 (grey). Colormapping was accordng to Table 3, prediction according to ref. 39. Colormaps show the U584B-LbIII region of linkage group 5 of the diploid *M. sativa* ssp. *coerulea* and *M. sativa* ssp. *quasifalcata* genetic map (40). 3A and 3B highlights the non-predicted and the predicted map, respectively.
Table 4. Genetic mapping of markers U71, U224, CG13, SHMT, Q5E és U492 on the tetraploid alfalfa population. Genotypes of the individuals plants were according to the size of the hybridizing band. If the band was similar to the size of the band from the Nod⁻ parent, the genotype code was 3 (purple). If the band was similar to the size of the band from the Nod⁺ parent, the genotype code was 5 (light green). Genotypes of the Nod⁺ and Nod⁻ individulas are coded by number 5 or 3, respectively. Genotypes in the table are highlighted according to the concept of colormapping, colors without number are predicted genotypes (see ref. 39).
Table 5. List of the sub-clones originating from BAC clones 28I12, 50E23, 2D11 és 67A11. The size of the insert in the sub-clones are also shown. For detailed description see Example 6. kb = kilobase pairs.
Table 6. Position of the exons and introns in the NORK gene according to Fig.9. Numbering starts from the first nucleotid of the Nhel site (see Fig. 8.).
Table 7. Cloning of the NORK gene into plant cloning vectors. The description of the vectors used for plant trasnformation and the details of the cloning is given in Example 12.
Table 8. List of the Agrobacterium strains carrying the NORK gene. The construction of the *Agrobacterium rhizogenes* and *A. tumefaciens* strains used for the two different transformation protocols are given in Examples 12A and 12B, respectively.
Table 9. List of the *A. rhizogenes* strains used for transformation and the transformation frequency. Nod⁻ plants were infected by *A. rhizogenes* strains as described in Example 12B. After emergence of the roots GUS staining was performed and the nodulation ability was tested. The second and the third row in the Table highlights the results obtained for the two transformation methods (combined). In brackets, the results from the second experiment are shown.
Table 10. Summary of the transformation experiments with *Medicago sativa* and *Agrobacterium tumefaciens.*
Table 11. Summary of the transformation experiments with *Nicotiana tabcum, Vicia villosa* and *Agrobacterium tumefaciens.*

### BEST MODE OF CARRYING OUT THE INVENTION

Hereinafter the invention is demonstrated by examples. These examples do not restrict the protected domain of the invention they are only for demonstration purposes.

### Example 1

### Genetic crosses and the analysis of F2 progeny of the NAB and NBW families

The tetraploid (2n=4x=32) *Medicago sativa* MN-1008 mutant line (28) has been provided us by Prof. D. Barnes (University of Minnesota, MN, USA) (primarily called MnNC-1008 (NN), but generally abbreviated as MN-1008), is unable to form symbiotic nodules in the presence of compatible bacteria. The mutant plant emerged spontaneously by means of crosses between cultivated alfalfa populations and subsequent self-matings (27). One individual (referred in laboratory notebook as MN-1008/17) of the purple flowered MN-1008 mutant alfalfa line has been used as maternal parent in genetic crosses. The paternal parent was one individual of the M. *sativa* cv. Nagyszénási (referred as MsNa/5) alfalfa population. Since the self-pollinating efficiency of the MN-1008/17 maternal parent was between 10-20 %, its flowers had to be sterilized (emasculated). Prior to crosses the so-called banner petals of the matured flowers of the maternal parents were trimmed by scissors and followed by splitting open of the flowers by squeezing them to avoid the anthers to hit the stigma. Then the flowers were immersed in 51 % ethanol for 3-4 sec and then washed in water. After the flowers dried the stigma was covered with the pollens from the male parent. The efficiency of the ethanol treatment was checked in control experiments: the self-pollinating efficiency of the treated flowers in the absence of foreign pollen was about 0.5 %.

The F1 progeny seeds coming from the above described cross were vernalized at 5-8 °C for two weeks, then germinated and planted out first into plastic pots with diameter 8 cm, and after the plants gained strength into pots with diameter 25 cm containing flower-soil termed NT. Plants are maintained to bloom in greenhouse at 22-25 °C with photoperiod of 16 hours lighting and 8 hours dark. The flowers were self-pollinated one by one manually by squeezing the base of the flowers (tripping). At this time the stamen knocked against the stigma and the pollens got into the stigma from the anthers. After self-pollination the F2 ovules appeared on the plants from which the matured seeds developed. The F2 generation were begun to nurse in symbiotic test to determine their symbiotic phenotype. The matured seeds were vernalized and their surface were sterilized by rinsing them in 70% ethanol for 30 sec and treating with 0.1 % HgCl₂ solution for 5 min followed with rinsing in sterile distilled water for five times. Then the seeds were swelled in sterile water for 8 hours. After swelling, the seeds were placed in equable distances from each other on the surface of plates in Petri dishes containing 1% agar in distilled water. The dishes were incubated upside down in dark to prevent the emerging roots to grow into the agar medium. Next day the seedlings with roots ofabout 0.5 - 1.0 cm length were then placed on the surface of 1% agar slants with nitrogen-free Gibson medium (41) and the plants were grown at 22- 25 °C with 16-h photoperiod (10 000 Lux). The seedlings were inoculated with *Sinorhizobium meliloti* 41 bacteria culture suspended in 1ml Gibson medium after 5-7 days of transfer.

The S. *meliloti* 41 bacteria culture were prepared for inoculate .as follows. An inoculation loop of S. *meliloti* 41 strain (42, 43) stored at -80 °C were inoculated into test tube containing 5 ml YTB medium (44). The bacteria culture was rotated in test tube for 24 hours. The grown-up culture were inoculated into a 500 ml flask containing 100 ml YTB medium and the culture were grown up to stationer phase for about 24 hours. The concentration of the bacteria suspension grown-up to stationer phase was between 1-3 x 10⁹ cells/ml. The bacteria were collected by centrifugation (in centrifuge Sorvall RC5 with GSA type angle rotor at 4 °C with 10 krpm for 10 min) under sterile conditions. The supernatant was discarded and the cells were resuspended in 100 ml Gibson medium (41). The concentrated bacteria culture was diluted in 100-fold in Gibson medium before inoculation. After dilution the concentration of the bacteria was 1-3 x 10⁷ cells/ml. The nodulation phenotype of the plants was scored 6 weeks after inoculation, afterwards plants were potted into soil. Plants were removed gently out of the pots and were evaluated once more for the presence or absence of nodules 2 months later (secondary control of nodulation). The plants were planted back into pots and grown further. The flowers of the F2 individuals selected later by genetic analysis (determination of the genotypes for different molecular markers) were self-pollinated as described above to generate F3 seeds which were tested for symbiotic nodulation.

After the crosses 62 F1 seedlings were planted and grown up. Forty-four of these plants produced sufficient flowers and F2 seeds (at least 50-100 seeds/individuals) after self-pollination to test their viability and analyze the segregation of symbiotic nodulation phenotype. Two F1 plants (NAB and NBW) producing the most vigorous F2 progeny in which the Nod⁻ phenotype could be evaluated were selected for further experiments. The germinating, viability and nodule forming characters of NAB and NBW F2 seeds are listed in Table 1. The segregation ratio of the Nod⁻ and Nod⁺ characters was between 1:50 and 1:55 that is comparable to the segregation ratio of a single, recessive character in a tetraploid F2 segregation population (theoretical value is 1:35).

Composition of plates containing 1 % agar in distilled water:
10 g Bacto agar
1 liter distilled water
Sterilization for 30 min at 121 °C

Composition of flower-soil termed NT:
1 part Florina flower-soil (commercially available)
1 part peat (commercially available)
1 part sand from river Maros (commercially available)

Composition of YTB medium:
1 % Bacto Tryptone
0.1 % Yeast extract
0.5% NaCl
1 mM MgSO₄
1 mM CaCl₂

### Example 2

### Identification of linked RAPD markers to the nn₁ gene of the Nod⁻ MN-1008 mutant plant using the NAB and NBW F2 families

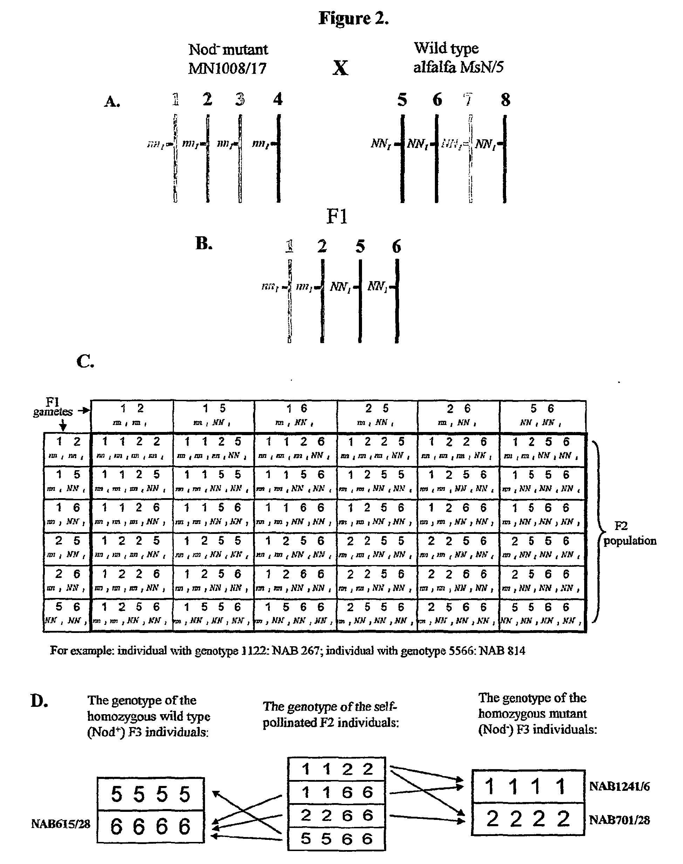

The identification of tightly linked markers to the *nn₁* gene, that is to the Nod⁻ character was performed with the so-called Bulked Segregant Analysis method (45). To perform the screen five Nod⁻ and five Nod⁺ plants were selected from the NAB and NBW families, respectively (Nod⁻ NBW plants: NBW6, NBW20, NBW37, NBW162, NBW790; Nod⁺ NBW plants: NBW9, NBW23, NBW74, NBW440, NBW718; Nod⁻ NAB plants: NAB65, NAB267, NAB637, NAB701, NAB908; Nod⁺ NAB plants: NAB26, NAB82, NAB139, NAB421, NAB814). The analysis of two F2 families enhanced the possibility to identify more linked markers. Since alfalfa is an outcrossing plant, the genome is heterozygous carrying different alleles for the genes. Analysing the inheritance of the alleles in a tetraploid population (as shown in Figure 2.) demonstrated that NAB F1 individual contained two Nod⁻ alleles (indicated as 1 and 2) and two Nod⁺ alleles (indicated as 5 and 6), while it did not carry the two other Nod⁺ alleles (indicated as 7 and 8). As it turned out later, the analysis of the NBW family allowed us to detect polymorphism of these Nod⁺ alleles (indicated as 7 and 8) and to identify additional linked molecular markers.

Total DNA was isolated from young leaves of the Nod⁻ and Nod⁺ plants using the QIAGEN Plant DNA Isolation Kit (DNeasy Plant Mini Kit; QIAGEN Inc. - USA; 28159 Avenue Stanford; Valencia CA 91355) according to the supplier's instructions. The concentration of the DNA samples were determined by spectrophotometer (OD₂₆₀= optical density measured at 260 nm) (1 OD₂₆₀ = 50 µg/ml DNS). The DNA samples were diluted to a concentration of 5 ng/µl, and the DNA samples of five Nod⁻ and Nod⁺ plants were mixed, to produce the Nod⁻ and Nod⁺ homozygous groups. The mixed DNA samples of the homozygous groups were used as templates in polymerase chain reactions (PCR): 12.8 µl sterile distilled water, 5 µl DNS, 2.5 µl 25 mM MgCl₂, 2.5 µl 10 x Taq polymerase buffer (Zenon Kft, H-6720. Szeged, Berzsenyi u. 3.), 2 µl Operon Primer (2.5 pmol/µl), and 0.2 µl Taq DNS polymerase (5 U/µl) (Zenon) were pipetted into PCR tubes of 500 µl volume. The PCRs were carried out in 40 cycles with the following steps: 5 sec at 94 °C, 1 min at 37 °C and 1 min at 72 °C. After completion of the PCR amplification, 5 µl AGE (AGE = 20 % sucrose, 0.1 % bromophenol blue, 0.1 % xylene cyanol) stain was added to the samples and then the amplified products were separated in 2 % agarose gel (Sigma Low EEO) of 15 x 20 x 0.4 cm with 2 V/cm voltage for three hours in 1 x TEA (40 mM Tris, pH 7.8, 50 mM EDTA, 50 mM acetic acid) buffer. After separation of the fragments, the gel were stained in 0.1 % ethidium bromide solution and exposed to UV light to take pictures. The first 520 10-mer primers of Operon Primer set (Operon Technologies, Alameda, Ca, USA) with different nucleotide composition were tested to amplify of the DNA of the two homozygous bulks for both the NAB and NBW families. The PCR amplifications with these short primers resulted in fragment pattern typical for the given primer. The detected differences in the pattern (polymorphism) could be used as RAPD molecular markers (46). As a result of the tests of the primers of the Operon Primer set, several amplified fragments could be identified that were amplified on the bulk of the Nod⁺ DNA but not on the bulk of the Nod⁻ DNA. Four out of them were analysed thoroughly: OPA6a (identified in NBW family), OPW8a (identified in NAB family), OPE8c és OPB13b (identified in both families). The sizes of the amplified products identified in the bulks of the Nod⁺ plants were as follows: OPA6a, about 650 bp; OPW8a, about 300 bp; OPB13b, about 850 bp; OPE8c, about 480 bp. Afterwards the the individuals DNAs were re-tested in PCR to identify the genotypes. In addition other individuals from the families (15 NAB and 8 NBW Nod⁻ plants, as well as 15 NAB and 22 NBW Nod⁺ plants) were tested for the same four RAPD markers. The result of the genotyping (thegenotypes) was displayed in color-map (39) in Table 2. The results presented in the table demonstrated unambiguously the linkage between the *nn₁* gene and the four RAPD markers and determined the genetic order of the markers.

For fine mapping, the NAB family has been selected since it was the largest F2 population. The F2 individuals with diallelic configuration have been selected from this family to produce F3 individuals by self-mating (see Figure 2.). Individuals with homozygous genotypes for the different alleles were searched by determination of their genotypes. Three F3 plants were selected: the plant designated as NAB1241/6 that is Nod⁻ and contains the alleles labelled with no. 1 in homozygous configuration, the Nod⁻ plant designated as NAB701/28 containing the alleles labelled with no. 2 in homozygous configuration around the genomic region of the mutation and the Nod⁺ plant designated as NAB615/28 carrying the alleles labelled with no. 6 in homozygous configuration. No individuals could be detected containing homozygous alleles labelled with no. 5 in this region.

### Example 3

### Genetic mapping of OPW8a, OPE8c, OPB13b és az OPA6a RAPD markers on the linkage map of diploid alfalfa (M. sativa)

In order to determine the map position of the *nn₁* gene on the linkage map of alfalfa, the closely linked RAPD markers (markers OPW8a, OPE8c, OPB13b and OPA6a) were mapped on the genetic map of diploid alfalfa (40). To execute this experiment, the genotypes of the individuals of the diploid segregation population had to be determined. DNA fragments were amplified as described above using the OPW8, OPE8, OPB13 and OPA6 primers, respectively and the total DNA of NAB814 and NBW9 plants as template DNA. After separation; the DNA fragments corresponding to markers OPW8a, OPE8c, OPB13b and OPA6a were isolated from the agarose gels, purified by the QIAGEN Fragment Isolation Kit (QIAEX II Gel Extraction Kit; Cat.No. 200 51 QIAGEN Inc). and their concentration determined by spectrophotometer (determination of OD at 260 nm). Fifty ng of the fragments were labelled with deoxycitidine-5'-[alfa-³²P] triphosphate (Code: FP-205, Izotóp Intézet Kft., 1121 Budapest, Konkoly Thege Miklós út 29-33.) using the Ready-To-Go DNA labelling (-dCTP) Kit (cat. number: 27-9240-01; Amersham Pharmacia Biotech Inc, Piscataway, NJ, USA) according to the suppliers' instructions. The HybondN⁺ nylon filters (cat. number: RPN3003B, Amersham Pharmacia Biotech Inc, Little Chalfont, Buckinghamshire, England) carrying the restriction enzyme digested DNA of the individuals of the diploid mapping population were hybridized with the hybridization probes prepared as described above. The filters were prepared by the following method: total DNA was isolated of the individuals of the F2 mapping population (47) as described in Example 2. Aliquots of 20 µg DNA were digested with *Dra*I*, Eco*RI, *Eco*RV and *Hind*III restriction enzymes (Fermentas AB, Vilnius, Lithuania), respectively and the restriction fragments were separated in 0,8 %-os agarose gels as described in Example 2. The fragments were transferred onto HybondN⁺ nylon membranes according to the suppliers' instructions and the filters were hybridized at 60 °C for 16 hours in CG buffer published by Church and Gilbert (48). After hybridization, washing was carried out three times for 20 minutes each at the temperature of the hybridization in the following solutions: 1.) 0.1% SDS and 1xSSPE (49); 2.) 0.1% SDS and 0.5xSSPE; 3.) 0.1% SDS and 0.1xSSPE. Autoradiography of the filters was at -80 °C using the Sterling Diagnostic (Sterling Diagnostic Imaging Inc., Newark, DE 19714 USA) X-ray films or the filters were exposed in the exposure casette of the phosphorimager scanning instrument (Phosphorimager^{™} 445 SI; Molecular Dynamics Inc., Sunnyvale, CA, USA) and after the scanning of the screens the results were analysed by the ImageQuant computer software. The hybridizing fragments corresponding to the alleles of the OPW8a, OPE8c, OPB13b and OPA6a markers were identified as RFLP bands and the genotypes of the individuals were determined. The map position of the markers were determined by the colormapping procedure (39): all of the four markers were mapped in linkage group 5 (LG5) close to the U224 marker. The genotypes of the OPW8a, OPE8c, OPB13b and OPA6a markers of the individuals in the diploid mapping population and the results of the genetic mapping are presented in Table 3.

### Example 4

### Genetic mapping of the RFLP markers between U584B and LbIII markers in the tetraploid population segregating the nn₁ gene

To ensure that the map position identified in the diploid population with molecular markers linked in fact to the *nn₁* gene (to the Nod⁻ pnenotype) the map position of some markers mapped previously in this region (between the U584B and Lblll markers) were determined in the tetraploid alfalfa genetic map. The segregation of five RFLP markers (U71, U224, CG13, SHMT and U492) and a specific PCR marker (marked with Q5E) generated by cloning the amplified fragment (preparing the pQ5E palsmid) of the RAPD marker designated as OPQ5E were analyzed in the NAB population segregating the *nn₁* gene.

Preparation of pQ5E plasmid: PCR amplification was performed using 50 ng total DNA of NAB814 plant (see the preparation of plant total DNA in Example 2) and OPQ5 primer as described in Example 2. The amplified products were separated in agarose gel (see Example 2) and the 1750 bp-long fragment corresponding to the Q5E marker were excised, reisolated from agarose gel and purified as described in Example 3. After adding 5.5 *µ*l 10x Klenow buffer to the 50 *µ*l eluated DNA solution, fragments were treated with 0.5 *µ*l (5 unit) Klenow fragment of the DNA polymares I according to the suppliers' (Fermentas AB) instructions. Then 4 *µ*l (1 µg) *Hin*cII-digested [prepared according to suppliers' (Fermentas AB) instructions], CIP(Calf Intestine Alkaline Phosphatase)-treated (dephosphorilation) and purified pUC19 plasmid (49), 7 *µ*l 10x ligase buffer and 3 *µ*l (15 U) T4 DNA Ligase enzyme (Fermentas AB) were added to the fragments. The ligation was performed at 14 °C for 24 hours. After this the ligation was transformed into 250 *µ*l E. coli DH5α kompetent cells (Amersham Pharmacia Biotech AB, Uppsala, Sweden) using the CaCl₂ procedure (50). After induction of the bacterial gene which resulted in resistance against ampicillin (phenotypical expression), the bacteria were plated on LB plates containing ampicillin (100 µg/ml) and incubated at 37 °C for 16 hours. Plasmid DNA was isolated from the resistant colonies by QIAGEN plasmid isolation kit (QIAGEN Plasmid Mini Kit; Cat. No. 12123 QIAGEN Inc.). One clone was selected by PCR amplification from the clones carrying the 1750 bp-long amplified product: BRC1464 = E. coli DH5α (pQ5E). The sequence of the insert was determined and specific primers were designed based on the sequence:
Q5EU1: 5'-CGGCGTCTTGCTAAAGGAGA-3';
Q5ED1: 5'-TATTCGACTCATCATGGTTA-3';
Q5EU2:5'-GTCAAATATCGATTCGTGAT-3';
Q5EU3: 5'-CTAAGAAAGGCTTTGGTTGG-3';
Q5ED3: 5'-TCACGAATCGATATTTG ACA-3').

Using the specific primers in PCR amplification, the dominant-OPQ5E marker was transformed into codominant Q5E marker and its more precise map position was determined in relation to the other markers.

The map position of markers U71, U224, CG13, Q5E és U492 have been published earlier (40). The SHMT marker (51) mapped between markers Q5E and U224 on the genetic map of diploid M. *sativa* (Kaló et al. manuscript in preparation). Markers U71, U224, CG13, SHMT and U492 were mapped as RFLP markers using the F2 NAB individuals, while the marker Q5E was mapped by specific PCR (94°C 30sec, 60°C 1 min, 72 °C 1 min in 40 cycles) as well as by RFLP in the tetraploid population. The inserts of the clones (47, 51) corresponding to RFLP markers and pQ5E clone were reisolated, labelled, hybridized, washed and autoradiographed according to the method described in Example 3. The filters were selected for hybridization containing the *Dra*I*, Eco*RI, *Eco*RV and HindIII-digested total DNA of the selected Nod⁻ és Nod⁺ NAB individuals. The genotypes of the individuals were determined for markers U71, U224, CG13, SHMT, Q5E and U492 by the hybridization signals corresponding to the different alleles. The order of the genes shown in Table 4 was determined by the colormap procedure. The summary of the genetic mapping of the *nn₁* gene displaying the steps from the identification of the linked markers to the confirmation of the map position in the tetraploid population is presented on Figure 3. Analyzing the mapping data, it turned out that the markers SHMT and Q5E were the most tightly linked markers to the *nn₁* gene. No recombination could be detected in the individuals of the analyzed population between markers SHMT and nn₁. One recombination could be detected between markers *nn₁* and Q5E in the analyzed individuals.

### Example 5

### Identification of overlapping BAC clones, constructing the contig

The primary BAC clones (67A11, 2D11 és 20K10) were isolated by two molecular markers (SHMT and Q5E, see Figure 3.) most tightly linked to *nn*₁ mutation in the following way: the Q5E fragment was isolated from plasmid pQ5E, while the SHMT fragment was excised from the plasmid containing the cDNA of the gene with Kpnl restriction enzyme (51). The fragments were reisolated after gel electrophoresis, labelled and the filters were hybridized, washed and autoradiographed as described in Example 3. The filters contained more than 30 000 clones of the BAC library constructed from the total DNA of *Medicago truncatula* a close relative of *Medicago sativa,* (53). The BAC clones in the library contained about 100 kb inserts of the total DNA of M. *truncatula.* Cloning was done by *Hind*III digestion of the total DNA and cloned into the *Hind*III restriction sites of the pBeloBACII vector (52) (Figure 4.). The filters and clones of the BAC library are available in a public collection (Clemson University Genome Institute,100 Jordan Hall, Clemson 29634-5727, South Carolina, USA).

Two primary BAC clones (clones 67A11 and 2D11) was identified by hybridization with SHMT fragment, and one primary BAC clone (20K10) with the Q5E fragment. An inoculation loop of the BAC clones 67A11, 2D11 and 20K10 stored in the clone collection at -80 °C were taken from the stock and inoculated into 500 ml flask containing 100 ml LB media and chloramphenicol in 15 µg/ml concentration, respectively. The clones were grown to stationer phase at 37 °C in a shaker (250 rpm). The plasmid DNA of BAC clones were purified by the QIAGEN Large Construct Kit according to the suppliers' instructions. The plasmid DNA of the three clones were subjected to single *Eco*RV and double *Eco*RV/*Not*I digestion and the fragments were separated in agarose gels. Based on the pattern of the single and double digestion, the *Eco*RV end-fragments of the clones could be selected. From these fragments the end fragment of the inserts could be detected by *Hind*III digestion. These end-fragments were used for chromosomal walking, that is to identify overlapping BAC clones. The *Hind*III end-fragments of the clone 67A11 were approximately 3300 és 800 bp, of the clone 2D11 were 3300 and 4000 bp and of the clone 20K10 were appr. 1080 és 1400 bp length, respectively. The six end-fragments were reisolated from agarose gel and repeated hybridization was performed to identify and isolate the secondary BAC clones (50E23, 64B5, 28112, 6A5). Based on the restriction digestion pattern (*Hin*dIII, *Eco*RI, *Eco*RV) of the primary and secondary BAC clones, the degree of the overlap of the clones could be determined and the position of the clones to each other could be established. In this way the Nod contig could be constructed (see Figure 5.).

### Example 6

### Subcloning of the Nod contig

The result of the genetic mapping revealed that the SHMT marker could be the closest marker to the *nn₁* gene therefore the chromosomal region around the SHMT was analyzed first thoroughly by delimiting the genomic region containing the *nn₁* gene and by determination of the sequence of this region. In parallel experiments the subcloning of two BAC clones (67A11, 2D11) identified by SHMT and the subcloning of clones 50E23 és 28I12 overlapping with 2D11 clone were needed. The subcloning was performed in three ways. On one hand (A) the ends of the BAC inserts in the contig were cloned, on the other hand (B) the 1-2 kb length fragments of the clones 67A11, 2D11 and 50E23 generated by sonicating were cloned, so random subcloning was performed, and on the third hand (C) cloning was realized after restriction enzyme digestion.

### Example 6A

### Subcloning of the end-fragments of the BAC inserts

The end-fragments of the BAC clones were idenified as described in Example 5 and the *Hind*III fragments were cloned into pUC19 plasmid in the following way: 4 µl (1 µg), *Hind*III-digested and dephosphorylated pUC19 plasmid (Fermentas AB), 14 µl 5x Ligase buffer, and 2 µl (10 unit) T4 DNA Ligase enzyme (Fermentas AB) were added to the DNA fragments purified with the QIAGEN DNA Isolation Kit (see Example 3) and eluated in 50 µl buffer. The ligation was executed at 14 °C for 24 hours. The ligation was transformed into 250 µl E. coli DH5α competent cells using the CaCl₂ procedure (see Example 4). The transformant colonies were checked by the restriction enzyme digestion of the plasmid DNA isolated with QIAGEN plasmid DNA Isolation Kit (see Example 3).

### Example 6B

### Random subcloning of the inserts of the 67A11, 50E23 and 2D11 BAC clones

20 µg DNA of the clones 67A11, 50E23 and 2D11 in 200 µl TE buffer (1 mM EDTA, 10 mM Tris-HCl, pH7.5), respectively, were sonicated for 10 sec with the smallest head of the MSE Ultrasonic Disintegrator equipment. The fragmentated DNA was separated in agarose gel and the 1-2 kb length fragments were excised, reisolated and purified as described in Example 3. The DNA fragments generated in this way were ligated into *Hinc*II-digested (Fermentas AB) and dephosphorilated pUC19 plasmid as described in Example 4 and then they were transformed into *E. coli* DH5α competent cells (see Example 6A). Plasmid DNA were isolated from the transformant colonies with QIAGEN Plasmid Isolation Kit and their contents for inserts were checked by electrophoresis in 1 % agarose gels.

### Example 6B

### Subcloning of the 67A11, 2D11, 50E23 and 28112 BAC clones after restriction enzyme digestion

The plasmid DNA of BAC clones 67A11, 2D11, 50E23 and 28112 were purified as described in Example 5 and 1 µg purified plasmid DNA were digested with *Nhe*I*, Xba*I*, Hin*2I*, Hin*6I*, Tai*I*, Taq*I*, Bam*HI, *Hind*III*, Eco*RI and *Sau*IIIA1 enzymes (Fermentas AB), respectively according to the suppliers' recommendation. The *Nhe*I and *Xba*I fragments were cloned into Xbal-digested (the *Nhe*I and *Xba*I result in compatible sticky ends) and dephosphorylated pUC19 vector, the *Hin*2I*, Hin*6I*, Tai*I*, Taq*I fragments were cloned into *Acc*I-digested and dephosphorilated pUC19 vector; the *Hind*III and *EcoR*I fragments were cloned into *Hind*III- and *EcoR*I-digested and dephosphorilated pUC19 vector and the *Bam*HI and *Sau*IIIA1 fragments were cloned into *Bam*HI-digested and dephosphorilated pUC19 vector. After digestion the restriction fragments were purified by the QIAGEN DNA Isolation Kit (see, Example 3), ligated into the corresponding enzyme-digested (*Nhe*I*, Acc*I*, Hind*III, *Eco*RI and *Bam*HI) and dephosphorilated pUC19 plasmid and transformed as described in the Example 6A. The transformant colonies were checked by digestion with the corresponding restriction enzyme after plasmid DNA isolation as described above (Example 6A).

The recombinant clones and their insert sizes generated with subcloning are presented in Table 5.

### Example 7

### Determination of the map position of the nn₁ gene more precisely in the contig by identification of the closest recombination sites at both sides of the mutation

The generation of the subclones from the BAC clones overlapping the *nn₁* mutation and the determination of the map position of the *nn₁* gene more precisely by this subclones were done parallelly. The generated subclones were used as genetic markers to determine the recombination sites more precisely. After having the recombination sites more precisely it was obvious that the detailed sequence analysis for BAC 67A11 and 2D11 was necessary.

Two Nod⁻ plants (NAB4156 és NAB4443) carrying recombination close to the *nn₁* mutation and two other plants that did not contain recombination close to the Nod region but they were opposite homozygotes (Nod⁺ NAB814 and Nod⁻ NAB2161) for the Nod character were used for genetic experiments. The clones G34P44 and G33P155 coming from BAC clone 67A11, the clone G3P126 coming from 2D11 and clone G18P4 arising from 28I12 BAC clone were converted to molecular markers. The inserts of the clones were excised with the proper restriction enzyme, the fragments were reisolated and used as hybridization probes to the filters containing the *Eco*RV-, *Hind*III- and *Dra*I-digested total DNA of above mentioned four plants. From the experimantal results (see Figure 6) it could be concluded that the position of the *nn₁* gene was delimited on a genomic region (Nod region) by two recombination detected by fragment G34P44 located on BAC clone 67A11 and by fragment G18P4 located on BAC clone 28I12.

### Example 8

### Determination and analysis of the sequence of the Nod region

In order to determine the sequences of the subclones obtained as described in Example 6, the ABI PRISM Dye Terminator Cycle Sequencing Ready Reaction Kit and the ABI PRISM BigDye Terminator Cycle Sequencing Ready Reaction Kit (Perkin Elmer Applied Biosystems; 850 Lincoln Centre Drive Foster City, CA94404 USA) was used according to the manufacturer's recommendation. The plasmid DNA for templates of the seqeuncing reaction were isolated by QIAGEN Plasmid Isolation Kit and their concentration were quantified using spectrofotometry (see Example 3.). The sequences of the amplified products labelled by flurorescent dyes were determined by ABI 373 and ABI 377 automated sequencer (Perkin Elmer Applied Biosystems; 850 Lincoln Centre Drive Foster City, CA94404 USA). Authenticity of the DNA sequences: the sequences were determined on both strand, in one direction the reactions were repeated 2-8 times, based on independent templates in ambiguous cases. The sequence data were stored in computer and their analyses were started with ordering them into overlapping sequences. The overlapping end-fragments of the BAC clones and of the subclones generated by restriction digestion of these BAC clones could help to assemble the sequences and to arrange the subclones generated by random fragmentation or restriction enzyme digestion and the BAC clones to each other. By the help of the available sequences, homologue genes were searched in the NCBI (National Center for Biotechnology Information http://www.ncbi.nlm.nih.gov/BLAST/) and the *Arabidopsis* (http://www.arabidopsis.org) databanks. Taking into consideration of the homology between the sequences, the common and general characters of the gene structures [consensus sequences as initiation and termination codons, open reading frame (ORF), consensus sequences characteristics of exons and introns such as GT-AG rule, the point of divergence, etc.] the starting- and end-point of the coding regions of the genes located in the Nod region were searched and the order and the orientation of the genes were determined. The results of this sequence analysis are presented schematically on Figure 7. the order and the orientation of the genes were determined. The results of this sequence analysis are presented schematically on Figure 7.

### Example 9.

### Detailed analysis of the NORK gene

The Nod⁻ phenotype of the mutant plant can be explained above all with a mutation in the NORK (NOD region linked Receptor Kinase) gene coding for a receptor kinase, that is why the sequence analyis of the wild type NORK gene on BAC clone 67A11 was carried out first. The NORK gene is located on an Nhel fragment of 8563 bp length and its nucleotide sequence - from the Nhel site to the next Nhel site - is SEQ ID NO:1. This nucleotide sequence identify the genomic sequence of the NORK gene from the *M. truncatula* A17 plant (the BAC library was constructed from this plant). To reveal the exact structure of the gene we have to know the exon/intron borders/positions which can be predicted from the genomic sequence based on the GT-AG rule and the branching point, however, it can be determined exactly only by knowing the cDNA sequence. For this purpose we isolated RNA from the roots of *M. truncatula* line A17, then we prepared cDNA from the mRNA by reverse transcription. cDNA samples were produced by PCR amplification using NORK specific primers(see Figure 9.), then the amplified fragments were sequenced either directly or after cloning as desribed in Example 8.

### Example 9A.

### Construction and sequencing of NORK specific cDNA sequences

The seeds of *M. truncatula* A17 were germinated after sterilization and planted into sterile soil. From the roots of the two-three week old plants the cDNA representing the NORK mRNAs as well, was prepared as follows. The roots of the plants were pulverized/ground under liquid nitrogen, the powder was solubilized in RNA extraction buffer (8 M guanidinium chloride, 20 mM MES, 20mM EDTA, 50 mM β-mercaptoethanol), then purified by extraction with a mixture of phenol-chlorophorme-isoamil-alcohol (25:24:1). After centrifugation the supernatant was precipitated at -20°C for 1 hour with 0.7 volume of 95% ethanol and 0.2 volume of 1 M acetic acid. After centrifugation the precipitated RNA was dissolved in DEPC (diethyl-pyrocarbonate)-treated distilled water. The cDNA was prepared from the RNA with the help of the RevertAid H Minus First Strand cDNA Synthesis Kit (Fermentas AB). The different RNS samples were treated with RNase free DNase (Fermentas AB) and then the cDNAs were synthetized with the help of oligo-dT primer and reverse transcriptase enzyme (Fermentas AB) in the presence of RNase inhibitor (Fermentas AB) at 42 °C. Overlapping fragments of the NORK cDNA were amplified from the prepared cDNA with different combinations of the primers shown in Figure 9. The PCR conditions were as follows: 95°C for 30 seconds, 55 °C for 1 minute, 72 °C for 1 minute, these steps were repeated 35-times. The specific NORK fragments amplified by RT-PCR from the cDNA were separated by agarose gel electrophoresis and purified with the help of the Fragment Isolation Kit of QIAGEN. The purified fragments were sequenced as described in Example 8. either directly or after cloning them into Hincll digested and phosphatase treated pUC19 vector. The full-length cDNA sequence could be constructed from the sequences of the overlapping cDNA fragments. The cDNA sequence of the NORK gene from the *M. truncatula* A17 plant is SEQ ID NO.2

### Example 9B.

### The alignment and analyis of the genomic and cDNA sequences of the NORK gene.

The exact posotion of the exons and introns could be determined by aligning the genomic and the cDNA sequences of the NORK gene. The alignment of the NORK gene sequences from the *M. truncatula* A17 plant is shown in Figure 11., the detailes about the position of exons and introns are presented in Table 6. The proposed start codone of the NORK gene is the ATG start codone (coloured green) at the 1153. bp of the *Nh*eI fragment (pNORK_Nhe5), while the proposed stop codone is the TAG stop codone (coloured violet) at the 7898. bp. In the putative promoter region at the 5' end of the gene can be found the TATA-box (743-55, 769-85) és CCAAT-box (675-79, 688-91 bp) sequences characteristic for the region. At the 3' end of the gene there are several sequences which may serve as transcriptional termination signals. There is no difference between the two sequences (genomic and cDNA) which is in accordance with the facts that the genomic and the cDNA is originated from the same plant, and there is only one gene in the genome.

By comparing the sequences to the entries of databases we realized that in the case of M. *truncatula,* in the so-called EST (Expressed Sequence Tags) databases there are partial cDNA sequences homologous to the NORK gene. These sequences were obtained by random sequencing, i.e. certain laboratories made sequencing reactions and single runs on several thousand cDNA clones of different libraries without any knowledge about their function. Such sequences from the M. *truncatula* plant can be found under accession numbers AW684661, AW685681 and BE203249. The appearance of the cDNA sequences in this form also support the expression of the NORK gene in the roots of legumes.

The activity of the NORK gene in the cells, i. e. it is expressed, is proven by the fact that we could detect NORK specific cDNA corresponding to the mRNA via RT-PCR. We presented as described in Example 14. that the NORK protein is synthetized from the mRNA. The amino acid sequence of the NORK protein can be deduced from the cDNA sequence. The amino acid sequence deduced from the longest Open Reading Frame (ORF) of the NORK cDNA is shown in Figure 12. The NORK protein consists of 924 amino acids, and it contains characteristic sequence motifs pointing to functions, which were revealed on the basis of similarity to consensus sequences in databases. The functional representation of the NORK protein and its characteristic sequence motifs are shown in Figure 13.

From the above data obtained in this way we concluded that one part of the molecule is located extracellularly (extracellular part) which is followed by an intracellular part via a transmembrane stretch. According to our knowledge the extracellular part contains a so-called LRR (Leucin Rich Repeats) region (according to the homologies: between amino acids 405-476) which may be involved in either the direct or the indirect recognition and in either the direct or the indirect binding of Nod factor. Based on the similarities, the proposed intracellular part has kinase activity. It is known for a specialist of molecular biology that the kinases participate in different signal transduction pathways, they initiate or transduce the biological signals by phosphorilating proteins. The NORK protein may phosphorilate its substrates within the cell and thus it initiates the signal transduction cascade leading the development of the nodule. Based on the structural analysis of the protein it is possible to suppose that the NORK protein is capable for autophosphorilation.

### Example 9C.

### The alignment of the cDNA sequence of M. truncatula A17 to those of the mutant. (Nod') and the wild type (Nod⁺) M. sativa plants.

With the help of primers designed to the NORK gene of *M. truncatula* (Figure 9.) genomic and cDNA sequences (Example 9A.) were amplified from F3 plants carrying in homozygous configuration the Nod⁻ alleles designated as number 1 and number 2 (NAB1241/6 and NAB701/28), as well as the Nod⁺ allele designated as number 6 (NAB615/28). The nucleotide sequences of the amplified products were determined directly or after cloning as described in Examples 8. and 9A. The cDNA sequence of the Nod⁻ alleles designated as number 1 and number 2, as well as the Nod⁺ allele designated as number 6 is SEQ ID NO: 4 and SEQ ID NO: 5, respectively, the amino acid sequences deduced from the cDNA sequences of the Nod⁻ alleles designated as number 1 and number 2, as well as the Nod⁺ allele designated as number 6 are SEQ ID NO: 6 and SEQ ID NO:7, respectively. The sequence analysis revealed that the sequences of the NORK alleles designated as number 1 and number 2 are identical, that is why only one sequence is presented.

The alignment of the cDNA sequences of the Nod⁻ and Nod⁺ alleles from *M. sativa* to the cDNA sequence of the *M. truncatula* NORK gene is shown in Figure 18. One can see from the comparison of the sequences that there are a number of differences between the sequences of M. *sativa* and that of *M. fruncatula,* and a few differences - in most cases causing no amino acid changes - can be found between the wild type and mutant alleles of *M. sativa.* The most dramatic change in the structure of the Nod⁻ allele carrying the *nn₁* mutation can be found at the 11. basepair in the 13. exon (E13) of the gene. The mutation in the triplet coding for tirosine resulted in a STOP codone in the sequence originated from the Nod⁻ plant. As a result of this mutation, the synthesis of the protein is terminated earlier, even before the synthesis of the putative active site of the kinase, and it can be supposed that the produced shorter protein cannot fulfil its function in the signal transduction..

The alignment of the amino acid sequences of the NORK proteins of the M. *truncatula* A17 és a *M. sativa* plants is shown in Figure 19. The comparison of the sequences reveals that the sequences of *M. sativa* differ at a number of positions from that of *M*. *truncatula* A17. The amino acid differences between the Nod⁺ alleles of *M. truncatula* A17 and *M. sativa* are probably the results of so-called neutral mutations which do not change the function. The comparison of the amino acid sequnces of the Nod⁻ and the Nod⁺ NORK alleles reveals that the most dramatic change in the structure of the NORK protein in the *M. sativa* mutant MN-1008 is that the protein consists of only 709 amino acids because there is a STOP codon in place of the tyrosine codon in position 710 which causes the termination of protein synthesis (see above). There are more than one amino acid changes in the protein originated from the Nod⁻ allele compared to that of the Nod⁺ allele which might result in the loss of function. It cannot be decided that in which order the mutations in the *nn₁* gene arose and which one was the first that in homozygous configuration resulted in the fail of nodule formation, i.e. in Nod⁻ phenotype.

### Example 10.

### The number of NORK genes in the Medicago genome and its distribution in the plant kingdom

The genetic mapping is a suitable tool to determine the copy number of the NORK gene. For this purpose, using the putative extracellular part of the NORK gene (this part is specific, there is no homologous sequence in the databasis) as a hybridization probe, hybridization experiments were carried out as described in Example 3. with the filters carrying the DNA of the individuals from the diploid and tetraploid mapping populations of M. *sativa.* The 1695 bp length probe, which is called as "NORK specific" probe, was prepared by PCR using primers Pr_RKU4 és Pr_RKD2X (Figure 9.) and a cDNA template originated from the M. *truncatula* A17 plant as described in Example 9A. The analysis of the autoradiograms showed that the segregating hybridizing fragments identify a single locus (Figures 20. and 21.), which means that there is only one copy of the NORK gene in the genome, i.e. the NORK gene is a single-copy gene.

The above described probe was used for the hybridization to the genomic DNA of different plants. With the help of the QIAGEN Plant DNA Isolation Kit genomic DNA was isolated as described in Example 2. from different legume plants: *M*. *truncatula* A17 és A20, *Pisum sativum* cv. Frisson, *Sesbania rostrata, Cassia emerginata, Desmodium* sp., *Vicia sativa, Melilotus alba, Trifolium pratense, Trifolium incarnatum, Vigna unguiculata, Macroptilium atropurpureum, Vigna radiata, Glycine max, Lotus corniculatus, Lotus japonicus* cv. Gifu, and from non-legume ones: *Nicotiana tabacum* cv. Small Havanna SR1, and rice. 15-20 µg of total DNA was digested by restriction enzymes *Eco*RI, *Eco*RV, *Dra*I or *Hind*III, the DNA fragments were separated by electrophoresis and bound to Hybond N+ filters (see. Example 3). The autoradiograms obtained from the hybridizations performed at 55°C using the "NORK specific" probe (Example 10.) can be seen in Figures 22., 23., 24., 25., 26., 27. One can see on the autoradiograms that there is only one or two strongly hybridizing fragments in most legumes which means that in most of the legumes the NORK gene is a low copy number gene, only one or few genes are present in the genome. The autoradiograms obtained after the hybridization experiments with non-legume plants show that in these species the presence of the NORK gene cannot be detected under our hybridization conditions.

### Example 11.

### Determination of the NORK cDNA sequence from other legumes.

The nucleotide sequences of the NORK gene weres determined from a single individual of two other legume species, pea (*Pisum sativum*) and vetch (*Vicia villosa*). The sequencing of the NORK cDNA from *Pisum sativum* cv. Frisson and *Vicia villosa* S-1 plants was carried out using the primers designed for the NORK gene of *Medicago truncatula* (Figure 9.) as described in Example 9A. Both *Pisum sativum* cv. Frisson and *Vicia villosa* S-1 has Nod⁺ phenotype, similarly to *Medicago truncatula,* diploid, autogamous plants, which means that the two alleles of their NORK gene has identical sequence. The nucleotide sequence of the amplified products was determined directly or after cloning as described in Examples 8. and 9A. SEQ ID NO : 8 and SEQ ID NO:9 are the NORK cDNA sequences of *Pisum sativum* cv. Frisson and *Vicia villosa* S-1, respectively, constructed from the sequences of overlapping fragments. SEQ ID NO:10 and SEQ ID NO:11 are the amino acid sequences deduced from the cDNA sequences of these plants.

The cDNA sequences determined by us from *Pisum sativum* and *Vicia villosa* plants as well as the cDNA sequence from the *Lotus japonicus* Miyakojima MG-20 plant deposited in the NCBI database under the Accession number AV410167 proves that the NORK gene is transcribed in these plants as well, , i.e. it is active. It is noteworthy to mention that in *Vicia villosa* there are either two alleles or two copies of the NORK gene (see Figures 29. and 31.). It can be concluded as well that the nucleotide sequence of the NORK gene in plants investigated by us is so homologous to that of *M. truncatula* that the primers designed for the NORK gene of *M. truncatula* could be used for the amplification of different parts of the NORK gene from alfalfa, pea and vetch.

### Example 12.

### Complementation of the Nod⁻ mutant M. sativa MN-1008 plant with the NORK gene.

In order to prove that the identified mutation in the NORK gene is responsible for the Nod⁻ mutant phenotype, the 8.5 kb *Nhe*I fragment carrying NORK gene (Table 5., Figures 7. and 8.) was cloned into plant transformation vectors (see Table 7.). The NORK gene was cloned in the so-called T-DNA part of the *Agrobacterium-based* plant transformation vectors. *Agrobacterium* species that entered the tissues of plants transfer the T-DNA from their plasmid into the plant cells (T-DNA transfer), which is followed by the integration of T-DNA (54). The random integration of T-DNA into different regions of the plant genome makes possible the stable maintenance and inheritance, as well as the expression of the genes of the T-DNA. There are two basic methods to introduce T-DNA based plasmids into plants: the *Agrobacterium tumefaciens* and the *Agrobacterium rhizogenes* mediated transfer. Using *A. tumefaciens* strains, transformed calli are obtained first, from which somatic embryos have to be induced which have to be regenerated to raise transgenic plants. This procedure can be carried out only by using so-called embryogenic plants. Using *A. rhizogenes* strains for infection, hairy roots grow on the infected part of the plant, and as a result a chimeric organism is formed in which only the hairy roots are transformed.

Since preliminary results showed that the plant (MN-1008) carrying the *nn₁* mutation is not embryogenic under standard conditions, i.e. plants cannot be regenerated from the calli transformed by the *A. tumefaciens* mediated method, two strategies were chosen for the genetic complementation.
(i) Using the *Agrobacterium rhizogenes* transformation method, when the ability for the symbiotic nodule formation of the transformed hairy roots can be investigated. Transformed roots of most legume species are able to form nodules(55, 56), that is why, in the case of complementation, the formation of nodules might be expected on the roots of the mutant plants.
(ii) The non-embryogenic MN-1008 plant was crossed as described in the Example 1. with an individual of an embryogenic line (Regen SY) which was provided by Dr. Deborah A. Samac, University of Minnesota, MN, USA (57). After the selection and self-pollination of the embryogenic F1 individuals, the symbiotic phenotype of the individuals of the F2 population was determined as described in the Example 1., and two plants from the Nod⁻ progeny (F2RN28/4, F2RN28/5) as well as two embryogenic F1 plants (F1RN28 és F1RN41) was used for the A. *tumefaciens* mediated transformation experiments.

### Example 12A.

### The cloning of the NORK gene into plant transformation vectors

The fragment carrying the NORK gene was cloned into several plant transformation vectors which have different copy number and provide different DNA-environment in the T-DNA that might affect the expression of the transformed gene.

The 8.5 kb Nhel fragment carrying the NORK gene was cloned into the different plant transformation vectors from plasmid pBRC1660 which was constructed as follows: From the G20P5 clone (see Table 5.) constructed as described in Example 6., the insert was cut by the restriction endonucleases Pael and *Sac*I and was ligated into the pOK12 (49) vector which was digested with the same enzymes and dephosphorilated by the CIP enzyme. From the obtained clone designated as pAT688 the insert was cleaved by the restriction endonucleases *Cla*I és *Sac*I and it was ligated into the vector pBluescript II SK (Stratagene, 11011 North Torrey Pines Road, La Jolla, CA, USA). From the clone pBRC1660 obtained as described above (maintained in *E. coli* DH5α strain under the code BRC1660) the NORK gene can be deliberated by several enzymes. The pOK12 vector was provided by Dr. J. Messing (Waksman Institute, Piscataway, NJ, USA).

The 11.8 kb Clal fragment harbouring the NORK gene with a 2.2 kb longer 5' (promoter) sequence was cloned into the different plant transformation vectors from plasmid pBRC1690 which was constructed as follows: The BAC clone 67A11 shown in Figure 5. was digested by Clal enzyme, then the fragment was reisolated from agarose gel as described in the Example 3. and was ligated into pBluescript II SK vector which was digested by the same restriction endonuclease and dephosphorilated by the CIP enzyme.

The pPK459 vector which was used for the construction of the pBRC1678 clone was developed as follows: The H*ind*III-*Eco*RI, fragment from the polylinker of the pSL301 plasmid (59) was cloned between the *Hind*III és *Eco*RI sites of the pGA471 vector described by An et al. (58). The pGA471 vector (58) and the pSL301 vector (59) were kindly provided by G. An (Washington State University, Pullman, WA, USA) and J. Brosius (Mt. Sinai School of Medicine, New York, NY, USA), respectively.

The pPR97 vector (60) used for the construction of clones pBRC1666 and pBRC1701 was provided by László Szabados (MTA, BRC, Szeged).

The pAT680 vector used for the construction of the pBRC1667 clone was constructed as follows: the T-DNA part of the pGREEN II vector described by Hellens et al. (61) was digested by Hpal enzyme and treated by the CIP enzyme and was ligated to an EcoRV fragment carrying the nopaline synthase gene promoter (nos) driven neomycin phosphotransferase II gene providing kanamycin resistance (nos-KmR casette), and the obtained clone was deposited in the strain collection under the name pAT678. After removing by EcoRV digestion from the carrier plasmid, the intron-containing *uid*A gene (62) coding for the β-glucuronodase gene and driven by 35S promoter of the cauliflower mosaic virus (35S-GUS-INT casette; 61) was built into the Stul digested CIP treated pAT678 vector. The T-DNA containing BgIII fragment was cloned from the obtained pAT679 plasmid into the BamHI site of the broad host range vector pAT672 which was constructed from the replication origo of plasmid pBBR1MCS-4 (63) and the kanamycin resistance gene of plasmid pK19 (64) as follows: The kanamycin resistance gene from plasmid pK19 was amplified as described in Example 4. using primers KMU (5'-GGCGATCGATAGACTGGGCGG) and KMD (5'-TCGTGATGGCAGGTTGGGCG), then the amplified fragment was cleaved by Clal and reisolated from agarose gel as described in Example 3. The isolated fragment was cloned into the Ehel and Clal digested pBBR1MCS-4 vector. The original restriction enzyme cutting sites from the resulted pAT671 vector were removed in two steps as follows: The DNA was digested by *Bam*HI and *Bsp*119I, then *Bam*HI and *Cla*I enzymes and the digested DNAs were treated in the presence of 0,05 mM dNTP with the Klenow fragment of DNA polymerase I as described in Example 4. and ligated. The overhanging ends of the DNA resulted after the *Bam*HI and *Bsp1*19I, as well as the *Bam*HI and *Cla*I digestions were filled in by the Klenow fragment which restored the recognition site (GGATCC) of *Bam*HI. Plasmids. pK19 (64) and pBBR1 MCS-4 (63) were kindly provided by D. Pridmore (Ciba-Geigy AG, Basel, Switzerland) and E. Kovach (Louisiana State University, Shreveport, LA, USA), respectively. Vector pGREEN II and casettes nos-KmR and 35S-GUS-INT can be obtained from the John Innes Centre, Norwich, England (detailed information: www.pgreen.ac.uk).

These cloning vectors (pPK459, pAT680, pPR97) were digested by restriction enzymes shown in the third column of Table 7., purified and dephosphorilated by the CIP enzyme as described in Example 4. After a second purification step 1 µg of vector DNA was ligated with 1 µg of the insert carrying the NORK gene. The insert carrying the NORK gene was deliberated from the plasmid pBRC1660 by enzymes shown in the third column of Table 7. then isolated from gel as described in Example 3. The ligated DNAs were transformed into *E. coli* strain DH5α, then after the checking of the transformant colonies (see Example 6A) the E. coli strains carrying the clones were deposited under the codes BRC1666, BRC1667, BRC1678 and BRC1701 (Table 7.).

The plasmids carrying the NORK gene (pBRC1666, pBRC1667, pBRC1678, pBRC1701) were purified from the *E. coli* strains and were transformed into the different *Agrobacterium rhizogenes* és *Agrobacterium tumefaciens* strains shown in Table 8. The transformations were carried out by electroporation as follows. Bacteria grown on solid LB medium containing 100 µg/ml of rifampicin antibiotics were inoculated into 5 ml of YENB (65) medium containing 100 µg/ml of rifampicin and were incubated overnight at 30 °C with good aeration. 200 ml of YENB medium was inoculated with this starter culture. The bacterium culture was grown with intensive aeration to late logarithmic phase (optical density at 600 nm, OD₆₀₀=0.6-0.8), then the cells were collected by centrifugation. The bacteria were washed at 0°C two times with 100-100 ml of sterile distilled water and two times with 10 ml of 1mM HEPES (pH=7) containing 10% of glicerol. Bacterial cells were resuspended at 0°C in 2-4 ml of sterile 1mM HEPES (pH=7) containing 10% of glicerol, and these electrocompetent cells were frozen in liquid nitrogen as 100 µl aliquots and stored at -80 °C till usage: 0.1-1 µg of plasmid DNA was added to the electrocompetent cells on ice, then the DNA was introduced into the cells at 1.25 kV/cm field strength with the help of to E.coli Pulser instrument (BioRad) according to the supplier's instructions. Immediatly after the electroporation, 1 ml of LB medium was added to the cells, then after an incubation at 30 °C for 120 minutes the cells were plated onto LB medium containing 100 µg/ml of rifampicin and antibiotics shown in Table 7.

### Example 12B.

### Transformation using Agrobacterium rhizogenes strains

The *A. rhizogenes* strains (BRC1673, BRC1675, BRC1679; Table 7.) carrying the NORK gene on plasmid can be introduced into the tissues of the Nod⁻ mutant M. *sativa* plants by two ways: 1.) through the sectioned surface of seedlings' radicle and 2.) through the sectioned surface of shoots of mature plants.

Transformation via cutting the radicles of seedlings: The seeds of the MN-1008 and the Nod⁻ plants (NAB701, NAB809, NAB897, NAB968, NAB2792) carrying *nn₁* mutation - which were identified and maintained as described in Example 1. - were sterilized and germinated on 1% water agar as desribed in Example 1. The rootlets of the 3-4 day old seedling were cut 5-10 mm below cotyledon and the wounded roots were dipped into a lawn of *A. rhizogenes* grown on solid LB medium. The *A. rhizogenes* strains (BRC1673, BRC1675, BRC1679) were streaked from the strain collection stored at -80 °C onto LB medium containing 100 µg/ml of rifampicin and antibiotics shown in Table 7. and were incubated at 30 °C for 3 days. The plants treated with the *Agrobacterium* strains were placed into squared (10x10 cm) Petri-dishes containing TM-1 medium (66) and were grown at 24°C with 16 hour light period and 10000 Lux light intenstity. After 5 days roots were appearing at the cut surface, and part of them were hairy root, i.e. transformed root.

2. Transformation through the sectioned shoots of mature plants: The young shoots of the MN-1008 and the Nod⁻ plants (NAB701, NAB809, NAB897, NAB968, NAB2792) identified and maintained as described in Example 1. that carry the *nn₁* mutation in homozygous configuration were cut from the plants and placed immediately into tap water. The sectioned surfaces of the shoots were dipped into melted parrafine to avoid the entering of the chemicals used for sterilization into the vascular tissues. Then the shoots were sterilized as follows: they were washed in 70% ethanol for 5 seconds then immediately in 20% bleech containing 0.05 % Tween 20 for 90 seconds. After 3 washing in sterile destilled water the shoots were cut into pieces of 3-5 cm. After dipping the root-proximal surface into *Agrobacterium rhizogenes* lawn grown as described in the previous paragraph the distal end of the plants were stabbed into TM-1 medium and were incubated at 22°C with 16 hour light period and 10000 Lux light intenstity. From the 20. day roots were appearing at the place of the wounding and some of them were transformed

Transformed roots could be identified by the so-called GUS-staining (67) because the T-DNA carries the uidA gene coding for the β-glucuronidase enzyme. The roots were stained fro the GUS activity as follows: root pieces were incubated at 37°C in dark for 12-24 hours in a buffer containing 100 mM TRIS (pH=7.4), 50 mM NaCl, 2 mM spermidine, 2 mM K₃Fe(CN)₆, and 2 mM X-GlcU (5-bromo-4-chloro-3-indol β-D-glucuronic acid) dye. The β-glucuronidase enzyme expressed in the transformed roots cleaves the dye and as a result a blue product precipitated in the cells is formed which can be detected both by eyes and by microscope. The *A. rhizogenes* strains used for transformation and the transformation efficiency is shown in Table 9. After the transformation 4 transformed roots were obtained, however, no nodule formed in the presence of compatible S. *meliloti.* The reason for this results might be that the T-DNA of the Ri plasmid harbours genes coding for enzymes involved in plant hormone biosynthesis and the expression and biological effect of these genes in the plant cells inhibit nodule formation (Á. Kondorosi, personal communication).

### Example 12C.

### Transformation using Agrobacterium tumefaciens strains

The A. *tumefaciens* transformation was carried out as follows: Twenty pieces of healthy, dark-green leaves were cut from the alfalfa plant to be transformed and placed immediately into Petri dishes containing tap-water. The leaves were sterilized as follows: after washing with 70 % ethanol for 5 seconds, they were placed immediately into a 20 % of bleech containing 0.05 % of Tween 20, and washed for 90 seconds. This was followed by three washing steps in sterile destilled water. The edges and the central vascular tissues of the sterilized leaves were removed and the two pieces were split in two. These leaf pieces - with all the edges cut - were placed into SHO medium containing *A. tumefaciens* which was prepared as follows: The *A. tumefaciens* strains (BRC1677, BRC1680, BRC1681, BRC1707; Table 8.) stored at -80°C were streaked on solid medium containing 100 µg/ml of rifampicin, as well as the proper antibiotics shown in Figure 7. and incubated at 30°C. A single colony was inoculated by loop into 3 ml of liquid YEP medium (in 20 ml tube) and the bacteria were grown by rolling (to provide aeration) to the stacioner phase (24 óra). The bacteria were collected by centrifugation as described earlier, the supernatant was poured away, and the bacteria were suspended in 12 ml of SHO medium. After keeping in the *Agrobacterium* suspension for 20 minutes the leaves were removed and the excess of suspension was blotted by sterile blotting paper, then the leaf pieces were placed onto B5h in Petri dishes (maximum 16 leaf pieces per plate). The plates were sealed by air-permeable bands and placed to 24°C and 16 hour light period with 10000 Lux light intenstity. After 1 week the leaves were removed from the medium, washed 3 times with sterile distilled water and placed onto B5hKmCb medium then were incubated as described above. After 3 weeks the leaf pieces forming calli were placed onto B5hKmCb medium and were further incubated as described above. The appearing dark green embryos were transferred onto MMSCb medium and were further incubated as described above. The plantlets forming roots and shoots were transferred into jars containing MMSCb medium where the putative transformants were raised. The developed plants were propagated in vitro as follows: a piece of the shoot containing 3-4 pair of leaves was cut under sterile conditions, placed into MMSCb medium in a jar and incubated as described above.

After strengthening of the shoots and roots of the propagated plants, DNA was isolated from the leaves of the plants as described in Example 2., then to detect the transformation events PCR reactions were carried out as described in the Example 3. by using primers specific for the *nptII* gene providing resistance against kanamycin (NPT-U: 5'-ACCCAGCCGGCCACAGTCG-3', NPT-D: 5'-GGGCGCCCGGTTCTTTTTG-3') and for the uidA gene encoding the β-glucuronidase enzyme (GUS-U: 5'-TTATGCGGGCAACGTCTGGTAT-3', GUS-D: 5'-AGTCCCGCTAGTGCCTTGTCC-3') that are within the T-DNA.

After transfering to nitrogen-free Gibson medium, the plants were infected with S. *meliloti* strain 41 and the process of nodulation was followed. Transformed derivatives of RN28 and RN41 were self-pollinated then germinated as described in Example 1. and we checked their nodule formation ability.

The results of the transformation experiments by using the *A*. *tumefaciens* strains are summarized in Table 10.

The composition of the YEP medium:
10 g/l Protease Peptone (Difco Laboratories)
10 g/l Bacto yeast extract (Difco Laboratories)
5 g/l NaCl
Sterilization: 121 C°, 20 minutes.

The composition of the LB medium:
10 g/l Bacto-tryptone (Difco Laboratories)
5 g/l Bacto yeast extract (Difco Laboratories)
5 g/l NaCl
pH=7.5
Sterilization: 121 C°, 20 minutes.

The composition of the YENB medium:
8 g/l Bact Nutrient Broth (Difco Laboratories)
7.5 g/l Bacto yeast extract (Difco Laboratories)
Sterilization: 121 C°, 20 minutes.

The composition of the SHO medium:

**Schenk and Hildebrant salt:**

| | |
|---|---|
| KNO₃ | 2500 mg/l |
| MgSO₄·7H₂O | 400 mg/l |
| NH₄H₂PO₄ | 300 mg/l |
| CaCl₂·2H₂O | 200 mg/l |
| MnSO₄·H₂O | 10 mg/l |
| H₃BO₃ | 5 mg/l |
| ZnSO₄·7H₂O | 1 mg/l |
| Kl | 1 mg/l |
| CuSO₄·5H₂O | 0.2 mg/l |
| NaMoO₄·2H₂O | 0.1 mg/l |
| COCl₂.6H₂O | 0.1 mg/l |
| FeSO₄·7H₂O | 15mg/l |
| Na₂EDTA | 20 mg/l |

Schenk and Hildebrant vitamine solution:

| | |
|---|---|
| myo-inositole | 1000 mg/l |
| Nicotinic acid | 5 mg/l. |
| Thiamine·HCl | 5 mg/l |
| Pyridoxine·HCl | 0.5 mg/l |
| 30 g/l saccharose | |
| 0.5 g/l MES (Sigma) | |
| pH= 7.5 (adjusted with KOH) | |
| Sterilization: 121 C°, 20 minutes. | |

The composition of the B5h medium:
1 liter of B5 soulution
30 ml of B5 amino acid stock solution
1 ml of B5 hormone stock solution

The composition of the B5 solution:

| Gamborgs' B5 salts: | |
|---|---|
| KNO₃ | 2500 mg/l |
| MgSO₄·7H₂O | 250 mg/l |
| (NH₄)₂SO₄ | 134 mg/l |
| CaCl₂·2H₂O | 150 mg/l |
| NaH₂PO₄·H₂O | 150 mg/l |
| MnSO₄·H₂O | 13.5 mg/l |
| H₃BO₃ | 3 mg/l |
| ZnSO₄·7H₂O | 2 mg/l |
| Kl | 0.75 mg/l |
| CuSO₄·5H₂O | 0.025 mg/l |
| NaMoO₄·2H₂O | 0.25 mg/l |
| CoCl₂·6H₂O | 0.025 mg/l |
| FeSO₄·7H₂O | 27.8 mg/l |
| Na₂EDTA | 37.3 mg/l |

| Gamborgs' B5 vitamine solution: | |
|---|---|
| myo-inositol | 100 mg/l |
| Nicotinic acid | 1 mg/l |
| Thiamine·HCl | 10 mg/l. |
| Pyridoxine·HCl | 1 mg/l |
| | |
| 0.5 g/l proline | |
| 40 g/l saccharose | |
| pH= 5.7 (adjusted with KOH) | |
| 8 g/l Phytoagar (GIBCO BRL) | |
| | |
| Sterilization: 121 C°, 20 minutes. | |

Before plating sterile amino acid and hormone stock solutions are added.

B5h amino acid stock solution:
6.65 g/250 ml L-glutamine
0.83 g/250 ml serine
0.004 g/ 250 ml adenine
0.083 g/250 ml glutathione

Filter sterilization. 30 ml of amino acid stock solution is added to 1 I of B5h medium before plating

B5h hormone stock solution:
1 mg/ml 2,4-D-(2,4-dichlore-phenoxy-acetic acid, GIBCO BRL)
0.1 mg/ml kinetin (GIBCO BRL)

Filter sterilization. 1 ml of homone stock solution is added to 1 l of B5h medium before plating

The composition of the B5hKmCb medium:
B5h medium containing 25 mg/l of kanamycine and 500 mg/l of carbenicilline.

The composition of the MMSCb medium:

| 4.3 g/l Murashige and Skoog salt (GIBCO BRL) | |
|---|---|
| 1 ml/l 1000x Nitsch and Nitsch vitamine stock solution: | |
| myo-inositol | 100mg/ml |
| glycine | 2 mg/ml |
| Nicotinic acid | 5 mg/ml |
| Pyridoxine-HCl | 0.5 mg/l |
| Thiamine-HCl | 0.5 mg/l |
| Folic acid | 5 mg/l |
| Biotine | 0.05 mg/l |
| 30 g/l saccharose | |
| pH= 5.7 (adjusted with KOH) | |
| 8 g/l Phytoagar (GIBCO BRL) | |
| Sterilization: 121 C°, 20 minutes. | |
| 500 mg/l of carbenicilline is added after sterilization and before plating. | |

### Example 13.

### The introduction of the NORK gene of M. truncatula into other (leguminous and non-leguminous) plants

The *Agrobacterium tumefaciens* strains described in Example 12. (BRC1677, BRC1680) carrying the NORK gene of M. *truncatula* were used for the introduction of the NORK gene into the legume plant vetch (*Vicia villosa*) and the non-legume tobacco (*Nicotiana tabacum* cv. Small. Havanna SR1) plant. The transformations were performed as described in Example 12C with the modification that in the case of *Vicia* 2 mg/l of 2,4-D and 0.2 mg/l of kinetin was used, while the pieces of the tobacco leaves were placed onto MS medium (68) containing 1 mg/l of benzyl-aminopurine (BAP) and 0.1 mg/l of naphtyl-acetic acid (NAA), as well as antibiotics (100 mg/l of kanamycin and 500 mg/l of carbenicillin).

After the transformations six *N. tabacum* and eight *V. villosa* plants originated from independent transformation events were regenerated (Table 11.). Total DNA from the transformed plants was isolated and PCR amplification using the Pr-RKU4---Pr-RKD5 primerpair (Figure 9.) was carried out as described in Examples 2. and 9., respectively. The sequence of amplified products (exon1-exon2 of the NORK gene) were determined as described in Example 8. The sequencing results revealed that all sequences determined from the transformed plants were identical to the DNA sequence of the M. *truncatula* NORK gene, which means that the transgenic plants contains the NORK gene of M. *truncatula.* Proteins from the roots of transformed tobacco plants were isolated as described in Example 14. and the presence of the NORK protein in the transformants was demonstrated by dot blot hybridization. These results show that the NORK gene is expressed in *N. tabacum.* The results of the transformation experiments and the characterization of the transformants are shown in Figure 32.

The composition of the MS medium:

| Murashige and Skoog salts: | |
|---|---|
| KNO₃ | 1900 mg/l |
| MgSO₄·7H₂O | 70 mg/l |
| NH₄NO₃ | 650 mg/l |
| CaCl₂·2H₂O | 40 mg/l |
| KH₂PO₄·H₂O | 70 mg/l |
| MnSO₄·H₂O | 22.3 mg/l |
| H₃BO₃ | 8.2 mg/l |
| ZnSO₄·7H₂O | 8.6 mg/l |
| Kl | 0.83 mg/l |
| CuSO₄·5H₂O | 0.025 mg/l |
| NaMoO₄·2H₂O | 0.25 mg/l |
| CoCl₂·6H₂O | 0.025 mg/l |
| FeSO₄·7H₂O | 27.8 mg/l |
| Na₂EDTA | 37.3 mg/l |

| Murashige and Skoog vitamine solution: | |
|---|---|
| myo-inositol | 100 mg/l |
| Nicotinic acid | 0.5 mg/l |
| Thiamine·HCl | 0.1 mg/l |
| Pyridoxine·HCl | 0.1 mg/l |
| Glycine | 2 g/l |
| Saccharose | 20 g/l |
| pH= 5.7 (adjusted with KOH) | |
| Phytoagar (GIBCO BRL) 8 g/l | |
| | |
| Sterilization: 121 C°, 20 minutes. | |

### Example 14

### Immunological detection of the NORK protein

In order to prove the presence of NORK protein in the cells of alfalfa, and in the transformed tobacco, a non-legume plant, two exons (2nd and 3rd exons) of the N-terminal (5') part of the NORK gene were expressed in *Escherichia coli.* Antibodies were produced against the purified proteins and the NORK protein was detected by immunological method (dot blot analysis).

### Example 14A.

### Cloning of the DNA fragment coding the N-terminal part of M. truncatula A17 NORK protein into the expression vector pTrcHisA

The pTrcHis (A, B and C) (Invitrogen BV Groningen, The Netherlands) expression vectors are made possible to express recombinant protein containing six histidine (His-tag) and a recognition site of enterokinase enzyme in fusion. The His-tag allows us to purify the recombinant protein in a single-step purification procedure (recommended by the suppliers). For this reasons this system was used to express the extracellular part of the NORK protein.

A DNA fragment of the M. *truncatula* NORK cDNA sequence described in Example 9 and showed in Figure 10 was produced by PCR amplification using the RKEx2U and Pst-A3 primers (Figure 9.) as it is described in Example 9A. The 957 bp long amplified cDNA fragment coded 319 amino acids (amino acids from 33 to 352) of the supposed extracellular part of the NORK protein. pTrcHisA vector was used for cloning to fuse the sequence of the NORK protein portion to be expressed and the His-tag in frame. The pTrcHisA vector and the amplified cDNA fragment were digested by *BamHI* and *PstI* restriction enzymes (Fermentas AB) according the supplier's instructions. The fragment and the vector prepared in this way were ligated (construct pTrcHisA::dNORK) as it is described in Example 4. The ligation is transformed into *E. coli* TOP10 competent cells detailed also in Example 4. After transformation and plating, plasmid DNA was isolated from the ampicillin resistant colonies using the QIAGEN Plasmid Isolation Kit (see Example 4.). The frame and the fidelity of the sequence of the insert were checked by sequence analysis (see Example 8.). The amplified and correctly inserted clone was termed TOP10(pTrcHisA::dNORK).

### Example 14B

### Expression of the extracellular part of the NORK protein in E. coli

The recombinant protein (rNORK) produced by the Xpress System Protein Expression system (construct pTrcHisA::dNORK) was purified by the Xpress System Protein Purification (Invitrogen BV Groningen, The Netherlands) according to the manufacturer's instructions. Overnight culture of the clone TOP10(pTrcHisA::dNORK) (see Example 14A.) grown in 2 ml of LB medium containing ampicillin in 50 µg/ml concentrations was diluted 250 times in 50 ml of the same medium. The culture was grown at 37°C with vigorous shaking to an OD₆₀₀=0.6. Then the expression of protein was induced by adding 0.5 ml IPTG solution (100 mM) and the culture was shaked for 5 more hours. Bacterial suspension was collected by centrifugation (centrifuge: Sorvall RC5, GSA rotor with the proper tubes, 4°C, 10 min, 8k rpm) than the bacterial pellet was resuspended in 10 ml of buffer (recommended by the kit: 20 mM NaH₂PO₄, 500 mM NaCl; pH 7.8). Disruption of bacteria was made by sonication 3 times for 10 sec using the smallest head of MSE Ultrasonic Disintegrator. The supernatant of the disrupted and collected bacterial suspension was bound to affinity column through the six histidine amino acid residue present in the fusion protein. Bacterial proteins were removed by three washing steps using solutions with decreasing pH (20 mM NaH₂PO₄, 500 mM NaCl; pH 7.8; 6.0 and 5.5). The recombinant protein was eluted from the column by 5 ml elution buffer of the kit (20 mM NaH₂PO₄, 500 mM NaCl; pH 4.0) having 1 mg/ml protein concentration. The eluted fusion protein was used directly to produce antibodies in rabbits.

### Example 14C

### Producing polyclonal antibodies against the extracellular part of the NORK protein

Three, 4 months-old rabbits were injected by native protein in 1 ml final volume dissolved in PBS (69) and purified as described in Example 14B, respectively. Approximately 100 µg of total purified protein (antigen) was injected subcutan into four different sites of the neck. For the primary injection 50% complete Freunds adjuvant (CFA) (cat. no: F-5881 Sigma-Aldrich Kft. Budapest) and 50% recombinant protein dissolved in PBS were used. Before the primary injection the rabbits were bled and the gained sera were used later as a negative (isotype) control.

After the fourth and seventh weeks of the primary injection boost injection was performed to achieve a heigher state of immunity (hyperimmunization). For the second injection 10% CFA + 40% incomplete Freunds adjuvant (IFA) (cat. no: F-5506 Sigma-Aldrich Kft. Budapest) and 50% recombinant protein dissolved in PBS were used. Ten days later the antibody production was checked. Serum was prepared from 1-5 ml blood obtained from ear's vein. The obtained blood was kept at room temperature for 4 hours and after coagulation it was centrifuged (2700g, 4°C, 10 min). A part of the supernatant (serum) was kept at 4°C untill utilization and the rest was stored at -20°C in 100 µl aliquots. The antibody content of the antisera was checked by ELISA (Enzyme-Linked Immunosorbent Assay) and dot blot analysis. The rabbits were kept alive and were injected again on the 7th week after the primary injection. For the third injection 50% IFA and 50% recombinant protein dissolved in PBS were used. On the 10th day after the third injection, the rabbits were bled and the samples were checked again by ELISA and dot blot analysis. Then 80 ml blood was taken from the rabbits.

### Example 14D

### ELISA

500 µg recombinant protein dissolved in PBS per well was plated into 96-well microtiter plate and incubated at 37°C for 2 hours. Then the plate was washed three times by washing buffer (0.05% Tween-20, 0.5 M NaCl in PBS) and was blocked by 0.5% gelatine (1 hr, 37°C). After three washes the plate was incubated at 37°C for 1 hour in the presence of 100 and 1000-fold diluted rabbit anti sera in 50µl washing buffer. After three repeated washes the plate was incubated (1 hr, 37°C) with the second antibodies diluted in 50µl washing buffer for 10000-fold. Horseradish peroxidase conjugated anti-rabbit IgG antibody (cat. No: A-0545 Sigma-Aldrich Kft. Budapest) was used as a secondary antibody. After four washes the plate was developed by 0.5 mg/ml OPD substrate (cat. no: P-5412 Sigma-Aldrich Kft. Budapest). OPD was dissolved in PBS containing 0.1 M citrate than 5 µl 30% H₂O₂ was added to it. After blocking the reaction by 4 M sulphuric acid (50 µl/well), the plates were read with a microplate ELISA Reader (Labsystem Multiscan Biochromatic) at 492 nm. The most specific antibody selected based on the results of ELISA test was used in 1000-fold dilution to investigate the extracts purified from roots of different plants.

### Example 14E.

### Protein purification from the roots of mutant (Nod⁻) and wild type (Nod⁺) Medicago sativa and transformed and control Nicotiana tabacum plants

Purification of proteins isolated from the roots of MN-1008 (Nod⁻) and NAB814 (Nod⁺) *Medicago sativa* as well as control (non-transformed) and transformed *Nicotiana tabacum* plants (see Example 13) were performed by the slightly modified methods of Huang and Berry (70) as well as Cheong and Hahn (59). 1 g fresh roots were homogenized in 1 ml homogenisation buffer (25 mM Tris-HCl pH 7.0, 30 mM MgCl₂, 2 mM dithiotreitol (DTT), 0.2 mM phenylmethylsulfonyl fluoride (PMSF)) in the presence of quartz sand on ice. The homogenate was filtered through four layers of cheesecloth (commercially available) and centrifuged at 6000g (4°C, 15 min). Supernatant and the crude membrane fraction obtained by centrifugation of the supernatant (20000g, 4°C, 30 min) were used for dot blot analysis.

### Example 14F

### Dot blot analysis of proteins purified from the roots of Medicago sativa and Nicotiana tabacum using specific antibodies against the NORK protein

Ten µg of protein samples purified from alfalfa and tobacco plants (the filtered and centrifuged supernatant of plant homogenized samples described in Example 14E) were dot-blotted onto nitrocellulose filter (Hybond-ECL cat. no: RPN303D, Amersham Pharmacia Biotech Inc, Little Chalfont, Buckinghamshire, England) (Figure. 32.). Dot blot analysis was performed with the 1000x diluted rabbit antisera as detailed below. Filters were blocked by blocking solution (2.5% milk powder, 1% polyvinyl-pyrrolidone (PVP), 0.1% Tween-20 in PBS) at room temperature for 1 hour. The filters were incubated in the same solution containing the generated antibodies at room temperature for another 1 hour. The filters were washed three times each for ten minutes in 50 ml washing solution (0.1 % Tween-20 in PBS). The filters were incubated further with the horseradish peroxidase-conjugated anti-rabbit IgG (5000x diluted in washing solution) at room temperature for 1 hour. After three washing steps the filters were developed by chemiluminescent reaction. After incubation of the filters in developing solution (100 mM Tris pH 8.5, 450 µM coumaric acid, 2.3 mM luminol and 0.03% H₂O₂) for 1 min, they were visualized by an X-ray film exposure (Sterling Diagnostic Imaging Inc., Newark, DE 19714 USA) (Figure 32.). The positive signal detected in the case of the Nod⁺ and Nod⁻ alfalfa as well as of the transformed tobacco plant containing the NORK gene demonstrated the reaction of the NORK protein with the produced specific antibody.

### Literature references:

1. Kiss, G.B. 1986. A szimbiotikus nitrogénkötés fokozásának lehetoségei. Növénytermelés 35:351-361.
2. Baldani, J. I., Caruso, L., Baldani, V. L. D., Goi, S. R., Dobereiner, J. 1997. Recent advances in BNF with non-legume plants. Soil Biology & Biochemistry 29: 911-922.
3. Pueppke, S. G. and Broughton, W. J. 1999. Rhizobium sp. Strain NGR234 and R. fredii USD257 share exceptionally broad, nested host range. Mol. Plant Microb. Interact. 12:293-318.
4. Norris, D.O. 1956. Legumes and the Rhizobium symbiosis. Emp. J. Exp. Agric. 24:247-270.
5. Schultze, M., Kondorosi, E., Ratet, P., Buiré, M., Kondorosi, A. 1994. Cell and molecular biology of Rhizobium-plant interaction. Int. Rev. Cytology 156:1-75.
6. Horváth, B., Kondorosi, E., John, M. , Schmidt, J., Török, I., Györgypál. Z., Barabás, I., Wieneke, U., Schell., J., Konsorosi, A. 1986. Organization, structure and symbiotic function of Rhizobium meliloti nodulation genes determining host specificity for alfalfa. Cell 46:335-343.
7. Schultze, M. , Kondorosi, A. 1995. What makes nodulation signals host-specific? Trends in Microbiology 3:370-372.
8. Long, S. R. 1989. Rhizobium-legume nodulation: life together in the underground. Cell 56: 203-214.
9. Györgypál, Z., lyer, N., Kondorosi, A. 1988. Three regulatory nodD alleles of divergent flavonoid-specificity are involved in host-dependent nodulation by Rhizobium meliloti. Mol. Gen. Genet. 212:85-92.
10. Bergman, K., Gulash-Hofee, M. , Hoverstadt, R. E., Larosiliere, R. C., Ronco, P. G., Su, L. 1988. Physiology os behavior mutants of Rhizobium meliloti: evidence for a dual chemotaxis pathway. J. Bacteriol. 170: 3249-3254.
11. Lerouge, P., Roche, P., Faucher, C., Maillet, F., Truchet, G., Prome, J. C. and Denarie, J. 1990. Symbiotic host-specificity of Rhizobium-meliloti is determined by a sulfated and acylated glucosamine oligosaccharide signal. Nature 344: 781-784.
12. Horváth, B., Bachem, C.W.B., Schell, J., Kondorosi, A. 1987. Host specific regulation of nodulation genes in Rhizobium memliloti is mediated be a plant signal, interacting with the nodD gene product. EMBO J. 6:841-848.
13. Kondorosi, E., Gyuris, J., Schmidt, J., John, M. , Duda, E., Hoffmann, B., Schell, J., Kondorosi, A. 1989. Positive and negative control of nod gene expression in Rhizobium meliloti is required for optimal nodulation. EMBO J. 8:1331-1340.
14. Caetano Annoles, G. and Gresshoff, P.M. 1991. Plant genetic control of nodulation. Ann. Rev. Microbiol. 45:345-382.
15. Bhuvanesvari, T.V:, Solheim, B. 1985. Root hair deformations in the white clover/Rhizobium trifolii symbiosis. Physiol. Plant. 63:25-34.
16. Peters, N.K., Frost, J.W., Long, S. 1986. A plant flavon, luteolin, induces expression of Rhizobium meliloti nodulation genes. Science 233:977-980.
17. Rolfe, B. G. and és Gressoff, P.M. 1988. Genetic analysis of legume nodulation. Ann. Rev. Plant Physiol. Plant Mol. Biol. 39:297-319.
18. Niebel, A., Gressent, F., Bono, J. J., Ranjeva, R., Cullimore, J. 1999. Recent advances in the study of Nod factor perception and signal transduction. Biochimie 81: 669-674.
19. Truchet, G., Barker, D. G., Camut, S., de Billy, F., Vasse, J., Huguet, T. 1989. Alfalfa nodulation in the absence of Rhizobium. Mol. Gen. Genet. 219:65-68.
20. Freiberg, C., Fellay, R., Bairoch, A., Broughton, W. J., Rosenthal, A., Perret, X., 1997. Molecular basis of symbiosis between Rhizobium and legumes. Nature 87: 394-401.
21. Covitz, P. A., L. S. Smith, Long, S. R. 1998. Expressed sequence tags from a root-hair-enriched Medicago truncatula cDNA library. Plant Physiology 117:1325-1332.
22. Gyorgyey, J., Vaubert, D., Jimenez-Zurdo, J. I., Charon, C., Troussard, L., Kondorosi, A., Kondorosi, E. 2000. Analysis of Medicago truncatula nodule expressed sequence tags. Molecular Plant-Microbe Interactions 13: 62-71.
23. Downie, J. A. and Walker, S. A. 1999. Plant responses to nodulation factors. Current Opinion in Plant Biology 2: 483-489.
24. Csanádi, G., Szécsi, J., Kaló, P., Kiss, P., Endre, G., Kondorosi, A., Kondorosi, E., Kiss, G.B. 1994. ENOD12, an early nodulin gene, is not required for nodule formation and efficient nitrogen fixation. Plant Cell 6: 201-213.
25. Schauser, L., A. Roussis, Stiller, J., Stougaard, J. 1999. A plant regulator controlling development of symbiotic root nodules. Nature 402:191-195.
26. Tanksley, S.D., Ganal, M. W., and Martin, G.B., 1995. Chromosome landing: a paradigm for map-based gene cloning in plants with large genomes. Trends in Genetics: 11: 63-68.
27. Peterson, M. A. and Barnes, D.K. 1981. Inheritance of ineffective nodulation and non-nodulation traits in alfalfa. Crop Science 21:611-616.
28. Barnes, D. K., Vance, C.P., Heichel, G.H., Peterson, M. A., Ellis, W.R. 1988. Registration of a non-nodulation and three ineffective nodulation alfalfa germplasms. Crop Sci. 28:721-722
29. Catoria, R., Galera, C., de Billy, F., Penmetsa, V., Journet, E-P., Maillet, F., Rosenberg, C., Cook, D., Gough, C., Denarie, J. 2000. Four genes of Medicago tuncatula controlling components of a Nod factor transduction pathway. The Plant Cell 12:1647-1665.
30. Walker, S.A., Viprey, V., and J. Downie, A.J. 2000. Dissection of nodulation signaling using pea mutants defective for calcium spiking induced by Nod factors and chitinoligomers. PNAS 97: 13413-13418.
31. Parniske, M. , Coomber, S., Kistner, C., Mulder, L., Pitzschke, A., Stougaard, J., Szczyglowski, K., Webb, J., Stracke, S. Plant Gnetics of symbiosis. In Abstract book of the EU conference of Molecular Genetics of Model Legumes, JIC, Norwich, June 24-28, 2000.
32. Dudley, M. E., and Long, S.R. 1989.A non-nodulating alfalfa mutant display neither root hair curling nor early cell division in response to Rhizobium meliloti. Plant Cell 1:65-72.
33. Ehrhardt, D. W., Wais, R., Long, S. R. 1996. Calcium spiking in plant root hairs responding to Rhizobium nodulation signals. Cell 85: 673-681.
34. Ambudkar, S. V., Sauna, Z. E., Muller, M. M., Kerr, K. M., Smith, M. M. 2000. Biochemical mechanism of action of multidrug resistance-linked ABC transporters. Chemistry and Physics of Lipids 107:1-15.
35. Schwiebert, E. M., Benos, D. J., Fuller, C. M. 1998. Cystic fibrosis: A multiple exocrinopathy caused by dysfunctions in a multifunctional transport protein. American Journal of Medicine 104: 576-590.
36. Song, W. Y., Wang, G. L., Chen, L. L., Kim, H: S., Pi, L. Y., Holsten, T., Gardner, J:, Wang, B., Zhai, W. X., Zhu, L. H., Fauquet, C., Ronald, P. 1995. A Receptor Kinase-Like Protein Encoded by the Rice Disease Resistance Gene, Xa21. Science 270: 1804-1806.
37. Li, J. M. and Chory, J. 1997. A putative leucine-rich repeat receptor kinase involved in brassinosteroid signal transduction. Cell 90 929-938.
38. Trotochaud, A. E., Hao, T., Wu, G., Yang, Z., Clark, S. E. 1999. The CLAVATA1 receptor-like kinase requires CLAVATA3 for its assembly into a signaling complex that includes KAPP and a rho-related protein. Plant Cell 11: 393-405.
39. Kiss, G.B., Kereszt, A., Kiss, P., Endre, G. 1998. Colormapping: a non-mathematical procedure for genetic mapping. Acta Biologica Hungarica 49:47-64.
40. Kaló, P., Endre, G., Zimányi, L., Csanádi, G., Kiss, G.B. 2000. Construction of an improved linkage map of diploid alfalfa (Medicago sativa). Theor. Appl. Genet. 100:641-657.
41. Gibson, A.H. 1980. Methods for legumes in glasshouses and controlled environment cabinets. In: Methods for evaluating biological Nitrogen Fixation. Bergersen, F.J. ed., John Wiley and Sons Ltd. p.139-184.
42. Szende, K, und Ördögh, F 1960. Die Lysogenie von Rhizobium meliloti. Naturwissenshaften 47:404-405.
43. De Lajudie, P., Willems, A., Pot, B., Dewettinck, D., Maestrojuan, G., Neyra, M. , Collins, M. D., Dreyfus, B., Kersters, K., and Gillis, M. 1994. Polyphasic taxonomy of rhizobia: Emendation of the genus Sinorhizobium and description of Sinorhizobium comb. nov., Sinorhizobium saheli sp. nov. and Sinorhizobium teranga sp. nov. Int. Journal of Systemic Bacteriol. 44:715-733.
44. Orosz L, Sváb Z, Kondorosi Á, Sík T 1973. Genetic studies on rhizobiophage 16-3. I. Genes and functions on the chromosome. Mol Gen Genet 125:341-350.
45. Michelmore, R.W., Paran, I., and Kesseli, R.V. 1991. Identification of markers linked to disease-resistance genes by bulked segregant analysis: A rapid method to detect markers in specific genomic regions by using segregating populations. Proc. Natl. Acad. Sci. USA, 88:9828-9832.
46. Williams, J.G.K., Kubelik, A.R., Livak, K.J., Rafalski, J.A. and Tingey, S.V. 1990. DNA polymorphisms amplified by arbitrary primers are useful as genetic markers. Nucl. Acids Res. 18:6531-6535.
47. Kiss, G.B., Csanádi, G., Kálmán, K., Kaló, P., Ökrész, L. 1993. Construction of a basic genetic map of Medicago using RFLP, RAPD, Isozyme and morphological markers. Mol Gen Genet 238:129-137.
48. Church, G. M. and Gilbert, W. 1984. Genomic sequencing. PNAS 81: 1991-1995.
49. Maniatis, T., Fritsch,E.F., Sambrook, J. 1982. Molecular Cloning; A Laboratory Manual; Cold Spring Harbor Laboratory. 447.oldal.
50. Vieira J., Messing J. 1991. New pUC-derived cloning vectors with different selectable markers and DNA replication origins. Gene 100:189-194.
51. Turner SR, Ireland R, Morgan C, Rawsthorne S 1992. Identification and localization of multiple forms of serine hydroxymethyltransferase in pea (Pisum sativum) and characterization of a cDNA encoding a mitochondrial isoform. Journal of Biol Chem 267:13528-13534.
52. Woo, S. S., Jiang, J. M. , Gill, B. S., Paterson, A. H. and Wing, R. A. 1994. Construction and characterization of a bacterial artificial chromosome library of Sorghum bicolor. Nucleic Acids Research 22: 4922-4931.
53. Nam YW, Penmetsa RV, Endre G, Uribe P, Kim DJ, Cook DR 1999. Construction of a bacterial artificial chromosome library of Medicago truncatula and identification of clones containing ethylene response genes. Theor. Appl. Genet. 98:638-646.
54. Koncz, C., and Rédei, G. 2000. A molekuláris genetika eszközei: Agrobacterium T-DNS és Arabidopsis. Molekuláris növénybiológia C. Könyvben, szerkesztök: Balázs E., Dudits D. Akadémiai Kiadó. 323-452. oldal.
55. Quandt, H.-J., Pühler A.,and Broer I. 1993. Transgenic root nodules of Vicia hirsuta: A fast and efficient system for the study of gene expression in indeterminate-type nodules. Mol. Plant-Microbe Interact. 6:699-706.
56. Stougaard, J. 1995. Agrobacterium rhizogenes as a vector for transforming higher plants. Application in Lotus comiculatus transformation. Methods Mol. Biol. 49:49-61.
57. Bingham, E. T., Hurley, L. V., Kaatz, D. M., and Saunders, J. W. 1975. Breeding alfalfa which regenerates from callus tissue in culture. Crop Sci. 15:719-721.
58. Brosius, J. 1989. Superpolylinkers in cloning and expression vectors. DNA. 8:759-777.
59. An, G., Watson, B. D., Stachel, S., Gordon, M. P., Nester, E.W. 1985. New cloning vehicles for transformation of higher plants. EMBO J. 4:277-284.
60. Szabados, L., Charrier, B., Kondorosi, A., de Bruijn, F., Ratet, P. 1995. New plant promoter and enhancer testing vectors. Mol. Breeding 1:419-423.
61. Hellens, R.P., Edwards, E. A., Leyland, N. R., Bean, S. Mullineaux, P.M. 2000. pGreen: a versatile and flexible binary Ti vector for Agrobacterium-mediated plant transformation. Plant Mol. Biol. 42: 819-832.
62. Vancanneyt, G., Schmidt, R., O'Connor-Sanchez, A., Willmitzer, L., Rocha-Sosa, M. 1990. Construction of an intron containing marker gene: Splicing of the intron in transgenic plants and its use in monitoring early events in Agrobacterium-mediated transformation. Mol. Gen. Genet. 220:245-250.
63. Kovach, M. E., Phillips, R. W., Elzer, P. H., Roop, R. M., Peterson, K. M. 1994. pBBR1MCS: a broad-host-range cloning vector. Biotechniques.16:800-802.
64. Pridmore, R. D. 1987. New and versatile cloning vectors with kanamycin-resistance marker. Gene 56:309-312.
65. Sharma, R. C., ans Schimke, R. T. 1996. Preparation of electrocompetent E. coli using salt-free growth medium. Biotechniques. 20:42-4.
66. Shahin, E. A. 1985. Totipotency of tomato protoplasts. Theor. Appl. Genet. 69:235-240.
67. Jefferson, R. A. 1989. The GUS reporter gene system. Nature 342:837-838.
68. Murashige, T., and Skoog, F. 1962. A revised medium for rapid growth and bioassays with tobacco tissue cultures. Physiol. Plant. 15:473-497.
69. Coico, R., Coligan, J.E., Kruisbeek, A.M., Margulies, D.H., Shevach, E.M., and Strober, W. Current Protocols in Immunology 1994. John Wiley & Sons Inc. USA.
70. Huang, L. S. and Berry, E. A. 1990. Purification and characterization of the proton translocating plasma membrane ATPase of red beet storage tissue. Biochim Biophys Acta 1039:241-52.
71. Cheong, J. J., and Hahn, M. G. 1991. A specific, high-affinity binding site for the hepta-beta-glucoside elicitor exists in soybean membranes. Plant Cell 3:137-47.

### organization Applicant

street : Temesvári krt. 62
City : szeged
state :
Country : Hungary
Postal Code : H-6726
PhoneNumber :
FaxNumber :
EmailAddress :
<110> OrganizationName : MTZA Szegedi Biológiai Központ Genetikai Intézete

### organization Applicant

street : Temesvári krt. 62
City : szeged
state :
Country : Hungary
PostalCode : H-6726
PhoneNumber :
FaxNumber :
EmailAddress :
<110> OrganizationName : MTA Szegedi Biológiai Központ Genetikai Intézete

### Application Project

<120> Title : Identification and cloning of receptor gene for symbiotic nitrogen fixation
<130> AppFileReference : 74.766/SZE
<140> CurrentAppNumber : PCT/HU02/00055
<141> CurrentFinngDate : 2002-06-19

### Earlier Applications

<150> PriorAppNumber : P0102561
<151> PriorFilingDate : 2001-06-20

### sequence

<213> OrganismName : Medicago truncatula A17
<400> PresequenceString :
<212> Type : DNA
<211> Length : 8564
   SequenceName : SEQ ID No. 8
   SepuenceDescription :

### custom codon

Sequence Name : SEQ ID No. 8

### Sequence

<213> OrganismName : Medicago truncatula A17
<400> PreSeauenceStrinq :

<212> Type: DNA
<211> Length : 3362
   SequenceName : SEQ ID No. 10
   SequenceDescription :

### Custom codon

Sequence Name : SEQ ID No. 10

### Sequence

<213> OrganismName : Medicago truncatula A17
<400> PreSequenceString :

<212> Type: PRT
<211> Length : 925
   SequenceName : SEQ ID No. 12
   SequenceDescription :

### Sequence

<213> OrganismName : Medicago sativa Nod-
<400> PreSequenceString :

<212> Type : DNA
<211> Length : 3378
   SequenceName : SEQ ID No. 14
   SequenceDescription :

### Custom codon

Sequence Name : SEQ ID No. 14

### Sequence

<213> organismName : Medicago sativa Nod+
<400> PreSequenceString:

<212> Type : DNA
<211> Length : 3339
   SequenceName : SEQ ID No. 15
   SequenceDescription :

### Custom codon

Sequence Name : SEQ ID No. 15

### Sequence

<213> OrganismName : Medicago sativa Nod-
<400> PreSequenceStnng :

<212> Type : PRT
<211> Length : 714
   sequenceName : SEQ ID No. 16
   SequenceDescription :

### Sequence

<213> OrganismName : Medicago sativa Nod+
<400> PreSequenceString :

<212> Type : PRT
<211> Length : 931
   SequenceName : SEQ ID No. 17
   SequenceDescription :

### Sequence

<213> OrganismName : Pisum sativum cv. Frisson
<400> PreSequenceString :

<212> Type : DNA
<211> Length : 3360
   sequenceName : SEQ ID No. 28
   SequenceDescription :

### Custom Codon

Sequence Name : SEQ ID No. 28

### Sequence

<213> OrganismName : Vicia villosa s-1
<400> PresequenceString :

<212> Type : DNA
<211> Length : 3420
   SequenceName : SEQ ID No. 29
   sequenceDescription :

### Custom Codon

sequence Name : SEQ ID No. 29

### Sequence

<213> OrganismName : Pisum sativum cv. Frisson
<400> PreSequencestring :

<212> Type : PRT
<211> Length : 922
   SequenceName : SEQ ID No. 30
   sequenceDescription :

### Sequence

<213> OrganismName : vicia villosa S-1
<400> PreSequenceString :

<212> Type : PRT
<211> Length : 1020
   SequenceName : SEQ ID No. 31
   SequenceDescription :

### SEQUENCE LISTING

<110> MTA Szegedi Biologiai Központ Genetikai Intézete
<120> Identification and cloning of receptor gene for symbiotic nitrogen fixation
<130> 74.7661SZE
<140> PCT/HU02/00055
   <141> 2002-06-19
<150> P0102561
   <151> 2001-06-20
<160> 11
<170> Patentin version 3.1
<210> 1
   <211> 8564
   <212> DNA
   <213> Medicago truncatula A17
<220>
   <221> misc_feature
   <222> (3302)..(3302)
   <223> unknown
<220>
   <221> misc_feature
   <222> (5037)..(5037)
   <223> unknown
<400> 1
   gctagcttaa tttgtgaagt agttttcagt cagctcatgc tgaacagtta tggtagtttt 60
   acaaggatgt aaggggttac ttgctctttt gtggtatcat tgagccactt tccactttct 120
   taattctttg ctatcaaatt taaatatatt atattattgt aggaaatatg aaaaagaaac 180
   cctagatgct ttaattgtgc taagcatata agatacaaaa aaaaaaaagt ctaatctctt 240
   ttgataggaa actgacacaa ggttcgatcc tcagaaccaa ctatgagact aatttttgct 300
   tgaattggtt aatcaactgc atcgcactca ttgaataaaa aagtacgttt agattgacgg 360
   tgaatttaac ataatcacag ttgaccactt tgattttcac gaaagttgca tgtaactttc 420
   gtgcttgaat ataaagtcac ggtgccacaa tggttttgtc aatatcgccg tgaatccaaa 480
   catgcactta atatgttttt atattaatgg aaacctatgt atataaatat aaaatactta 540
   agcatatata tccttgcaac aataccaaaa aagtttaaaa tttgctccaa agatactatt 600
   cacggtttct attgtgatgc atccatggaa tgagaaggaa gaaaacagaa gaagacgaag 660
   gactggtctg tccaattatt taaaccatat gaatattttt atatacagat aatgaagcaa 720
   ccaccactaa aacaataaga ataaataata atgttcaaca tcatcaataa taagaataat 780
   aatgtttaac atcaacaata aaactatagg aaaaaaacat aatcaactat gcattgtact 840
   aattcaatct ctaactcgtc ttccatctct ttcctagcta cctcctgcag tttcctttcc 900
   aggcctaaag tcaaacacca tattttaaca atattctttc ttctacagct tttatacaag 960
   ttcactatat tataggattg atcagggttc attttttctt tctttgaaaa atctctaagg 1020
   ggtgctgttt ccaaggcaga gaatgaaata gaattcagaa gaatttttat gttactataa 1080
   aggaaagatg aaaagttagt tagcatggat tcaagtttga taaccctatg gggtaaaatc 1140
   tctttcagat tatgatggag ttacaagtta ttaggatatt tagattggtt gtggcatttg 1200
   ttctttgttt gtgtatattt atcagatcag cttcttctgc aactaaaggt tagtagctaa 1260
   atactataat tctttaagat cataataata tctattactt gatttctttc cctttaaaca 1320
   tagaaaacaa catattttaa ttaacatgaa aggccatggg atgatcataa ttaataatga 1380
   gaagaaaata aataagaaac ttgttttttt tacaaggatt gtaaaataga actagtagtt 1440
   tcatactttc aatactgaga atcttgaaac aatttcactt tttctttatg ttgctagaat 1500
   ttctttcaag ggaaaatcca attttgtaca aaatgaattt aacttgtgac attttccttg 1560
   tagggtttga gagcatagca tgttgtgctg attcaaatta cacagatcca aaaaccaccc 1620
   taacttatac aacagatcac atctggttct ctgataaaag aagttgcaga caaatacccg 1680
   aaattttgtt tagccacaga agcaataaaa atgttcgaaa atttgaaata tatgaaggaa 1740
   agagatgtta taatttgcca acagttaagg atcaagtata tttgataagg ggcatatttc 1800
   cctttgatag tttaaattct tcgttttatg tttcgatcgg ggtaacagaa ctaggcgaat 1860
   taagatcgtc taggctcgag gacttggaaa ttgagggagt ttttagagcc accaaagact 1920
   acatagattt ctgcttattg aaggaggatg tcaatccctt catttctcag attgaattga 1980
   ggccattacc tgaagaatac ctacatggtt tcggtactag tgttttaaaa ctgataagca 2040
   gaaacaatct tggtgacaca aatgatgata taaggtatgt gatcttactt tatttttagg 2100
   tagattccac ctctatttta cagggagtgt ctctcaggaa acctaaaagg cttagggttt 2160
   gtcactactt ggttcttatg gaagcatcat atgttcatac ttagtagata tatattgtat 2220
   aaaaatcaac atctttctcc gtaagggata gaaattgcaa ttcatgttat gttgagcata 2280
   tttaaaccaa aataatttga ggaatatgat gcatgaacct ttctgccaaa tgcatgacat 2340
   aacctatgtt tcactttaca tatagaatta taagatgtgt ttacatcttt atattaactt 2400
   tgatctttat gatgcactga gatacacctg aatttaaata acaaatggag aagcaagaat 2460
   ctagaactct accattcaat ccattgtaat atggaactca caagaattaa ctattgccac 2520
   ttcagattaa ataccctaag agtgttttgt ggatgaggga atgttttgag ggaatgtaat 2580
   gttttgaggg aattcctcaa aacattacat tacctcaaaa tattccccca tccaaacaca 2640
   ctataaagga tcctaatcta gtgtagcctt agttttctat tgaagtgatt tgtgtttaga 2700
   gtttggtctg catctgcaac tttgttttgt tgctgtaatg ccatgactat gaacactgac 2760
   agaaaggtct cataatatcg gtatctatca attttaggtt cccagatgac caaaatgata 2820
   gaatctggaa acggaaagaa acttcaactc caacatctgc ccttccactg tctttcaatg 2880
   tcagcaatgt tgacctcaaa gacagtgtca cacctcctct acaagtccta caaacagctc 2940
   ttactcaccc tgagcgattg gagttcgtcc atgatggcct cgagaccgat gattatgaat 3000
   actctgtgtt tctccacttt cttgaactaa atggcactgt cagagcagga caaagggtgt 3060
   ttgacatcta tctaaacaat gagattaaaa aggagaaatt tgatgttttg gctggagggt 3120
   ccaagaacag ttacactgcc ttgaacattt cagcaaatgg atcactcaat ataaccttag 3180
   tcaaggcatc tggatctgag tttggacccc ttttgaatgc ctatgaaatc ctgcaggcac 3240
   ggtcgtggat tgaagagacc aaccaaaaag attgtaagtg tacaataaga tgctaaattg 3300
   angaatttta ttaaccttta accaattttc tatttcattc tccctcttac actaacactt 3360
   tttttccttt cagtggaagt tattcagaag atgagagaag aactgctgct gcacaaccaa 3420
   gaaaatgaag cattggagag ttggagtgga gacccttgta tgattttccc ctggaaagga 3480
   ataacatgtg atgattcaac tggttcatct attatcacta agctgtaagt ccttccactt 3540
   tttagggtct gtttagattt gcttatttga gtttatctat tgaaataaac acttatgaca 3600
   ctgtttggaa gagcttatga aaacaactta tagtttatac gaaaacaagt tgactttgtt 3660
   atatctattt tatagaaata gcttataagt aagaacttat atgataagcg tttatgctat 3720
   aaacgctcaa tttaactgtt tatccaaaca ggactttaat gcatccatat gtgtttacaa 3780
   gttttgctct attttctgca gggatctttc ttccaataat ctcaagggag caattccttc 3840
   cattgtcact aagatgacca atttacaaat actgtactgc ttctaaatct catttaaaaa 3900
   catccacatt atttttgtgg gaacagtgga ctgtttttat ccattagtta ataatgttgt 3960
   aacatttttg ttatgcgcag gaactgagcc acaaccagtt cgatatgtta ttcccctcgt 4020
   ttccaccgtc ctccttgctg atatcattgt aattatctct tctcatgttt aacaaatgaa 4080
   ctaggatgat actaatatga gcttatagca cttctattat ccttgtaaat gttaacataa 4140
   acaagcacat gcagattata gaactaaaat atgatagaat atgctgtata tagtcccaac 4200
   tcatgttggt tacataaggt agatattaga cagtctcaat gctgaaacca atatctgtgt 4260
   gcatgatgca tcttaatctt taaaagattt tatgattaag gctgttctct atggcacttc 4320
   agtaggagca gtaacacatc tatgtgaaag ttccatatga aatccttgag aaatatgttt 4380
   tgacattatg tttcatatat tgctgaattt ctttcttcac caaggttcga atcttagaga 4440
   aatttcctac agttattagt gccattgaaa aattactact aaatcttttt caaatgattg 4500
   atctacattt atggatcagg gatcttagct acaatgatct ttcaggatgg cttccagaat 4560
   ccattatctc actgccacat ttaaaatcat tgtaagtttt atatgttggc attctacttc 4620
   atccatatta ggaagctatt ttcgattgtt gtatattttt aatatacttc atttattcag 4680
   cttgtatttg atttatttcc agatattttg gctgcaatcc atctatgagt gacgaagata 4740
   caacaaagtt gaacagttca ctaatcaata cagagtatga agtattattg atgcataaca 4800
   atagaagttt ttaaaataaa ataaaataaa tcaaaatttt actcgtttct gttttcacag 4860
   ttatgggaga tgcaaagcaa aaaaaccaaa gtttggacaa gtattcgtga ttggagctat 4920
   tacaagtgga tcacttttga ttactttggc tgttggaatt ctattttttt gccgttatag 4980
   acacaagtca attactttgg aaggatttgg aaagacctac ccaatggcaa caagtangca 5040
   ttttatcttt tataatctat tgtaacatat gtctatctgc attctaatgt aacatatttg 5100
   ctctttagac taacaaacaa aaactatagt cacatcattc tgcatagaca atctcagatc 5160
   acaagatcta agtgttatct ttaacataga gcatggttta caaatacagg gaatacattt 5220
   ttataaccct gagattaaat caacttttct tatacagtca gacaggatct gcacataatg 5280
   ttcaacacaa tgataaaata agagaaatat atttaaaaaa actaacatcc agtgtgaatt 5340
   cctagatata tggctacaaa aaaatctata ttccatctgt acaataacaa gaatatcaat 5400
   tgaaaaaaga atacttatga acatgataga tgatattagt ttctgtagag gatcattact 5460
   atgatggaag tgatgatttt agcatgtttg gtttggtcta atctacattg tatatatgac 5520
   attctccaac aaaaacatgc agatataatt ttctctttgc caagcaaaga cgatttcttc 5580
   ataaagtctg tatcagttaa accgttcact ttggagtata tagagcaggc tacagaacag 5640
   tacaaaactt taataggtga aggaggattt ggctctgttt acaggggcac tctagacgat 5700
   ggtcaagaag tggcagtgaa agtgcggtca tccacatcaa ctccgggaac ccgagaattt 5760
   gataatgagg tataatatgc attatcactt tattagagca acaagattcc cacagaatgt 5820
   tcctaattaa agctttggcc atatatatag tagtttaaga atttgttcgg ttcctatcac 5880
   accatctgat tggtctatga tggatcatgc agctaaacct actttcagct atacaacatg 5940
   agaacctggt gcctcttctg ggttactgta atgagtatga tcaacaaatt ctcgtgtatc 6000
   ctttcatgtc caatggctct ttgctagata gactatacgg taaatatctc acaattcttt 6060
   tagcagatat gttttaatac acagtaattc tgaaatttaa tttcataaca ggggaagcat 6120
   caaagagaaa aatattagac tggccaacta gactctctat tgctctcggt gcagctcagg 6180
   caagtaccat gatgttgttt tacttttact aagttgtgca tatgtactaa ttaagcttgc 6240
   atcatatcag agaggataaa acatgaaatt gtatgtaaac atttcatatg aaaacataaa 6300
   actaattcaa atgaacaatg aaaaaagaga agcgtataca atgattatgt cttaccacag 6360
   gcgcagggac ttgaatataa gatacatatt gatttctcta ctgaacatat aaactggaga 6420
   caatcacaag caaaatgatg aggctatatt gattgacaag gacaatcata ttgttttact 6480
   gtgattagaa cgatacagta aagatatttt agagggaata ctgtttacta ctataagagt 6540
   gactgatagt tcggatgttg ctaatggaaa ttgataagat ggtcaacatt gacttagtga 6600
   aatctgtctg acaactaagt attttggccc tcacggatct actttggcat aaagaaaaac 6660
   ttcttgaaat gatgcatcct aaagttcttt attcttcaca taataaactc agtttttgac 6720
   ctaactgact tcactacagt tttcgtccat aacatattta ggacactaaa atgatcataa 6780
   gttttctgat tgagcaggtt tggcatatct tcacacattt ccaggacgtt ctgtaataca 6840
   cagggacgta aaatcgagca atatactgct ggatcagagc atgtgtgcta aggttgcaga 6900
   ttttggtttc tcaaaatatg ctcctcagga aggagacagt tatgtttccc ttgaagtaag 6960
   aggaactgca gggtatctgg atcctgagta agtgaactta aatccgtctt aatctgaatg 7020
   tctcatactg ctctctcatc tatcactttc agcaaaatat tcaatacatt tcccacacac 7080
   aagtttgttg gaaccatcag aaaactgaag aaatgtttct gattatattc ctattctgaa 7140
   tctgaagtgt atattaattt taaacataca aaactaaagt tcccatatgg tgactacaaa 7200
   tgataagaga tatttatcca tgtgaatttg aatgggatga agaagaatct aacataccta 7260
   gtcccatttg tttcacatgt tacagagagt aaccatgaca tgaacaaatt tcacaatagc 7320
   tgacgtttat ccatatgcag gtactacaaa acccagcaat tatctgaaaa aagtgatgtt 7380
   ttcagctttg gtgtggttct tcttgaaatt gtaagcggac gggaacctct caacataaag 7440
   agaccaagga tcgagtggag cttggttgaa tgggtatgat ccatgacccc atttttttta 7500
   caaatataat tttcaagtgg ttcatatatt ttagtaggta ctaacacata actttcatgc 7560
   aggctaaacc atacataaga gcatcaaagg tggatgaaat tgtagatcct ggcatcaagg 7620
   gaggatatca tgcagaggca ttgtggagag ttgtggaagt agcactacaa tgtctagaac 7680
   cctactcaac atatcggcca tgcatggttg atattgtccg cgagttggag gatgctctca 7740
   ttattgaaaa caatgcatct gaatacatga aatccataga cagccttgga ggatccaacc 7800
   gctactcaat tgttatggac aaacgggcgc tgccttcaac tacatctaca gcagaatcaa 7860
   ctatcacaac ccaaaccttg tcacaccctc aaccgagata gtaaatgggt cgatggaatt 7920
   cttttgattt gtttttgatc attgctttag taatatcaca ttttaaatgg taaaggagaa 7980
   aaatactact tctgattgta tttccatcca ctctatgttt cttgaaactg aatctctctt 8040
   gctcagcccc agtttttatg ggtgaaaaga ataatttggg tcaaatgcaa gtgaaaccat 8100
   atggtgcata atttaaaagc catatcatat catttgccaa gtccaaagta aaaatttcac 8160
   aaactagtta gattgcgatt tagtctatag acacttcaac agagctatat acactatggt 8220
   tgacttgcga ctaattcgct caagcaggag gaacacatat atatgggaaa cacttgtaga 8280
   actattttgt ttatagaaat gaaaatattt tccgttttta atttttgttt tcattaagtt 8340
   tgaattattt ttactttggt tcatacatga ttctaaatta tgtttgaagt tatggtaaga 8400
   aagttgtcat tgcgaaaaaa tacggccgca attgccgctg tgcatcttca gctcaagtga 8460
   ttgtctcttg gtgattgttc aacagaccct caacattgct catcctatca gaacccaact 8520
   tccaataggc atttgttcta cttaaaagaa aatcggaagc tagc 8564
<210> 2
   <211> 3362
   <212> DNA
   <213> Medicago truncatula A17
<400> 2
<210> 3
   <211> 925
   <212> PRT
   <213> Medicago truncatula A17
<400> 3
   Met Met Glu Leu Gln Val Ile Arg Ile Phe Arg Leu Val Val Ala Phe
   1 5 10 15
   Val Leu Cys Leu Cys Ile Phe Ile Arg Ser Ala Ser Ser Ala Thr Lys
   20 25 30
   Gly Phe Glu Ser Ile Ala Cys Cys Ala Asp Ser Asn Tyr Thr Asp Pro
   35 40 45
   Lys Thr Thr Leu Thr Tyr Thr Thr Asp His Ile Trp Phe Ser Asp Lys
   50 55 60
   Arg Ser Cys Arg Gln Ile Pro Glu Ile Leu Phe Ser His Arg Ser Asn
   65 70 75 80
   Lys Asn Val Arg Lys Phe Glu Ile Tyr Glu Gly Lys Arg Cys Tyr Asn
   85 90 95
   Leu Pro Thr Val Lys Asp Gln Val Tyr Leu Ile Arg Gly Ile Phe Pro
   100 105 110
   Phe Asp Ser Leu Asn Ser Ser Phe Tyr Val Ser Ile Gly Val Thr Glu
   115 120 125
   Leu Gly Glu Leu Arg Ser Ser Arg Leu Glu Asp Leu Glu Ile Glu Gly
   130 135 140
   Val Phe Arg Ala Thr Lys Asp Tyr Ile Asp Phe Cys Leu Leu Lys Glu
   145 150 155 160
   Asp Val Asn Pro Phe Ile Ser Gln Ile Glu Leu Arg Pro Leu Pro Glu
   165 170 175
   Glu Tyr Leu His Gly Phe Gly Thr Ser Val Leu Lys Leu Ile Ser Arg
   180 185 190
   Asn Asn Leu Gly Asp Thr Asn Asp Asp Ile Arg Phe Pro Asp Asp Gln
   195 200 205
   Asn Asp Arg Ile Trp Lys Arg Lys Glu Thr Ser Thr Pro Thr Ser Ala
   210 215 220
   Leu Pro Leu Ser Phe Asn Val Ser Asn Val Asp Leu Lys Asp Ser Val
   225 230 235 240
   Thr Pro Pro Leu Gln Val Leu Gln Thr Ala Leu Thr His Pro Glu Arg
   245 250 255
   Leu Glu Phe Val His Asp Gly Leu Glu Thr Asp Asp Tyr Glu Tyr Ser
   260 265 270
   Val Phe Leu His Phe Leu Glu Leu Asn Gly Thr Val Arg Ala Gly Gln
   275 280 285
   Arg Val Phe Asp Ile Tyr Leu Asn Asn Glu Ile Lys Lys Glu Lys Phe
   290 295 300
   Asp Val Leu Ala Gly Gly Ser Lys Asn Ser Tyr Thr Ala Leu Asn Ile
   305 310 315 320
   Ser Ala Asn Gly Ser Leu Asn Ile Thr Leu Val Lys Ala Ser Gly Ser
   325 330 335
   Glu Phe Gly Pro Leu Leu Asn Ala Tyr Glu Ile Leu Gln Ala Arg Ser
   340 345 350
   Trp Ile Glu Glu Thr Asn Gln Lys Asp Leu Glu Val Ile Gln Lys Met
   355 360 365
   Arg Glu Glu Leu Leu Leu His Asn Gln Glu Asn Glu Ala Leu Glu Ser
   370 375 380
   Trp Ser Gly Asp Pro Cys Met Ile Phe Pro Trp Lys Gly Ile Thr Cys
   385 390 395 400
   Asp Asp Ser Thr Gly Ser Ser Ile Ile Thr Lys Leu Asp Leu Ser Ser
   405 410 415
   Asn Asn Leu Lys Gly Ala Ile Pro Ser Ile Val Thr Lys Met Thr Asn
   420 425 430
   Leu Gln Ile Leu Asn Leu Ser His Asn Gln Phe Asp Met Leu Phe Pro
   435 440 445
   Ser Phe Pro Pro Ser Ser Leu Leu Ile Ser Leu Asp Leu Ser Tyr Asn
   450 455 460
   Asp Leu Ser Gly Trp Leu Pro Glu Ser Ile Ile Ser Leu Pro His Leu
   465 470 475 480
   Lys Ser Leu Tyr Phe Gly Cys Asn Pro Ser Met Ser Asp Glu Asp Thr
   485 490 495
   Thr Lys Leu Asn Ser Ser Leu Ile Asn Thr Asp Tyr Gly Arg Cys Lys
   500 505 510
   Ala Lys Lys Pro Lys Phe Gly Gln Val Phe Val Ile Gly Ala Ile Thr
   515 520 525
   Ser Gly Ser Leu Leu Ile Thr Leu Ala Val Gly Ile Leu Phe Phe Cys
   530 535 540
   Arg Tyr Arg His Lys Ser Ile Thr Leu Glu Gly Phe Gly Lys Thr Tyr
   545 550 555 560
   Pro Met Ala Thr Asn Ile Ile Phe Ser Leu Pro Ser Lys Asp Asp Phe
   565 570 575
   Phe Ile Lys Ser Val Ser Val Lys Pro Phe Thr Leu Glu Tyr Ile Glu
   580 585 590
   Gln Ala Thr Glu Gln Tyr Lys Thr Leu Ile Gly Glu Gly Gly Phe Gly
   595 600 605
   Ser Val Tyr Arg Gly Thr Leu Asp Asp Gly Gln Glu Val Ala Val Lys
   610 615 620
   Val Arg Ser Ser Thr Ser Thr Gln Gly Thr Arg Glu Phe Asp Asn Glu
   625 630 635 640
   Leu Asn Leu Leu Ser Ala Ile Gln His Glu Asn Leu Val Pro Leu Leu
   645 650 655
   Gly Tyr Cys Asn Glu Tyr Asp Gln Gln Ile Leu Val Tyr Pro Phe Met
   660 665 670
   Ser Asn Gly Ser Leu Leu Asp Arg Leu Tyr Gly Glu Ala Ser Lys Arg
   675 680 685
   Lys Ile Leu Asp Trp Pro Thr Arg Leu Ser Ile Ala Leu Gly Ala Ala
   690 695 700
   Arg Gly Leu Ala Tyr Leu His Thr Phe Pro Gly Arg Ser Val Ile His
   705 710 715 720
   Arg Asp Val Lys Ser Ser Asn Ile Leu Leu Asp Gln Ser Met Cys Ala
   725 730 735
   Lys Val Ala Asp Phe Gly Phe Ser Lys Tyr Ala Pro Gln Glu Gly Asp
   740 745 750
   Ser Tyr Val Ser Leu Glu Val Arg Gly Thr Ala Gly Tyr Leu Asp Pro
   755 760 765
   Glu Tyr Tyr Lys Thr Gln Gln Leu Ser Glu Lys Ser Asp Val Phe Ser
   770 775 780
   Phe Gly Val Val Leu Leu Glu Ile Val Ser Gly Arg Glu Pro Leu Asn
   785 790 795 800
   Ile Lys Arg Pro Arg Ile Glu Trp Ser Leu Val Glu Trp Ala Lys Pro
   805 810 815
   Tyr Ile Arg Ala Ser Lys Val Asp Glu Ile Val Asp Pro Gly Ile Lys
   820 825 830
   Gly Gly Tyr His Ala Glu Ala Leu Trp Arg Val Val Glu Val Ala Leu
   835 840 845
   Gln Cys Leu Glu Pro Tyr Ser Thr Tyr Arg Pro Cys Met Val Asp Ile
   850 855 860
   Val Arg Glu Leu Glu Asp Ala Leu Ile Ile Glu Asn Asn Ala Ser Glu
   865 870 875 880
   Tyr Met Lys Ser Ile Asp Ser Leu Gly Gly Ser Asn Arg Tyr Ser Ile
   885 890 895
   Val Met Asp Lys Arg Ala Leu Pro Ser Thr Thr Ser Thr Ala Glu Ser
   900 905 910
   Thr Ile Thr Thr Gln Thr Leu Ser His Pro Gln Pro Arg
   915 920 925
<210> 4
   <211> 3378
   <212> DNA
   <213> Medicago sativa Nod-
<400> 4
   ttccaggcct aaagtcaaac accatatttt aacaatattc tttcttctac aagggtataa 60
   cttttataca agttcactat attataggat tgatcaaggt tcattttttc tttctttgaa 120
   aaatctctaa ggggtgctgt ttccaaggca gaaaatgaaa tagaattcag aagaattttt 180
   atggtactaa agggaagatg agaagttagt tagcatggat tcaaatttga taaccctttg 240
   gggtaaaatc tctttcagat tataatggag ctacaagtta ttaggatatt tagattggtt 300
   gtggcatgtg ttctttgttt gtgtatattt atcagatcag cttcttctgc aactaaaggg 360
   tttgagagca tagcatgttg tgctgattcc aattacacag atccaaaaac caccctaact 420
   tatacaacag atcacatctg gttctctgat aaaagaagtt gcagaccaat acccgaaatt 480
   ttgtttagcc acagaagcaa taaaaatgtt cgaatatttg aaatagatga aggaaagaga 540
   tgttatactt tgccaacaat taaggatcaa gtatatttga taaggggtgt atttcccttt 600
   gatagtttaa attcttcgtt ttatgtttat atcggggtaa cagaactagg tgaattaaga 660
   tcgtctagac tcgaggactt ggaaattgag ggagttttta gagccaccaa agactatatt 720
   gatttctgct tattgaagga ggatgtcaat cccttcattt ctcagattga attgaggcca 780
   ttacctgaag aatacctaca tggtttcgct actagtgttt taaaactgat aagcagaaac 840
   aatcttggtg acataaatga tgatatcagg ttcccagatg accgaaatga tagaatctgg 900
   aaacggaaag caacttcaac tccatcatct gcccttccac tgtctttcaa tgtcagcaat 960
   gttgacctca aagacagtgt cgcacctcct ctacaagtcc tacaaacagc tcttactcac 1020
   cctgagcgat tggagtttgt ccatgatggc ctcgagaccg atgattatga atactctgtg 1080
   tttctccact ttcttgaact aaatggcact gtcagagcag gacaaagggt gtttgacatc 1140
   tatctaaaca atgagattaa aaaggagaag tttgatgttt tggctggagg gtccaagaac 1200
   agttacactg ccttgaacat ttcagcaaat ggatcactca atataacctt agtcaaggca 1260
   tctggatctg agtttggacc ccttttgaat gcctatgaaa tcctgcaggc acggtcgtgg 1320
   attgaagaga ccaaccaaaa agatttggaa cttattcaga agacaagaga agaactgctg 1380
   ctgcacaacc aagaaaatga agcattggag agttggagtg gagacccttg tatgattttc 1440
   ccctggaaag gaataacatg tgatgattca actggttcat ctattatcac tatgctggat 1500
   ctttcttcca ataatctcaa gggagcaatt ccttactttg tcactaagat gaccaattta 1560
   caaatactga acctgagcca caaccagttc gattcgttat tcccctcgtt tccaccgtcc 1620
   tccttgctga tatcattgga tctgagctac aatgatcttg atggacggct tccagaatcc 1680
   attatctcac tgccacattt aaaatcatta tattttggct gcaatccata tatgaaggac 1740
   gaagatacaa caaagttgaa cagttcacta atcaatacag attatgggag atgcaaagga 1800
   aaaaaaccaa agtttggaca agtattcgtg attggagcta ttacaagtgg atcacttttg 1860
   attactttgg ctgttggaat tctatttttt tgccgttata gacacaagtc aattactttg 1920
   gaaggatttg gtggaaagac ctacccaatg gcaacaaata taatcttctc tttgccaagc 1980
   aaagacgatt tcttcataaa gtctgtatca gttaaaccat tcactttgga gtatatagag 2040
   caggctacag aacagtacaa aactttgata tgtgaaggag gatttggttc tgtttacaga 2100
   ggcactctag acgatggtca agaagtggca gtgaaagtgc ggtcatccac atcaactcag 2160
   ggaaccaagg aatttgataa cgagctaaac ctactttcag ctatacaaca tgagaacctg 2220
   gtgcctcttc tgggttactg taatgagtat gatcaacaaa ttctcgtgta tccattcatg 2280
   tctaatggct ctttgctaga tagattatac ggggaagcat caaagagaaa aatattagac 2340
   tggccaacta gactctctat tgctctcggt gcacctcgag gtttggcata gcttcacaca 2400
   tttccaggac cttctgtaat acacagggac ctaaaatcga gcaatatact gctggatcag 2460
   agcatgtgtg ctaaggttgc agattttggt ttctcaaaat acgctcctca ggaaggagac 2520
   agttatgttt cccttgaagt aagaggaact gcagggtatc tggatcctga gtactacaaa 2580
   acccagcaat tatctgaaaa aagtgatgtt ttcagctttg gtgtggttct acttgaaatt 2640
   gtaagtggac gggaacctct caacataaag agaccacgga tcgagtggag cttggttgaa 2700
   tgggctaaac catacataag agcatcaaag gtggatgaaa ttgtagatcc tggcatcaag 2760
   ggaggatatc atgcagaagc attgtggaga gttgtggaag tagcactgca atgtctagaa 2820
   ccctactcaa catataggcc atgcatggtt gatattgtcc gcgagttgga ggatgctctc 2880
   attattgaaa acaatgcatc tgaatacatg aaatccatag acagccttgg aggatccaac 2940
   cgctactcaa ttgttatgga caaacgggcg ctgccttcaa ctacatctac agcagaatca 3000
   actatcacaa cccaaacctt gacacaccct caaccgagat agtaaatggg tcgatggaat 3060
   tcttttgatt tgtttttgat cattgcttta gtaatatccc attttcaatg gtaaaggaga 3120
   aaaatactac ttttgattgt attttcatcc actctatgtt tcttgaaact gaatctctct 3180
   tgctcagccc cagtttttat gggtgaaaag aataatttgg gtcaaatgca agtgaaacca 3240
   tatggtgcat aatttgaaag ccatattata tcatttgcca agtccaaagt aaaaatttca 3300
   caaactagtt agattgcgat ttagtctata gacacttcaa cagagctata tacactatgg 3360
   ttgacttgcg actaattc 3378
<210> 5
   <211> 3339
   <212> DNA
   <213> Medicago sativa Nod+
<220>
   <221> N
   <222> (2149)..(2149)
   <223> unknown
<220>
   <221> misc_feature
   <222> (2149)..(2149)
   <223> unknown
<220>
   <221> misc_feature
   <222> (2149)..(2149)
   <223> unknown
<400> 5
   ccatatttta acaatattct ttcttctaca agggtataac ttttatacaa gttcactata 60
   ttataggatt gatcaaggtt cattttttct ttctttgaaa aatctctaag gggtgtggtt 120
   tccaaggcag aaaatgaaat agaatgcaga agaatttgta tggtactata aagggaagat 180
   gaaaagttag ttagcatgga ttcaagtttg ataacccttt ggggtaaaat ctctttcaga 240
   ttatgatgga gctacaagtt attaggatat ttagattggt tgtggcatgt gttctttgtt 300
   tgtgtatatt tatcagatca gcttcttctg caactaaagg gtttgagagc atatcatgtt 360
   gtgctgattc caattacaca gatccaaaaa caaccctaac ttatacaaca gatcacatct 420
   ggttctctga taaaagaagt tgcagaccaa tacccgaaat tttgtttagc cacagaagca 480
   ataaaaatgt tcgaatattt gaaatagatg aaggaaagag atgttatact ttgccaacaa 540
   ttaaggatca agtatatttg ataaggggtg tatttccctt tgatagttta aattcttcgt 600
   tttatgttta tatcggggta acagaactag gtgaattaag atcgtctaga ctcgaggact 660
   tggaaatcga gggagttttt agagccacca aagactatat tgatttctgc ttattgaagg 720
   aagatgtcaa tcccttcatt tctcagattg aattgaggcc attacctgaa gaatacctac 780
   atggtttcgc tactagtgtt ttaaaactga taagcagaaa taatcttggt gacacaaatg 840
   atgatataag gttcccagat gaccaaaatg atagaatctg gaaacggaaa gcaacttcaa 900
   ctccatcatc tgcccttccc ctgtcttcca atgtcagcaa tgttgacctc aaagacagtg 960
   tcacacctcc tctacaagtc ctacaaacag ctcttactca ccctgagcga ttggagttcg 1020
   tccatgatgg cctcgagacc gatgattatg aatactctgt gtttctccac tttcttgaac 1080
   taaatggcac tgtcagagca ggacaaaggg tgtttgacat ctatctaaac aatgagatta 1140
   aaaaggagaa gtttgatgtt ttggctggag ggtccaagaa cagttacact gccttgaaca 1200
   tttcagcaaa tggatcactc aatataacct tagtcaaggc atctggatct gagtttggac 1260
   cccttttgaa tgcctatgaa atcctgcagg cacggtcgtg gattgaagag accaaccaaa 1320
   aagatttgga acttattcag aagatgagag aagaactgct gctgcacaac cgagaaaatg 1380
   aagcattgga gagttggagt ggagaccctt gtatgatttt cccctggaaa ggaataacat 1440
   gtgatgattc aactggttca tctattatca ctatgctgga tctttcttcc aataatctca 1500
   agggagcaat tccttacttt gtcactaaga tgaccaattt acaaatactg aacctgagcc 1560
   acaaccagtt cgattcgtta ttcccctcgt ttccaccgtc ctccttgctg atatcattgg 1620
   atctgagcta caatgatctt gatggacggc ttccagaatc cattatctca ctgccacatt 1680
   taaaatcatt atattttggc tgcaatccat atatgaagga cgaagataca acaaagttga 1740
   acagttcact aatcaataca gattatggga gatgcaaagg aaaaaaacca aagtttggac 1800
   aagtattcgt gattggagct attacaaggg gatcactttt gattactttg gctgttggaa 1860
   ttctattttt ttgccgttat agacacaagt caattacttt ggaaggattt ggtggaaaga 1920
   cctacccaat ggcaacaaat ataatcttct ctttgccaag caaagacgat ttcttcataa 1980
   agtctgtatc agttaaacca ttcactttgg agtatataga gcaggctaca gaacagtaca 2040
   aaactttgat aggtgaagga ggatttggtt ctgtttacag aggcactcta gacgatggtc 2100
   aagaagtggc agtgaaagtg cggtcatcca catcaactca gggaacccna gaatttgata 2160
   atgagctaaa cctactttca gctatacaac atgagaacct ggtgcctctt ctgggttact 2220
   gtaatgagta tgatcaacaa attctcgtgt atccattcat gtccaatggc tctttgctag 2280
   atagactata cggggaagca tcaaagagaa aaatattaga ctggccaact agactctcta 2340
   ttgctctcgg tgcagctcga ggtttggcat atcttcacac atttccagga cgttctgtaa 2400
   tacacaggga cgtaaaatcg agcaatatac tgctggatca gagcatgtgt gctaaggttg 2460
   cagattttgg tttctcaaaa tacgctcctc aggaaggaga cagttatgtt tcccttgaag 2520
   taagaggaac tgcagggtat ctggatcctg agtactacaa aacccagcaa ttatctgaaa 2580
   aaagtgatgt tttcagcttt ggtgtggttc tacttgaaat tgtaagtgga cgggaacctc 2640
   tcaacataaa gagaccacgg atcgagtgga gcttggttga atgggctaaa ccatacataa 2700
   gagcatcaaa ggtggatgaa attgtagatc ctggcatcaa gggaggatat catgcagaag 2760
   cattgtggag agttgtggaa gtagcactgc aatgtctaga accctactca acatataggc 2820
   catgcatggt tgatattgtc cgcgagttgg aggatgctct cattattgaa aacaatgcat 2880
   ctgaatacat gaaatccata gacagccttg gaggatccaa ccgctactca attgttatgg 2940
   acaaacgggc gctgccttca actacatcta cagcagaatc aactatcaca acccaaacct 3000
   tgacacaccc tcaaccgaga tagtaaatgg gtcgatggaa ttcttttgat ttgtttttta 3060
   tcattgcttt agtaatatcc cattttaaat ggtaaaggag aaaaatacta cttttgattg 3120
   tattttcatc cactctatgt ttcttgaaac tgaatctctc ttgctcagcc ccagttttta 3180
   tgggtgaaaa gaataatttg ggtcaaatgc aagtgaaacc atatggtgca taatttgaaa 3240
   gccatattat atcatttgct aagtccaaag taaaaatttc acaaactagt tagattgcga 3300
   tttagtctat acacacttca acagagctat atacactat 3339
<210> 6
   <211> 714
   <212> PRT
   <213> Medicago sativa Nod-
<400> 6
   Asn Leu Phe Gln Ile Ile Met Glu Leu Gln Val Ile Arg Ile Phe Arg
   1 5 10 15
   Leu Val Val Ala Cys Val Leu Cys Leu Cys Ile Phe Ile Arg Ser Ala
   20 25 30
   Ser Ser Ala Thr Lys Gly Phe Glu Ser Ile Ala Cys Cys Ala Asp Ser
   35 40 45
   Asn Tyr Thr Asp Pro Lys Thr Thr Leu Thr Tyr Thr Thr Asp His Ile
   50 55 60
   Trp Phe Ser Asp Lys Arg Ser Cys Arg Pro Ile Pro Glu Ile Leu Phe
   65 70 75 80
   Ser His Arg Ser Asn Lys Asn Val Arg Ile Phe Glu Ile Asp Glu Gly
   85 90 95
   Lys Arg Cys Tyr Thr Leu Pro Thr Ile Lys Asp Gln Val Tyr Leu Ile
   100 105 110
   Arg Gly Val Phe Pro Phe Asp Ser Leu Asn Ser Ser Phe Tyr Val Tyr
   115 120 125
   Ile Gly Val Thr Glu Leu Gly Glu Leu Arg Ser Ser Arg Leu Glu Asp
   130 135 140
   Leu Glu Ile Glu Gly Val Phe Arg Ala Thr Lys Asp Tyr Ile Asp Phe
   145 150 155 160
   Cys Leu Leu Lys Glu Asp Val Asn Pro Phe Ile Ser Gln Ile Glu Leu
   165 170 175
   Arg Pro Leu Pro Glu Glu Tyr Leu His Gly Phe Ala Thr Ser Val Leu
   180 185 190
   Lys Leu Ile Ser Arg Asn Asn Leu Gly Asp Ile Asn Asp Asp Ile Arg
   195 200 205
   Phe Pro Asp Asp Arg Asn Asp Arg Ile Trp Lys Arg Lys Ala Thr Ser
   210 215 220
   Thr Pro Ser Ser Ala Leu Pro Leu Ser Phe Asn Val Ser Asn Val Asp
   225 230 235 240
   Leu Lys Asp Ser Val Ala Pro Pro Leu Gln Val Leu Gln Thr Ala Leu
   245 250 255
   Thr His Pro Glu Arg Leu Glu Phe Val His Asp Gly Leu Glu Thr Asp
   260 265 270
   Asp Tyr Glu Tyr Ser Val Phe Leu His Phe Leu Glu Leu Asn Gly Thr
   275 280 285
   Val Arg Ala Gly Gln Arg Val Phe Asp Ile Tyr Leu Asn Asn Glu Ile
   290 295 300
   Lys Lys Glu Lys Phe Asp Val Leu Ala Gly Gly Ser Lys Asn Ser Tyr
   305 310 315 320
   Thr Ala Leu Asn Ile Ser Ala Asn Gly Ser Leu Asn Ile Thr Leu Val
   325 330 335
   Lys Ala Ser Gly Ser Glu Phe Gly Pro Leu Leu Asn Ala Tyr Glu Ile
   340 345 350
   Leu Gln Ala Arg Ser Trp Ile Glu Glu Thr Asn Gln Lys Asp Leu Glu
   355 360 365
   Leu Ile Gln Lys Thr Arg Glu Glu Leu Leu Leu His Asn Gln Glu Asn
   370 375 380
   Glu Ala Leu Glu Ser Trp Ser Gly Asp Pro Cys Met Ile Phe Pro Trp
   385 390 395 400
   Lys Gly Ile Thr Cys Asp Asp Ser Thr Gly Ser Ser Ile Ile Thr Met
   405 410 415
   Leu Asp Leu Ser Ser Asn Asn Leu Lys Gly Ala Ile Pro Tyr Phe Val
   420 425 430
   Thr Lys Met Thr Asn Leu Gln Ile Leu Asn Leu Ser His Asn Gln Phe
   435 440 445
   Asp Ser Leu Phe Pro Ser Phe Pro Pro Ser Ser Leu Leu Ile Ser Leu
   450 455 460
   Asp Leu Ser Tyr Asn Asp Leu Asp Gly Arg Leu Pro Glu Ser Ile Ile
   465 470 475 480
   Ser Leu Pro His Leu Lys Ser Leu Tyr Phe Gly Cys Asn Pro Tyr Met
   485 490 495
   Lys Asp Glu Asp Thr Thr Lys Leu Asn Ser Ser Leu Ile Asn Thr Asp
   500 505 510
   Tyr Gly Arg Cys Lys Gly Lys Lys Pro Lys Phe Gly Gln Val Phe Val
   515 520 525
   Ile Gly Ala Ile Thr Ser Gly Ser Leu Leu Ile Thr Leu Ala Val Gly
   530 535 540
   Ile Leu Phe Phe Cys Arg Tyr Arg His Lys Ser Ile Thr Leu Glu Gly
   545 550 555 560
   Phe Gly Gly Lys Thr Tyr Pro Met Ala Thr Asn Ile Ile Phe Ser Leu
   565 570 575
   Pro Ser Lys Asp Asp Phe Phe Ile Lys Ser Val Ser Val Lys Pro Phe
   580 585 590
   Thr Leu Glu Tyr Ile Glu Gln Ala Thr Glu Gln Tyr Lys Thr Leu Ile
   595 600 605
   Cys Glu Gly Gly Phe Gly Ser Val Tyr Arg Gly Thr Leu Asp Asp Gly
   610 615 620
   Gln Glu Val Ala Val Lys Val Arg Ser Ser Thr Ser Thr Gln Gly Thr
   625 630 635 640
   Lys Glu Phe Asp Asn Glu Leu Asn Leu Leu Ser Ala Ile Gln His Glu
   645 650 655
   Asn Leu Val Pro Leu Leu Gly Tyr Cys Asn Glu Tyr Asp Gln Gln Ile
   660 665 670
   Leu Val Tyr Pro Phe Met Ser Asn Gly Ser Leu Leu Asp Arg Leu Tyr
   675 680 685
   Gly Glu Ala Ser Lys Arg Lys Ile Leu Asp Trp Pro Thr Arg Leu Ser
   690 695 700
   Ile Ala Leu Gly Ala Pro Arg Gly Leu Ala
   705 710
<210> 7
   <211> 931
   <212> PRT
   <213> Medicago sativa Nod+
<220>
   <221> MISC_FEATURE
   <222> (641)..(641)
   <223> unknown
<400> 7
   Asn Leu Phe Gln Ile Met Met Glu Leu Gln Val Ile Arg Ile Phe Arg
   1 5 10 15
   Leu Val Val Ala Cys Val Leu Cys Leu Cys Ile Phe Ile Arg Ser Ala
   20 25 30
   Ser Ser Ala Thr Lys Gly Phe Glu Ser Ile Ala Cys Cys Ala Asp Ser
   35 40 45
   Asn Tyr Thr Asp Pro Lys Thr Thr Leu Thr Tyr Thr Thr Asp His Ile
   50 55 60
   Trp Phe Ser Asp Lys Arg Ser Cys Arg Pro Ile Pro Glu Ile Leu Phe
   65 70 75 80
   Ser His Arg Ser Asn Lys Asn Val Arg Ile Phe Glu Ile Asp Glu Gly
   85 90 95
   Lys Arg Cys Tyr Thr Leu Pro Thr Ile Lys Asp Gln Val Tyr Leu Ile
   100 105 110
   Arg Gly Val Phe Pro Phe Asp Ser Leu Asn Ser Ser Phe Tyr Val Tyr
   115 120 125
   Ile Gly Val Thr Glu Leu Gly Glu Leu Arg Ser Ser Arg Leu Glu Asp
   130 135 140
   Leu Glu Ile Glu Gly Val Phe Arg Ala Thr Lys Asp Tyr Ile Asp Phe
   145 150 155 160
   Cys Leu Leu Lys Glu Asp Val Asn Pro Phe Ile Ser Gln Ile Glu Leu
   165 170 175
   Arg Pro Leu Pro Glu Glu Tyr Leu His Gly Phe Ala Thr Ser Val Leu
   180 185 190
   Lys Leu Ile Ser Arg Asn Asn Leu Gly Asp Thr Asn Asp Asp Ile Arg
   195 200 205
   Phe Pro Asp Asp Gln Asn Asp Arg Ile Trp Lys Arg Lys Ala Thr Ser
   210 215 220
   Thr Pro Ser Ser Ala Leu Pro Leu Ser Ser Asn Val Ser Asn Val Asp
   225 230 235 240
   Leu Lys Asp Ser Val Thr Pro Pro Leu Gln Val Leu Gln Thr Ala Leu
   245 250 255
   Thr His Pro Glu Arg Leu Glu Phe Val His Asp Gly Leu Glu Thr Asp
   260 265 270
   Asp Tyr Glu Tyr Ser Val Phe Leu His Phe Leu Glu Leu Asn Gly Thr
   275 280 285
   Val Arg Ala Gly Gln Arg Val Phe Asp Ile Tyr Leu Asn Asn Glu Ile
   290 295 300
   Lys Lys Glu Lys Phe Asp Val Leu Ala Gly Gly Ser Lys Asn Ser Tyr
   305 310 315 320
   Thr Ala Leu Asn Ile Ser Ala Asn Gly Ser Leu Asn Ile Thr Leu Val
   325 330 335
   Lys Ala Ser Gly Ser Glu Phe Gly Pro Leu Leu Asn Ala Tyr Glu Ile
   340 345 350
   Leu Gln Ala Arg Ser Trp Ile Glu Glu Thr Asn Gln Lys Asp Leu Glu
   355 360 365
   Leu Ile Gln Lys Met Arg Glu Glu Leu Leu Leu His Asn Arg Glu Asn
   370 375 380
   Glu Ala Leu Glu Ser Trp Ser Gly Asp Pro Cys Met Ile Phe Pro Trp
   385 390 395 400
   Lys Gly Ile Thr Cys Asp Asp Ser Thr Gly Ser Ser Ile Ile Thr Met
   405 410 415
   Leu Asp Leu Ser Ser Asn Asn Leu Lys Gly Ala Ile Pro Tyr Phe Val
   420 425 430
   Thr Lys Met Thr Asn Leu Gln Ile Leu Asn Leu Ser His Asn Gln Phe
   435 440 445
   Asp Ser Leu Phe Pro Ser Phe Pro Pro Ser Ser Leu Leu Ile Ser Leu
   450 455 460
   Asp Leu Ser Tyr Asn Asp Leu Asp Gly Arg Leu Pro Glu Ser Ile Ile
   465 470 475 480
   Ser Leu Pro His Leu Lys Ser Leu Tyr Phe Gly Cys Asn Pro Tyr Met
   485 490 495
   Lys Asp Glu Asp Thr Thr Lys Leu Asn Ser Ser Leu Ile Asn Thr Asp
   500 505 510
   Tyr Gly Arg Cys Lys Gly Lys Lys Pro Lys Phe Gly Gln Val Phe Val
   515 520 525
   Ile Gly Ala Ile Thr Arg Gly Ser Leu Leu Ile Thr Leu Ala Val Gly
   530 535 540
   Ile Leu Phe Phe Cys Arg Tyr Arg His Lys Ser Ile Thr Leu Glu Gly
   545 550 555 560
   Phe Gly Gly Lys Thr Tyr Pro Met Ala Thr Asn Ile Ile Phe Ser Leu
   565 570 575
   Pro Ser Lys Asp Asp Phe Phe Ile Lys Ser Val Ser Val Lys Pro Phe
   580 585 590
   Thr Leu Glu Tyr Ile Glu Gln Ala Thr Glu Gln Tyr Lys Thr Leu Ile
   595 600 605
   Gly Glu Gly Gly Phe Gly Ser Val Tyr Arg Gly Thr Leu Asp Asp Gly
   610 615 620
   Gln Glu Val Ala Val Lys Val Arg Ser Ser Thr Ser Thr Gln Gly Thr
   625 630 635 640
   Xaa Glu Phe Asp Asn Glu Leu Asn Leu Leu Ser Ala Ile Gln His Glu
   645 650 655
   Asn Leu Val Pro Leu Leu Gly Tyr Cys Asn Glu Tyr Asp Gln Gln Ile
   660 665 670
   Leu Val Tyr Pro Phe Met Ser Asn Gly Ser Leu Leu Asp Arg Leu Tyr
   675 680 685
   Gly Glu Ala Ser Lys Arg Lys Ile Leu Asp Trp Pro Thr Arg Leu Ser
   690 695 700
   Ile Ala Leu Gly Ala Ala Arg Gly Leu Ala Tyr Leu His Thr Phe Pro
   705 710 715 720
   Gly Arg Ser Val Ile His Arg Asp Val Lys Ser Ser Asn Ile Leu Leu
   725 730 735
   Asp Gln Ser Met Cys Ala Lys Val Ala Asp Phe Gly Phe Ser Lys Tyr
   740 745 750
   Ala Pro Gln Glu Gly Asp Ser Tyr Val Ser Leu Glu Val Arg Gly Thr
   755 760 765
   Ala Gly Tyr Leu Asp Pro Glu Tyr Tyr Lys Thr Gln Gln Leu Ser Glu
   770 775 780
   Lys Ser Asp Val Phe Ser Phe Gly Val Val Leu Leu Glu Ile Val Ser
   785 790 795 800
   Gly Arg Glu Pro Leu Asn Ile Lys Arg Pro Arg Ile Glu Trp Ser Leu
   805 810 815
   Val Glu Trp Ala Lys Pro Tyr Ile Arg Ala Ser Lys Val Asp Glu Ile
   820 825 830
   Val Asp Pro Gly Ile Lys Gly Gly Tyr His Ala Glu Ala Leu Trp Arg
   835 840 845
   Val Val Glu Val Ala Leu Gln Cys Leu Glu Pro Tyr Ser Thr Tyr Arg
   850 855 860
   Pro Cys Met Val Asp Ile Val Arg Glu Leu Glu Asp Ala Leu Ile Ile
   865 870 875 880
   Glu Asn Asn Ala Ser Glu Tyr Met Lys Ser Ile Asp Ser Leu Gly Gly
   885 890 895
   Ser Asn Arg Tyr Ser Ile Val Met Asp Lys Arg Ala Leu Pro Ser Thr
   900 905 910
   Thr Ser Thr Ala Glu Ser Thr Ile Thr Thr Gln Thr Leu Thr His Pro
   915 920 925
   Gln Pro Arg
   930
<210> 8
   <211> 3360
   <212> DNA
   <213> Pisum sativum cv. Frisson
<220>
   <221> misc_feature
   <222> (393)..(892)
   <223> unknown
<220>
   <221> misc_feature
   <222> (394)..(892)
   <223> unknown
<220>
   <221> misc_feature
   <222> (893)..(1161)
   <223> unknown
<220>
   <221> misc_feature
   <222> (2785)..(3284)
   <223> unknown
<220>
   <221> misc_feature
   <222> (3285)..(3360)
   <223> unknown
<400> 8
   ttttttaaca atttttcttt ttctaatttc taaccctcca tcaaattgag aacttttgta 60
   aaatggtttc aaatcctttc acaaaggtat aacttttgta catgtttact atattagagg 120
   attgatcaag ttccctttga aaaatctcta gggggtgaaa tagaattcag aagaattttt 180
   atggtactat agggaagatg gagagttagt tagcatggat tcgagtttga gaaccctttg 240
   gggtaaccta tctttcggat tatgatggag ctacgagtta tttgtataat aagattggtt 300
   gtggcgtgtg ttctttgtct gtgtatattt atcagatcag cttcttctgc aactgaaggg 360
   tttgagagca tagcatgctg tgctgattcg aannnnnnnn nnnnnnnnnn nnnnnnnnnn 420
   nnnnnnnnnn nnnnnnnnnn nnnnnnnnnn nnnnnnnnnn nnnnnnnnnn nnnnnnnnnn 480
   nnnnnnnnnn nnnnnnnnnn nnnnnnnnnn nnnnnnnnnn nnnnnnnnnn nnnnnnnnnn 540
   nnnnnnnnnn nnnnnnnnnn nnnnnnnnnn nnnnnnnnnn nnnnnnnnnn nnnnnnnnnn 600
   nnnnnnnnnn nnnnnnnnnn nnnnnnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnnn nnnnnnnnnn nnnnnnnnnn 1080
   nnnnnnnnnn nnnnnnnnnn nnnnnnnnnn nnnnnnnnnn nnnnnnnnnn nnnnnnnnnn 1140
   nnnnnnnnnn nnnnnnnnnn ntttgatgta ttggaaggag gatccaagta cagttacact 1200
   gtcttgaaca tttcagcaaa tggatcactc aatataacct tagtcaaggc atctggatct 1260
   aagtttggac cccttttgaa tgcctatgag atcctgcagg cgcgaccatg gatcgatgag 1320
   accgaccaaa cagatctgga agttattcag aagatgagaa aagaactgct gctgcaaaac 1380
   caagacaatg aagcattgga gagttggagt ggagatcctt gtatgctttt tccatggaaa 1440
   ggagtagcat gtgatggttc aaatggttcg tctgtcatca ctaagctgga tctttcctcc 1500
   agtaatctca aaggaacaat tccttccagt gtcactgaga tgaccaaatt acaaatactg 1560
   aacctgagcc acaaccattt cgatggttat atcccctcat ttccaccgtc ctccttgttg 1620
   atatcagtag atctgagcta caatgaccta acgggacagc ttccagaatc cattatctca 1680
   ctgccacatt taaactcatt atattttggc tgcaatcaac acatgagaga cgatgatgaa 1740
   gccaaattga acagttcact aatcaataca gattatggga gatgtaatgc aaaaaaaccc 1800
   aaatttggac aagtattcat gattggagct attacaagtg gatcaatttt gattactttg 1860
   gctgttgtaa ttctattctt ttgccgttat agacacaagt caattacttt ggaaggattt 1920
   ggtggaaaga cctacccaat ggcaacaaat ataatttttt ccttgccaag caaagacgat 1980
   ttcttcataa agtctgtatc agttaagcca ttcactttgg agtatataga gttggcaaca 2040
   gagaagtaca aaactttgat aggtgaagga ggatttggct ctgtttaccg gggcactcta 2100
   gacgatggac aagaagtggc agtgaaagtc cggtcagcca catcaactca gggaacccga 2160
   gaatttgaca atgagctaaa cctactttca gctatacaac atgagaacct ggtgcctctt 2220
   cttggttact gtaatgaata tgatcaacaa attcttgtat atcctttcat gtctaatggc 2280
   tctttgctag acagactata tggggaaccg gcaaagagaa aaatactaga ctggccaact 2340
   agactctcaa ttgctctagg agcagctcga ggtttggcat atcttcacac gtttcctgga 2400
   cgatctgtaa tacacaggga cgtaaaatcg agcaatatac tactggacca tagcatgtgt 2460
   gctaaggttg cagattttgg tttctcaaaa tacgctcctc aggaaggaga cagttatgtt 2520
   tcccttgaag taagaggaac cgcaggatat ctggatcctg agtactacaa aacccagcaa 2580
   ttatctgaaa agagtgatgt tttcagcttt ggcgtggttc tactagaaat tgtaagtggc 2640
   cgggaacctc tcaacataaa gagaccacga gtcgagtgga gcttggttga atgggctaaa 2700
   ccatatataa gagcatcaaa ggtggatgaa attgtagatc ctggcatcaa gggaggatat 2760
   catgcagagg cactgtggag agttnnnnnn nnnnnnnnnn nnnnnnnnnn nnnnnnnnnn 2820
   nnnnnnnnnn nnnnnnnnnn nnnnnnnnnn nnnnnnnnnn nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnnnnnnn nnnnnnnnnn nnnnnnnnnn nnnnnnnnnn 3060
   nnnnnnnnnn nnnnnnnnnn nnnnnnnnnn nnnnnnnnnn nnnnnnnnnn nnnnnnnnnn 3120
   nnnnnnnnnn nnnnnnnnnn nnnnnnnnnn nnnnnnnnnn nnnnnnnnnn nnnnnnnnnn 3180
   nnnnnnnnnn nnnnnnnnnn nnnnnnnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnnnnn nnnnnnnnnn nnnnnnnnnn nnnnnnnnnn nnnnnnnnnn 3360
<210> 9
   <211> 3420
   <212> DNA
   <213> Vicia villosa S-1
<220>
   <221> misc_feature
   <222> (1)..(500)
   <223> unknown
<220>
   <221> misc_feature
   <222> (501)..(740)
   <223> unknown
<220>
   <221> misc_feature
   <222> (2838)..(3337)
   <223> unknown
<220>
   <221> misc_feature
   <222> (3338)..(3420)
   <223> unknown
<400> 9
   nnnnnnnnnn nnnnnnnnnn nnnnnnnnnn nnnnnnnnnn nnnnnnnnnn nnnnnnnnnn    60
   nnnnnnnnnn nnnnnnnnnn nnnnnnnnnn nnnnnnnnnn nnnnnnnnnn nnnnnnnnnn   120
   nnnnnnnnnn nnnnnnnnnn nnnnnnnnnn nnnnnnnnnn nnnnnnnnnn nnnnnnnnnn   180
   nnnnnnnnnn nnnnnnnnnn nnnnnnnnnn nnnnnnnnnn nnnnnnnnnn nnnnnnnnnn   240
   nnnnnnnnnn nnnnnnnnnn nnnnnnnnnn nnnnnnnnnn nnnnnnnnnn nnnnnnnnnn   300
   nnnnnnnnnn nnnnnnnnnn nnnnnnnnnn nnnnnnnnnn nnnnnnnnnn nnnnnnnnnn   360
   nnnnnnnnnn nnnnnnnnnn nnnnnnnnnn nnnnnnnnnn nnnnnnnnnn nnnnnnnnnn   420
   nnnnnnnnnn nnnnnnnnnn nnnnnnnnnn nnnnnnnnnn nnnnnnnnnn nnnnnnnnnn   480
   nnnnnnnnnn nnnnnnnnnn nnnnnnnnnn nnnnnnnnnn nnnnnnnnnn nnnnnnnnnn   540
   nnnnnnnnnn nnnnnnnnnn nnnnnnnnnn nnnnnnnnnn nnnnnnnnnn nnnnnnnnnn   600
   nnnnnnnnnn nnnnnnnnnn nnnnnnnnnn nnnnnnnnnn nnnnnnnnnn nnnnnnnnnn   660
   nnnnnnnnnn nnnnnnnnnn nnnnnnnnnn nnnnnnnnnn nnnnnnnnnn nnnnnnnnnn   720
   nnnnnnnnnn nnnnnnnnnn tggcatagac ttctgcttat tgaaggagga tgccaatccc   780
   ttcatttctc agcttgaact gaggccatta cctgaagaat acatgcatga tttctctacc   840
   agcgttttaa aactgataaa cagaaataat ctttgtggca tagaagacga catcaggttc   900
   cctgttgacc aaaatgatag aatctggaaa gcaacttcaa ctccatcata tgctgttcca 960
   ctgtctttca atgtcagtga tgttgatctc aacggcaaag tgacacctcc tcgacaagtc 1020
   ctacaaacag ctcttactca ccctgatcga ttggtgttcg tccacgacgg cctcgagacc 1080
   gatgattatg aatactctgt gttactctac tttcttgaac taaataacac tgtcaaagca 1140
   ggacaaaggg tgtttgacat ctatctaaac agtgagatta aaaaggagag ttttgatgta 1200
   tcggaaggag gatccaagta cagttacatc accttgaaca tttcagcaaa tggatcactc 1260
   aatataacct tagccaaggc atctggatct aagtttggac cccttttgaa tgcttatgaa 1320
   atcctgcagg cgcgaccatg gatcgatgag accgaccaaa cagatgtgga agttattcag 1380
   aagttgagaa aagaactact gctgcaaaac caagacaatg aagcattgga gagttggagt 1440
   ggagatcctt gtatgctgtt tccctggaaa ggagtagcat gcgatggttc aaatggttcg 1500
   tctgtcatca ctaagctgga tctttcctcc agtaatctca aaggaacaat cccttccagt 1560
   gtcactgaga tgaccaaatt acaaatactg aacctgagcc acaaccactt cgatggctat 1620
   atcccctcgt ttccatcgtc ttccttgttg atatcagtag atctgagcta caatgaccta 1680
   acgggacagc ttccagaatc cattatctca ctgccacatt taaactcatt gtattttggt 1740
   tgcaatcaac acatgagcga cgatgatgaa gccaaattga acagttcact aatcagtaca 1800
   gattatggga gatgcaaggc aaaaagcccc aaatttggac aagtattcat gattggagca 1860
   attacaagtg gatcgatttt gattactttg gctgttggaa ttctattttt ttgccgttat 1920
   agacacaagt caattacttt ggaaggattt ggtggaaaga cctacccgat ggcaacaaat 1980
   ataattttct ccttgccaag caaagacgat ttcttcataa agtctgtatc agttaaaccg 2040
   ttctctttgg agtatataga gttggcaaca gagaagtaca aaactttgat aggtgaagga 2100
   gggtttggct ctgtttaccg aggcactcta gacgatggtc aagaagtggc agtgaaagtc 2160
   cggtcagcca catcaactca gggaacccga gaatttgaca atgagctaaa cctactttca 2220
   gctatacaac atgagaacct ggtgcctctt cttggttact gtaatgaata tgatcaacaa 2280
   attcttgtgt accctttcat gtctaatgga tctttgctag atagactata tggggaacca 2340
   gcaaagagaa aaatactaga ctggccaact agactctcta ttgctctagg agcagctcga 2400
   ggtttggcat atcttcacac atttcctgga cggtctgtaa tacacaggga cgtaaaatcg 2460
   agcaatatac tactggacca tagcatgtgt gctaaggtcg cagattttgg tttctcaaaa 2520
   tacgctcctc aggaaggaga cagttatgtt tcccttgaag taagaggaac tgcagggtat 2580
   ctggatcccg agtactacaa aacccagcaa ttatctgaaa agagtgatgt ttttagtttt 2640
   ggcgtggttc tactagaaat tgtaagtggc cgggaacctc tcaacataaa gagaccacga 2700
   gtcgagtgga gcttggttga atgggctaaa ccatacataa gagcatcaaa ggtggatgaa 2760
   attgtagatc ctggcatcaa gggaggatat catgcagagg cactgtggag agtggtggaa 2820
   gtagcactgc aatgtctnnn nnnnnnnnnn nnnnnnnnnn nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnnnnnnn nnnnnnnnnn nnnnnnnnnn nnnnnnnnnn 3060
   nnnnnnnnnn nnnnnnnnnn nnnnnnnnnn nnnnnnnnnn nnnnnnnnnn nnnnnnnnnn 3120
   nnnnnnnnnn nnnnnnnnnn nnnnnnnnnn nnnnnnnnnn nnnnnnnnnn nnnnnnnnnn 3180
   nnnnnnnnnn nnnnnnnnnn nnnnnnnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnnnnn nnnnnnnnnn nnnnnnnnnn nnnnnnnnnn nnnnnnnnnn 3360
   nnnnnnnnnn nnnnnnnnnn nnnnnnnnnn nnnnnnnnnn nnnnnnnnnn nnnnnnnnnn 3420
<210> 10
   <211> 922
   <212> PRT
   <213> Pisum sativum cv. Frisson
<220>
   <221> M)SC_FEATURE
   <222> (43)..(299)
   <223> unknown
<220>
   <221> MISC_FEATURE
   <222> (891)..(922)
   <223> unknown
<220>
   <221> MISC_FEATURE
   <222> (841)..(890)
   <223> unknown
<400> 10
   Met Glu Leu Arg Val Ile Cys Ile Ile Arg Leu Val Val Ala Cys Val
   1 5 10 15
   Leu Cys Leu Cys Ile Phe Ile Arg Ser Ala Ser Ser Ala Thr Glu Gly
   20 25 30
   Phe Glu Ser Ile Ala Cys Cys Ala Asp Ser Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Xaa Xaa Xaa Xaa Xaa Xaa Xaa Xaa Xaa Xaa Xaa Xaa Xaa Xaa
   100 105 110
   Xaa Xaa Xaa Xaa Xaa Xaa Xaa Xaa Xaa Xaa Xaa Xaa Xaa Xaa Xaa Xaa
   115 120 125
   Xaa Xaa Xaa Xaa Xaa Xaa Xaa Xaa Xaa Xaa Xaa Xaa Xaa Xaa Xaa Xaa
   130 135 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Xaa Xaa Xaa Xaa
   165 170 175
   Xaa Xaa Xaa Xaa Xaa Xaa Xaa Xaa Xaa Xaa Xaa Xaa Xaa Xaa Xaa Xaa
   180 185 190
   Xaa Xaa Xaa Xaa Xaa Xaa Xaa Xaa Xaa Xaa Xaa Xaa Xaa Xaa Xaa Xaa
   195 200 205
   Xaa Xaa Xaa Xaa Xaa Xaa Xaa Xaa Xaa Xaa Xaa Xaa Xaa Xaa Xaa Xaa
   210 215 220
   Xaa Xaa Xaa Xaa Xaa Xaa Xaa Xaa Xaa Xaa Xaa Xaa Xaa Xaa Xaa Xaa
   225 230 235 240
   Xaa Xaa Xaa Xaa Xaa Xaa Xaa Xaa Xaa Xaa Xaa Xaa Xaa Xaa Xaa Xaa
   245 250 255
   Xaa Xaa Xaa Xaa Xaa Xaa Xaa Xaa Xaa Xaa Xaa Xaa Xaa Xaa Xaa Xaa
   260 265 270
   Xaa Xaa Xaa Xaa Xaa Xaa Xaa Xaa Xaa Xaa Xaa Xaa Xaa Xaa Xaa Xaa
   275 280 285
   Xaa Xaa Xaa Xaa Xaa Xaa Xaa Xaa Xaa Xaa Xaa Phe Asp Val Leu Glu
   290 295 300
   Gly Gly Ser Lys Tyr Ser Tyr Thr Val Leu Asn Ile Ser Ala Asn Gly
   305 310 315 320
   Ser Leu Asn Ile Thr Leu Val Lys Ala Ser Gly Ser Lys Phe Gly Pro
   325 330 335
   Leu Leu Asn Ala Tyr Glu Ile Leu Gln Ala Arg Pro Trp Ile Asp Glu
   340 345 350
   Thr Asp Gln Thr Asp Leu Glu Val Ile Gln Lys Met Arg Lys Glu Leu
   355 360 365
   Leu Leu Gln Asn Gln Asp Asn Glu Ala Leu Glu Ser Trp Ser Gly Asp
   370 375 380
   Pro Cys Met Leu Phe Pro Trp Lys Gly Val Ala Cys Asp Gly Ser Asn
   385 390 395 400
   Gly Ser Ser Val Ile Thr Lys Leu Asp Leu Ser Ser Ser Asn Leu Lys
   405 410 415
   Gly Thr Ile Pro Ser Ser Val Thr Glu Met Thr Lys Leu Gln Ile Leu
   420 425 430
   Asn Leu Ser His Asn His Phe Asp Gly Tyr Ile Pro Ser Phe Pro Pro
   435 440 445
   Ser Ser Leu Leu Ile Ser Val Asp Leu Ser Tyr Asn Asp Leu Thr Gly
   450 455 460
   Gln Leu Pro Glu Ser Ile Ile Ser Leu Pro His Leu Asn Ser Leu Tyr
   465 470 475 480
   Phe Gly Cys Asn Gln His Met Arg Asp Asp Asp Glu Ala Lys Leu Asn
   485 490 495
   Ser Ser Leu Ile Asn Thr Asp Tyr Gly Arg Cys Asn Ala Lys Lys Pro
   500 505 510
   Lys Phe Gly Gln Val Phe Met Ile Gly Ala Ile Thr Ser Gly Ser Ile
   515 520 525
   Leu Ile Thr Leu Ala Val Val Ile Leu Phe Phe Cys Arg Tyr Arg His
   530 535 540
   Lys Ser Ile Thr Leu Glu Gly Phe Gly Gly Lys Thr Tyr Pro Met Ala
   545 550 555 560
   Thr Asn Ile Ile Phe Ser Leu Pro Ser Lys Asp Asp Phe Phe Ile Lys
   565 570 575
   Ser Val Ser Val Lys Pro Phe Thr Leu Glu Tyr Ile Glu Leu Ala Thr
   580 585 590
   Glu Lys Tyr Lys Thr Leu Ile Gly Glu Gly Gly Phe Gly Ser Val Tyr
   595 600 605
   Arg Gly Thr Leu Asp Asp Gly Gln Glu Val Ala Val Lys Val Arg Ser
   610 615 620
   Ala Thr Ser Thr Gln Gly Thr Arg Glu Phe Asp Asn Glu Leu Asn Leu
   625 630 635 640
   Leu Ser Ala Ile Gln His Glu Asn Leu Val Pro Leu Leu Gly Tyr Cys
   645 650 655
   Asn Glu Tyr Asp Gln Gln Ile Leu Val Tyr Pro Phe Met Ser Asn Gly
   660 665 670
   Ser Leu Leu Asp Arg Leu Tyr Gly Glu Pro Ala Lys Arg Lys Ile Leu
   675 680 685
   Asp Trp Pro Thr Arg Leu Ser Ile Ala Leu Gly Ala Ala Arg Gly Leu
   690 695 700
   Ala Tyr Leu His Thr Phe Pro Gly Arg Ser Val Ile His Arg Asp Val
   705 710 715 720
   Lys Ser Ser Asn Ile Leu Leu Asp His Ser Met Cys Ala Lys Val Ala
   725 730 735
   Asp Phe Gly Phe Ser Lys Tyr Ala Pro Gln Glu Gly Asp Ser Tyr Val
   740 745 750
   Ser Leu Glu Val Arg Gly Thr Ala Gly Tyr Leu Asp Pro Glu Tyr Tyr
   755 760 765
   Lys Thr Gln Gln Leu Ser Glu Lys Ser Asp Val Phe Ser Phe Gly Val
   770 775 780
   Val Leu Leu Glu Ile Val Ser Gly Arg Glu Pro Leu Asn Ile Lys Arg
   785 790 795 800
   Pro Arg Val Glu Trp Ser Leu Val Glu Trp Ala Lys Pro Tyr Ile Arg
   805 810 815
   Ala Ser Lys Val Asp Glu Ile Val Asp Pro Gly Ile Lys Gly Gly Tyr
   820 825 830
   His Ala Glu Ala Leu Trp Arg Val Xaa Xaa Xaa Xaa Xaa Xaa Xaa Xaa
   835 840 845
   Xaa Xaa Xaa Xaa Xaa Xaa Xaa Xaa Xaa Xaa Xaa Xaa Xaa Xaa Xaa Xaa
   850 855 860
   Xaa Xaa Xaa Xaa Xaa Xaa Xaa Xaa Xaa Xaa Xaa Xaa Xaa Xaa Xaa Xaa
   865 870 875 880
   Xaa Xaa Xaa Xaa Xaa Xaa Xaa Xaa Xaa Xaa Xaa Xaa Xaa Xaa Xaa Xaa
   885 890 895
   Xaa Xaa Xaa Xaa Xaa Xaa Xaa Xaa Xaa Xaa Xaa Xaa Xaa Xaa Xaa Xaa
   900 905 910
   Xaa Xaa Xaa Xaa Xaa Xaa Xaa Xaa Xaa Xaa
   915 920
<210> 11
   <211> 1020
   <212> PRT
   <213> Vicia villosa S-1
<220>
   <221> MISC_FEATURE
   <222> (1)..(247)
   <223>
<220>
   <221> MISC_FEATURE
   <222> (1)..(247)
   <223> unknown
<220>
   <221> MISC_FEATURE
   <222> (947)..(1020)
   <223> unknown
<400> 11
   Xaa Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Xaa Xaa Xaa Xaa Xaa Xaa Xaa Xaa Xaa Xaa Xaa
   20 25 30
   Xaa Xaa Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Xaa Xaa Xaa Xaa Xaa Xaa Xaa Xaa Xaa Xaa Xaa Xaa Xaa Xaa
   100 105 110
   Xaa Xaa Xaa Xaa Xaa Xaa Xaa Xaa Xaa Xaa Xaa Xaa Xaa Xaa Xaa Xaa
   115 120 125
   Xaa Xaa Xaa Xaa Xaa Xaa Xaa Xaa Xaa Xaa Xaa Xaa Xaa Xaa Xaa Xaa
   130 135 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Xaa Xaa Xaa Xaa
   165 170 175
   Xaa Xaa Xaa Xaa Xaa Xaa Xaa Xaa Xaa Xaa Xaa Xaa Xaa Xaa Xaa Xaa
   180 185 190
   Xaa Xaa Xaa Xaa Xaa Xaa Xaa Xaa Xaa Xaa Xaa Xaa Xaa Xaa Xaa Xaa
   195 200 205
   Xaa Xaa Xaa Xaa Xaa Xaa Xaa Xaa Xaa Xaa Xaa Xaa Xaa Xaa Xaa Xaa
   210 215 220
   Xaa Xaa Xaa Xaa Xaa Xaa Xaa Xaa Xaa Xaa Xaa Xaa Xaa Xaa Xaa Xaa
   225 230 235 240
   Xaa Xaa Xaa Xaa Xaa Xaa Xaa Gly Ile Asp Phe Cys Leu Leu Lys Glu
   245 250 255
   Asp Ala Asn Pro Phe Ile Ser Gln Leu Glu Leu Arg Pro Leu Pro Glu
   260 265 270
   Glu Tyr Met His Asp Phe Ser Thr Ser Val Leu Lys Leu Ile Asn Arg
   275 280 285
   Asn Asn Leu Cys Gly Ile Glu Asp Asp Ile Arg Phe Pro Val Asp Gln
   290 295 300
   Asn Asp Arg Ile Trp Lys Ala Thr Ser Thr Pro Ser Tyr Ala Val Pro
   305 310 315 320
   Leu Ser Phe Asn Val Ser Asp Val Asp Leu Asn Gly Lys Val Thr Pro
   325 330 335
   Pro Arg Gln Val Leu Gln Thr Ala Leu Thr His Pro Asp Arg Leu Val
   340 345 350
   Phe Val His Asp Gly Leu Glu Thr Asp Asp Tyr Glu Tyr Ser Val Leu
   355 360 365
   Leu Tyr Phe Leu Glu Leu Asn Asn Thr Val Lys Ala Gly Gln Arg Val
   370 375 380
   Phe Asp Ile Tyr Leu Asn Ser Glu Ile Lys Lys Glu Ser Phe Asp Val
   385 390 395 400
   Ser Glu Gly Gly Ser Lys Tyr Ser Tyr Ile Thr Leu Asn Ile Ser Ala
   405 410 415
   Asn Gly Ser Leu Asn Ile Thr Leu Ala Lys Ala Ser Gly Ser Lys Phe
   420 425 430
   Gly Pro Leu Leu Asn Ala Tyr Glu Ile Leu Gln Ala Arg Pro Trp Ile
   435 440 445
   Asp Glu Thr Asp Gln Thr Asp Val Glu Val Ile Gln Lys Leu Arg Lys
   450 455 460
   Glu Leu Leu Leu Gln Asn Gln Asp Asn Glu Ala Leu Glu Ser Trp Ser
   465 470 475 480
   Gly Asp Pro Cys Met Leu Phe Pro Trp Lys Gly Val Ala Cys Asp Gly
   485 490 495
   Ser Asn Gly Ser Ser Val Ile Thr Lys Leu Asp Leu Ser Ser Ser Asn
   500 505 510
   Leu Lys Gly Thr Ile Pro Ser Ser Val Thr Glu Met Thr Lys Leu Gln
   515 520 525
   Ile Leu Asn Leu Ser His Asn His Phe Asp Gly Tyr Ile Pro Ser Phe
   530 535 540
   Pro Ser Ser Ser Leu Leu Ile Ser Val Asp Leu Ser Tyr Asn Asp Leu
   545 550 555 560
   Thr Gly Gln Leu Pro Glu Ser Ile Ile Ser Leu Pro His Leu Asn Ser
   565 570 575
   Leu Tyr Phe Gly Cys Asn Gln His Met Ser Asp Asp Asp Glu Ala Lys
   580 585 590
   Leu Asn Ser Ser Leu Ile Ser Thr Asp Tyr Gly Arg Cys Lys Ala Lys
   595 600 605
   Ser Pro Lys Phe Gly Gln Val Phe Met Ile Gly Ala Ile Thr Ser Gly
   610 615 620
   Ser Ile Leu Ile Thr Leu Ala Val Gly Ile Leu Phe Phe Cys Arg Tyr
   625 630 635 640
   Arg His Lys Ser Ile Thr Leu Glu Gly Phe Gly Gly Lys Thr Tyr Pro
   645 650 655
   Met Ala Thr Asn Ile Ile Phe Ser Leu Pro Ser Lys Asp Asp Phe Phe
   660 665 670
   Ile Lys Ser Val Ser Val Lys Pro Phe Ser Leu Glu Tyr Ile Glu Leu
   675 680 685
   Ala Thr Glu Lys Tyr Lys Thr Leu Ile Gly Glu Gly Gly Phe Gly Ser
   690 695 700
   Val Tyr Arg Gly Thr Leu Asp Asp Gly Gln Glu Val Ala Val Lys Val
   705 710 715 720
   Arg Ser Ala Thr Ser Thr Gln Gly Thr Arg Glu Phe Asp Asn Glu Leu
   725 730 735
   Asn Leu Leu Ser Ala Ile Gln His Glu Asn Leu Val Pro Leu Leu Gly
   740 745 750
   Tyr Cys Asn Glu Tyr Asp Gln Gln Ile Leu Val Tyr Pro Phe Met Ser
   755 760 765
   Asn Gly Ser Leu Leu Asp Arg Leu Tyr Gly Glu Pro Ala Lys Arg Lys
   770 775 780
   Ile Leu Asp Trp Pro Thr Arg Leu Ser Ile Ala Leu Gly Ala Ala Arg
   785 790 795 800
   Gly Leu Ala Tyr Leu His Thr Phe Pro Gly Arg Ser Val Ile His Arg
   805 810 815
   Asp Val Lys Ser Ser Asn Ile Leu Leu Asp His Ser Met Cys Ala Lys
   820 825 830
   Val Ala Asp Phe Gly Phe Ser Lys Tyr Ala Pro Gln Glu Gly Asp Ser
   835 840 845
   Tyr Val Ser Leu Glu Val Arg Gly Thr Ala Gly Tyr Leu Asp Pro Glu
   850 855 860
   Tyr Tyr Lys Thr Gln Gln Leu Ser Glu Lys Ser Asp Val Phe Ser Phe
   865 870 875 880
   Gly Val Val Leu Leu Glu Ile Val Ser Gly Arg Glu Pro Leu Asn Ile
   885 890 895
   Lys Arg Pro Arg Val Glu Trp Ser Leu Val Glu Trp Ala Lys Pro Tyr
   900 905 910
   Ile Arg Ala Ser Lys Val Asp Glu Ile Val Asp Pro Gly Ile Lys Gly
   915 920 925
   Gly Tyr His Ala Glu Ala Leu Trp Arg Val Val Glu Val Ala Leu Gln
   930 935 940
   Cys Leu Xaa Xaa Xaa Xaa Xaa Xaa Xaa Xaa Xaa Xaa Xaa Xaa Xaa Xaa
   945 950 955 960
   Xaa Xaa Xaa Xaa Xaa Xaa Xaa Xaa Xaa Xaa Xaa Xaa Xaa Xaa Xaa Xaa
   965 970 975
   Xaa Xaa Xaa Xaa Xaa Xaa Xaa Xaa Xaa Xaa Xaa Xaa Xaa Xaa Xaa Xaa
   980 985 990
   Xaa Xaa Xaa Xaa Xaa Xaa Xaa Xaa Xaa Xaa Xaa Xaa Xaa Xaa Xaa Xaa
   995 1000 1005
   Xaa Xaa Xaa Xaa Xaa Xaa Xaa Xaa Xaa Xaa Xaa Xaa
   1010 1015 1020

## Claims

1. Nucleotide sequence coding for the polypeptide SEQ ID NO: 3 as shown in Figure 12 having NOD region specific receptor kinase (NORK) gene activity.

2. Nucleotide sequence coding for the Nod⁺ M. sativa NORK polypeptide having the SEQ ID NO: 5 as shown in Figure 15 which is a genomic or synthetic DNA molecule.

3. The recombinant form of any of the nucleotide sequences of claims 1 and 2 which comprises a functional promoter sequence.

4. Antibody capable of specifically binding to the polypeptides encoded by the DNA sequences according to any of claims 1 and 2.

5. Transgenic plant which comprises the nucleotide sequence of any of claims 1 and 2.

6. The transgenic plant according to claim 5 which is a crop plant.

7. The transgenic plant according to claim 5 which is tobacco plant.

8. Transformant cell which contains the nucleotide sequence according to any of claims 1 and 2.

9. The polypeptide SEQ ID NO: 3 as shown in Figure 12 and a fragment thereof encoded by polynucleotide sequences which are capable of complementing the NOD⁻ phenotype of a M. sativa nodulation-defective plant.

10. Process for preparing plants of NOD⁺ phenotype from plants of NOD⁻ phenotype **characterized by** transforming a plant or the reproducing form thereof with a DNA cording to any of claims 1 and 2.

## Patentansprüche

1. NOD regionspezifisches Rezeptorkinase (NORK) Genaktivität aufweisendes Nukleotidsequenz, welches ein Polypeptid SEQ ID NO:3 der Abbildung 12 kodiert.

2. Das Nod⁺ M. Sativa NORK Polypeptid SEQ ID NO: 5 der Abbildung 15 kodierendes Nukleotidsequenz, welches ein genomisches oder synthetisches DNS-Molekül ist.

3. Die rekombinante Form einer der Nukleotidsequenzen der Anspüche 1 und 2, welches ein funkzionelles Promotersequenz enthält.

4. Ein, zu den durch die DNS Sequenzen der Anspüche 1 und 2 kodierten Polypeptide spezifisch zu binden fähiger Antikörper.

5. Eine der Nukleotidsequenzen der Ansprüche 1 und 2 enthaltende transgenische Pflanze.

6. Die transgenische Pflanze von Anspruch 5, welche eine Nutzpflanze ist.

7. Die transgenische Pflanze von Anspruch 5, welche eine Tabakpflanze ist.

8. Eine der Nukleotidsequenzen der Ansprüche 1 und 2 enthaltende transformierte Zelle.

9. Das Polypeptid SEQ ID NO: 3 der Abbildung 12 oder dessen Fragment, welches durch die NOD⁻ Phenotyp einer M. sativa nodulation-defektiven Pflanze komplementierungsfähigen Polynukleotidsequenz kodiert wird.

10. Verfahren zur Herstellung von Pflanzen vom NOD⁺ Phenotyp aus Pflanzen vom NOD⁺ Phenotyp **dadurch gekennzeichnet, dass** man eine Pflanze oder eine Reproduktionsform derselben durch eine DNS der Ansprüche 1 und 2 transformiert.

## Revendications

1. Séquence nucléotidique codant pour le polypeptide de séquence SEQ ID N° 3, tell que présentée à la figure 12, ayant une activité de gène récepteur kinase spécifique de la région NOD (NORK).

2. Séquence nucléotide codant pour le polypeptide NORK de *M.sativa* Nod⁺ ayant le séquence SEQ ID N° 5, telle que présentée à la figure 15, qui est une molécule d'ADN génomique ou synthétique.

3. Forme recombinante de l'une quelconque des séquences nucléotidiques selon les revendications 1 et 2, qui comprend une séquence promotrice fonctionnelle.

4. Anticorps capable de se lier spécifiquement aux polypeptides codés par les séquences d'ADN selon l'une quelconque des revendications 1 et 2.

5. Plante transgénique qui comprend la séquence nucléotidique selon l'une quelconque des revendications 1 et 2.

6. Plante transgénique selon la revendication 5, qui est uneplante cultivée.

7. Plante transgénique selon la revendication 5, qui est le tabac.

8. Cellule transformée qui contient la séquence nucléotidique selon l'une quelconque des revendications 1 et 2.

9. Polypeptide de séquence SEQ ID N° 3, telle que présentée à la figure 12, et fragment de celui-ci codé par des séquences polynucléotidiques qui sont capables de complémenter le phénotype NOD⁻ d 'une plante *M.sativa* déficiente en nodulation.

10. Procédé de préparation de plantes à phénotype NOD⁺ à partir de plantes à phénotype NOD⁻, **caractérisé par** la transformation d'une plante ou de sa forme reproductrice avec un ADN selon l'une quelconque des revendication 1 et 2.
